(19) Europäisches Patentamt
European Patent Office
Office européen des brevets

(11) **EP 4 329 849 B1**

(12) **EUROPEAN PATENT SPECIFICATION**

(45) Date of publication and mention
of the grant of the patent:
**10.09.2025 Bulletin 2025/37**

(21) Application number: **22725285.5**

(22) Date of filing: **29.04.2022**

(51) International Patent Classification (IPC):
*A61M 16/00* (2006.01)    *A61M 15/00* (2006.01)
*A61M 16/10* (2006.01)    *A61B 5/08* (2006.01)
*A61K 31/01* (2006.01)    *A61K 31/095* (2006.01)
*A61K 31/12* (2006.01)    *A61K 31/60* (2006.01)

(52) Cooperative Patent Classification (CPC):
**A61M 16/0066; A61B 5/082; A61B 5/087;
A61M 15/0003; A61M 15/0021; A61M 15/0026;
A61M 15/0066; A61M 15/0068; A61M 16/1065;**
A61M 15/0006; A61M 2202/0007; A61M 2202/0275;
A61M 2205/3303; A61M 2205/3334;
A61M 2205/3368;                          (Cont.)

(86) International application number:
**PCT/GB2022/051110**

(87) International publication number:
**WO 2022/229665 (03.11.2022 Gazette 2022/44)**

(54) **INHALER**

INHALATOR

INHALATEUR

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.04.2021 GB 202106161**

(43) Date of publication of application:
**06.03.2024 Bulletin 2024/10**

(73) Proprietor: **3D Engineering Design Limited
Mansfield Nottinghamshire NG19 8RL (GB)**

(72) Inventors:
• **MARSHALL, Stephen John
Mansfield Nottinghamshire NG19 8SG (GB)**
• **CHAPMAN, Alan
Chesterfield Derbyshire S44 6TP (GB)**
• **NARSHBHAI, Sanjay
Birmingham West Midlands B27 6QD (GB)**
• **DRABBLE, Richard
Chesterfield Derbyshire S43 4RS (GB)**

(74) Representative: **Forresters IP LLP
Skygarden
Erika-Mann-Straße 11
80636 München (DE)**

(56) References cited:
WO-A1-2004/011068    WO-A2-2015/066562
WO-A2-2016/060863

(52) Cooperative Patent Classification (CPC): (Cont.)
A61M 2205/505; A61M 2205/52; A61M 2205/581;
A61M 2205/582; A61M 2205/583; A61M 2230/43;
A61M 2230/432; A61M 2230/46; A61M 2230/50

**Description**

The present disclosure relates to an inhaler device and more particularly, although not exclusively, to a variable dose inhaler device that offers improved end user functionality.

Background of the invention

Small, handheld inhalers for delivering medicaments to the respiratory tract of a user are well-known and very widely used. Examples of prior art inhalers are disclosed in WO2016/060863, WO2015/066562 and WO2004/011068. Conventional inhalers deliver a generally fixed dose of medicament from a canister upon each instance of use, i.e. upon triggering of the canister. However, there are a variety of reasons why it may be beneficial to vary the dose.

EP3185940 discloses a variable dose inhaler having a controller and personalised drug dose release mechanism and a spirometry test mechanism. The user exhales via the spirometry test mechanism and the controller determines a drug dose to be delivered using a sensor for the exhaled air flow. An air source adjusts the air pressure applied through a venturi nozzle and the dose is delivered to the user.

Figures 16 and 16A of EP3185940 disclose a variable dose inhaler mechanism in which a wall of a chamber is adjusted to vary the internal volume of the chamber. Once the volume is set, positive pressure in the delivery mechanism fills the chamber and a valve opens to allow the chamber contents to be delivered to the user.

These examples of variable dose inhaler provide specific delivery mechanisms and sensing arrangements. However, they do not accommodate the significant complexity that can arise in trying to accommodate a wide variety of user requirements and different medicament delivery scenarios.

Statements of Invention

The invention is defined by the appended claims.

It is an aim of the present technology to provide an improved dose delivery mechanism or control scheme for certain usage requirements. It is an additional or alternative aim to provide an inhaler, e.g. a variable dose inhaler, with sensing capabilities for a variety of user requirements.

According to a first aspect of the technology, there is provided an inhaler configured to dispense a dosage of one or more inhalable therapeutic agent to a user, the inhaler comprising a sensor to detect one or more biomarker present in gases expired by the user.

The sensor output may be used to determine a dosage volume or frequency for dispensing by the inhaler. The dosage may be varied/personalised according to the presence and/or concentration of said one or more biomarkers. The inhaler may comprise a variable dose mechanism and a controller arranged to control the dosage dispensed to the user. The controller may determine the dosage based at least in part upon the output of the sensor.

The sensed biomarker may be selected from: ketone; volatile organic compounds (VOC) including saturated and unsaturated hydrocarbons, sulphur-containing VOC and nitrogen-containing VOC; nitric oxide; dimethyl sulphide; ethanol; acetaldehyde; short chain alkanes (e.g. ethane and/or pentane); cyclic monoterpenes, e.g. limonene. Preferably the biomarker may be nitric oxide.

The sensor may be configured to determine a concentration of said one or more biomarker, including the fractional concentration of exhaled nitric oxide (FeNO).

Said dosage of one or more inhalable therapeutic agent may be dispensed into a flow passage of the inhaler. The flow passage may be within the inhaler. The sensor may be configured to detect the presence of said one or more biomarkers in a fluid flow within the flow passage. The user may therefore inhale medicament and exhale to use the sensor via the same flow passage.

Said sensor may be fluidly connected to the flow passage via a fluid channel. The flow passage may comprise a mouthpiece. The flow passage may comprise a dispensing outlet or nozzle. The flow passage may comprise an outlet such that, expiratory gases from the user enter the inhaler via the mouthpiece and exit the inhaler via the outlet. The outlet may be provided downstream of the mouthpiece and/or nozzle during user exhalation.

The flow passage may be integrally formed within the inhaler. The therapeutic agent may be dispensed via an outlet or nozzle. A closure/shield may be provided to selectively obscure the outlet or nozzle. The closure may rotatable or pivotable into/out of engagement with the outlet or nozzle. The closure may seal against or abut the outlet or nozzle. The closure may be configured to abut against the outlet/nozzle during exhalation by the user.

Said flow passage may comprise a flow diverter configured to at least partially obscure the flow passage, such that at least a portion of exhaled air is diverted to the sensor. The flow diverter may at least partially obscures the flow passage in a deployed condition, wherein the flow diverter is selectively actuatable between a retracted condition and said deployed condition.

The inhaler may comprise a filter for the expiratory air of the user. The inhaler may comprise a plurality of filters, optionally in series. The filter may comprise a moisture filter, such as a Peltier moisture filter. The filter may comprise a particulate filter e.g. a HEPA filter.

The inhaler may comprise a pump for pumping expiratory gases to the sensor. The inhaler may comprise a further sensor for the expiratory flow and/or an operational condition of the inhaler. The further sensor may comprise an ambient condition sensor, such as a humidity or temperature sensor. The further sensor may comprise a carbon dioxide sensor, a carbon monoxide sensor or other expiratory or inspiratory flow content sensor.

The further sensor may comprise a flow rate or pressure sensor, e.g. a spirometry sensor. The further sensor may comprise a dosage counter, dosage frequency sensor or dosage interval sensor.

The sensor may be removably attached to the inhaler. The sensor may be provided within a removable module. The sensor may be attached to the inhaler adjacent the flow passage. The sensor may be attached to the inhaler at a lower end thereof. The sensor may be attached to the inhaler a position removed the mouthpiece (i.e. such that the mouthpiece is not obstructed).

The sensor may be provided within a sensor module having one or more further components for cooperation with the operation of the sensor, the sensor module being removably attachable to the inhaler without preventing the dispensing a dosage of one or more inhalable therapeutic agents by the inhaler. The sensor may be provided as part of a sensor system, the inhaler further comprising a controller for: monitoring operation of the sensor system; controlling operation of a dose delivery mechanism of the inhaler; and/or analysing an output of the sensor.

According to a further aspect, there is provided a method of determining a personalised dosage for a variable dose inhaler comprising: detecting one or more variables using a sensor provided on the inhaler; analysing input parameters, and adjusting the personalised dosage delivered to the subject according to the one or more variables and/or input parameters.

The method may be performed by a controller of the inhaler or a further processor/computer arranged to communicate with the controller of the inhaler.

The inhaler may comprise a data store, such as a non-volatile memory device, and a log of historic sensor readings may be maintained on the data store. There may be provided a method or system for monitoring usage of the inhaler by accessing the data store.

One or more variables or input parameters used by the method or inhaler for setting a variable dose may comprise:

- dose and frequency of inhaled therapies over the preceding number of days, e.g. seven or more,
- one or more environmental condition
- any training mode data as described herein
- measured flowrate using integral flow meter (spirometry), such as FEV1, or PEF, or a parameter derived therefrom
- data from biomarker analysis, e.g. where the biomarker comprises FeNO.

According to a further aspect of the technology, there is provided a training system for an inhaler comprising: a sensor to detect an inhaled flow profile of the user; and a feedback system to provide one or more instructions to the user based the flow profile.

According to a further aspect, there is provided an inhaler configured to dispense a dosage of one or more inhalable therapeutic agent to a user, the inhaler comprising: a sensor to detect a mechanical input by the user upon the inhaler during use; a controller arranged to compare said detected mechanical input with a predetermined value of mechanical input, where based upon said comparison the controller controls dosage delivery to the user and/or controls output of user feedback via a user feedback device of the inhaler.

The controller may control a training or testing mode of the inhaler. The controller may provide feedback indicative of a mismatch or difference between the detected mechanical input and the predetermined value of mechanical input.

The controller may selectively permit or denies delivery of a dosage to the user based upon the comparison. The inhaler may comprise a valve for selectively inhibiting delivery of the therapeutic agent to the user and the controller controls operation of said valve. The valve may obscure a delivery nozzle.

The controller may vary a timing of the dosage delivery relative to a timing of the detected mechanical input. The controller may vary a volume of the dosage delivered to the user based upon said comparison.

The sensor may comprise a flow or pressure sensor for an inspiratory or expiratory flow applied by the user. The sensor may monitor a magnitude and/or duration of the inspiratory or expiratory flow. The sensor may comprise an orientation/tilt or agitation/inertia sensor. The controller may monitor a flow profile and selects a point or magnitude value of said profile for the comparison.

The inhaler may comprise a flow passage having a mouthpiece and an outlet such that expiratory gases from the user enter the inhaler via the mouthpiece and exit the inhaler via the outlet, where said flow passage comprises a flow diverter or closure configured selectively to at least partially obscure the flow passage, the controller controlling actuation of the closure and/or monitoring a status of the closure.

The user feedback device may comprise a user interface or display and the feedback comprises qualitative or quantitative information about the user's usage of the inhaler.

The sensor may comprise a spirometry sensor (e.g. a flow meter). The sensor may be located within a linear flow passage extending between a mouthpiece on a front side of the inhaler and an outlet on a rear side of the inhaler.

The inhaler may comprise a data store (e.g. memory) for maintaining a historic log of sensor readings, said log being accessible to provide a log of inhaler usage by the user.

According to a further aspect there may be provided a controller for an inhaler according to any previous aspect, where the controller is arranged to receive an inhaler sensor signal and to control dosage delivery to the user and/or control output of user feedback in dependence upon said sensor signal.

According to a further aspect of the technology, there is provided: a variable dose inhaler comprising: a reservoir comprising a product to be dispensed; a dosing chamber having an internal volume configured to contain a predetermined dose of the product, the dosing chamber having an actuatable wall for varying the internal volume of the dosing chamber; an outlet for delivering the predetermined dose of the product from the dosing chamber to a user; a valve mechanism having a valve member actuatable between a first position for fluid communication from the reservoir to the dosing chamber and a second position for fluid communication from the dosing chamber to the outlet.

The predetermined dose/internal volume may continuously variable (i.e. may take any value between a minimum and maximum value).

The valve member may inhibit fluid communication from the inlet to the dosing chamber in the second position and/or inhibits fluid communication from the dosing chamber to the outlet in the first condition.

The valve member may be rotatable between the first and second positions. The valve member may be circular/cylindrical. The valve member may rotate about a vertical axis is use. The valve member may rotate about a horizontal axis is use.

The valve member may be mounted within a valve casing/block, the valve casing having separate openings for fluid communication with each of the reservoir, the dosing chamber and the outlet, the valve member being selectively actuatable to occlude one said opening whilst permitting fluid communication between the other two said openings.

The valve member may comprise a plurality of said channels defining discrete fluid pathways. The channel may extend though/across the valve member. The channels may be spaced. The channels may be angled.

The first fluid pathway may fluidly connect the inlet and the dosing chamber when the valve member is in the first position, and the second fluid pathway connects the outlet and the dosing chamber when the valve member is in the second position. The valve member may be actuatable to a third position where the dosing chamber is closed from both the reservoir and the outlet.

The valve member may be interposed an airway and the reservoir. The airway may be interposed the valve member and the reservoir. The reservoir may be mounted vertically above the valve member in use, such that medicament flows into the dosing chamber under the action of gravity.

The valve member may comprise a rotatable member configured to abut and/or seal against a stationary member. The stationary member and the rotatable member may be coaxial. The valve member and/or the stationary member may comprise a disc. The valve member and/or the stationary member may comprise a low friction material. The valve member and/or the stationary member may comprise a ceramic and/or ceramic coating.

The dosing chamber may be movable/translatable. The valve member and the dosing chamber may be mounted to a movable carriage. The carriage may be movable toward/away from an airway and/or reservoir. The carriage may be movable in at least two directions. The dosing chamber/carriage may be movable between a first position where the dosing chamber is fluidly connected to a reservoir and a second position when the dosing chamber is fluidly connected to an airway/mouthpiece.

A first fluid pathway of the valve member may fluidly connect the inlet and the dosing chamber when the valve member is in the first position, and a second fluid pathway of the valve member may connect the outlet and the dosing chamber when the valve member is in the second position.

The reservoir may comprise a detachable canister. A valve of the reservoir may engage the valve casing such that the valve is in an open position during engagement therewith.

A sealing member may be provided between the valve member and the valve casing. The outlet may comprise a nozzle and/or a sump. The nozzle may comprise an atomising nozzle. The nozzle may extend into a fluid pathway operatively connected to a mouthpiece.

The actuatable wall of the dosing chamber may comprises a piston, the piston movable along the dosing chamber to vary the internal volume of the dosing chamber. The piston may be linearly moveable. The piston may comprise a tapered head. The head may correspond to the shape of the dosing chamber outlet. The piston may seal against the outlet and/or the valve member. The piston may extend into the valve member in an extended state. The piston may comprise a resiliently deformable head.

The actuatable wall may be actable via a screw thread or worm drive. The actuatable wall may be actable via a motor. The actuable wall may be at partially surround by a wall/sleeve/collar. The actuable wall may comprise a engagement member configured to be received within a track in wall/sleeve/collar such that relative rotation therebetween provides axial movement of the actuable wall. The engagement member may comprise a pin or detent. The engagement member may comprise a screw thread. The track/engagement member may be helical.

The dosing chamber may be rotatable. The valve member may be fixed/mounted to the dosing chamber. The valve member may be supported on a bearing. The bearing may comprise a thrust bearing.

The actuatable wall of the dosing chamber may be moveable during filling of the dosing chamber from the reservoir and/or during dispensing of the dose from the dosing chamber to the outlet. The actuatable wall may be configured to move to increase the internal volume of the dosing chamber when the valve member is in the first position and/or to decrease the internal volume of the dosing chamber when the valve member is in the second position.

The inhaler may comprise a controller arranged to control movement of the actuatable wall in use during flow to/from the dosing chamber. The controller may determine a stop position for the actuatable wall of the dosing chamber corresponding to the internal volume for the predetermined dose. The controller may be arranged to control movement of the actuatable wall in use during flow to/from the dosing chamber.

The controller may determine a stop position for the actuatable wall of the dosing chamber corresponding to the internal volume for the predetermined dose. The controller may control movement of the actuatable wall according to a reading from the one or more said sensors.

The inhaler may comprise one or more sensors and the controller controls movement of the actuatable wall according to a reading from one or more said sensors.

The inhaler may comprise a second valve member, the second valve member configured to selectively obscure the outlet. The second valve member may extend across or into a fluid pathway. The second valve member may pivotable/rotatable. The second valve member may engage the sump/nozzle. The second valve member may be provided adjacent the mouthpiece.

The inhaler may comprise a data store, e.g. a memory, comprising a record of the volume/mass of therapeutic agent dispensed and/or a time at which the therapeutic agent is dispensed. The memory may comprise a record of the volume of therapeutic agent in the reservoir and the controller may be arranged to record the volume dispensed for each dose to the user. The controller may maintain a log of remaining volume of the product in the reservoir after each instance of use of the inhaler. The inhaler may provide an indication of the volume dispensed, the volume remaining in the reservoir and/or the number of doses remaining in the reservoir.

The product may comprise a therapeutic agent and/or medicament. The inhaler may comprise a reservoir configured to contain a therapeutic agent for inhalation by the user; and a valve movable between a first configuration configured to prevent release of the therapeutic agent from the reservoir and a second configuration configured to release a dose of the therapeutic agent from the reservoir in response to the detection of the mechanical input by the sensor.

According to a further aspect of the technology, there is provided: an inhaler comprising: an electronic air pressure or flow rate sensor configured to detect a pressure drop or flow due to inhalation by the user; a reservoir configured to contain a product for inhalation by the user; and a valve movable between a first configuration configured to prevent release of product from the reservoir and a second configuration configured to release a dose of the product from the reservoir in response to the detection of the pressure drop or increased flow rate by the sensor.

The reservoir may comprise a canister. The canister may or may not be metallic. The canister may be removable/detachable from the inhaler. The valve may be provided on, within or otherwise incorporated with the canister. The valve may comprise a metered dose valve. The valve may comprise a movable stem. The stem may be resiliently biased toward a closed position.

An actuation mechanism may actuate the valve mechanism. The actuation mechanism may comprise a breath actuated mechanism (BAM). The actuation mechanism may be electronically controlled. The actuation system may be configured to operate in response to detection of inhalation by the user. The actuation mechanism may be mounted to or otherwise form part of the inhaler. The actuation mechanism may comprise an electric motor and/or solenoid. The actuation mechanism may comprise an eccentrically rotatable cam.

The reservoir or canister may be biased toward a dispensing condition (e.g. toward an open state of the valve). The reservoir or canister may be biased toward an air passage and/or sump. The reservoir or canister may be biased toward depression of the stem. The bias may be provided via a biasing mechanism. The reservoir or canister may be mounted on/within a carriage or sleeve. The carriage may be interposed the canister and the biasing mechanism. The actuation may engage the sleeve.

The controller may trigger selective movement of the valve to the second configuration only if a pressure drop or flow rate applied by inspiration or expiration of the user meets a predetermined criterion. The criterion may comprise a predetermined magnitude of change in pressure and/or flow rate. The criterion may comprise a predetermined magnitude of change in pressure and/or flow rate across a predetermined time period.

The controller may only trigger movement of the valve to dispense the dose of therapeutic agent once a delay has elapsed after first sensing a pressure drop or flow rate that meets the predetermined criterion. The delay may occur after an initial change in pressure and/or flow rate

The inhaler may comprise a notification device and the controller outputs a notification via said notification device to the user in the event that the mechanical input does not meet the predetermined criterion. The notification device may notify the user of the magnitude or duration of a pressure drop or flow rate achieved during inhalation or expiration by the user.

The inhaler may comprise a dosing chamber, the dosing chamber being filled with a predetermined dose to prime the inhaler prior to sensing the mechanical input and/or prior to movement of the valve to the second configuration.

The dosing chamber may comprise a variable volume dosing chamber.

The notification device may comprise a visual, audible and/or haptic indicator. The notification device may comprise a visual display (e.g. an LCD display). The inhaler may comprise indicator means for indicating the inhaler is primed. The inhaler may comprise a testing or training mode whereby the inhaler is used to sense a mechanical input achieved by a user without triggering movement of the valve.

The inhaler may be configured to sense and/or record flow rate and/or pressure drop or other mechanical input by the user, e.g. as part of a log of inhaler usage. A plurality of variables may be recorded for each usage event, such as dosage volume, time and one or more mechanical input by the user.

The inhaler may comprise an outlet for delivering the dose of the product from the reservoir to a user, and a second valve member, the second valve member configured to selectively obscure the outlet.

The second valve may open in response to priming of the inhaler and/or sensing of the mechanical input by the user

According to a further aspect of the technology, there is provided: an inhaler comprising a reservoir configured to contain a product for inhalation by the user and an outlet for delivery of the product from the reservoir to a user's airway, wherein the inhaler comprises a sensor for sensing agitation of the product in the reservoir.

The sensor may comprise an accelerometer and/or inertia sensor.

The inhaler may comprise a dispensing system, the dispensing system configured to dispense a dose via the outlet only in the event that the sensed agitation meets a predetermined criterion. The criterion may comprise one or more of a magnitude of agitation, a frequency/rate of agitation, a duration of agitation and/or a direction of agitation. The criterion may comprise a threshold for magnitude, frequency, duration and/or direction of agitation over or within a predetermined time period.

The controller may be arranged to process the sensor output to determine whether the sensed agitation meets the predetermined criterion and to selectively trigger delivery of the product from the reservoir to the user's airway based upon said determination.

The inhaler may comprise a tilt sensor, the controller configured to dispense a dose of the therapeutic agent when the inhaler is within a predetermined orientation range. The orientation range may be +/- 20 degrees from a predetermined orientation, preferably, +/-20 degrees. The predetermined orientation may be such that a mouthpiece end of the inhaler is arranged in vertically downward position.

The inhaler may comprise a variable dose dispensing system. The reservoir/canister may comprise a computer-readable memory device for identifying the reservoir/canister.

According to a further aspect of the technology, there is provided: a canister for an inhaler, the canister comprising a product to be dispensed during use when mounted to an inhaler and a valve for controlling release of the product, wherein the canister comprises an agitation mechanism, the agitation mechanism arranged to be driven to agitate the product within the canister.

The agitation mechanism may comprise a member mounted within the product in the canister on a drive member arranged to impart a predetermined motion to the member.

The agitation mechanism may comprise a stirring mechanism. The agitation mechanism may comprise an impeller or paddle. The canister may comprise a rotor and a shaft for driving rotation of the rotor.

The shaft may be supported by opposing sides of the canister. The shaft may extend between opposing upper and lower walls of the canister, e.g. being received in the upper and lower walls at opposing ends of the shaft. The shaft may comprise a spindle. The impellor or paddle may extend only part way along the length of the spindle. The impellor or paddle may extend only part way along the height of the canister.

The canister may comprise a computer-readable memory device for identifying the canister.

The canister/inhaler may comprise a motor configured to drive the agitator, the agitator releasably connectable to at least a portion of the motor.

The agitator may form part of an electric motor. The agitator may form rotor part of an electric motor. The agitator may comprise a magnet. The agitator may comprise a material configured to create an induced magnetic field. The agitator may comprise a rotor portion configured to be received within a stator portion on the inhaler. The stator portion may be fixed to the inhaler.

An inhaler may comprise the reservoir containing a product to be dispensed and the inhaler comprises a drive mechanism or power source for actuation of the agitator.

According to further aspect of the technology, there is provided: an inhaler comprising a reservoir containing a therapeutic agent to be dispensed, an outlet for dispensing the therapeutic agent to a user and a dosing chamber arranged to hold a predetermined amount of therapeutic agent from the reservoir for delivery of a dose of the therapeutic agent to the outlet; wherein the inhaler comprises a controller and a computer-readable memory device for maintaining a log of the doses delivered to the user and/or the remaining amount of therapeutic agent in the reservoir.

The predetermined amount may comprise a predetermined mass or volume of the therapeutic agent. The inhaler may subtract the predetermined volume of the dosing chamber from a log of volume in the reservoir each time the therapeutic agent is dispensed.

The controller may maintain a log of dose count remaining in the reservoir. The controller may determine the dose count from the determination of the volume of therapeutic agent remaining in the reservoir. The controller may divide the remaining volume value by the value of the predetermined volume of therapeutic agent held by the dosing chamber. The controller may maintain a log of the volume of therapeutic agent dispensed for each dose, e.g. as well as a dose count.

The reservoir may comprise the computer readable memory device and/or the log of remaining volume (i.e. the remaining number of does and/or remaining mass/volume is recorded on the reservoir). The reservoir may be a removable/ replaceable reservoir, the controller comprising a reader/writer for the memory device of the reservoir. The controller may maintain a complete log of each dose and remaining volume starting with an initial volume of the reservoir. The log may be readable from the memory device of and/or the inhaler comprises a transmitter for transmitting the log to a third party.

A variable dose inhaler may comprise the reservoir.

According to a further aspect of the technology, there is provided: a canister for use with an inhaler, the canister comprising a computer-readable memory device comprising any or any combination of: the name of a product or products contained within the canister; the name of one or more active ingredients of the product contained within the canister; a predetermined dose or dose range of one or more of the products or ingredients contained within the canister; the total volume of product within the canister; the number of doses of the product contained within the canister.

The memory device may comprise security data configured to validate the authenticity of the canister to an inhaler device.

The memory device may comprise: a value of number of doses or volume of product dispensed by the canister; and/or a value of number of doses or volume of product remaining in the canister.

The canister may comprise a controller for reading/writing to the memory device or arranged to be in communication with a controller of an inhaler for reading/writing to the memory device.

According to a further aspect of the technology, there may be provided a method of monitoring a patient comprising using the inhaler according to any of the preceding aspects and accessing the log maintained by the controller.

According to a further aspect of the technology, there may be provided a method of treatment of a patient using the inhaler according to any of the preceding aspects, the method comprising accessing the log maintained by the controller and determining a dosage or regimen for treatment of the patient based upon the log. The method may comprise implementing the dosage or regimen using the inhaler.

Determining or varying a dosage or regimen may comprise varying a volume or mass of therapeutic agent delivered to a patient per individual dose by the inhaler. Additionally or alternatively it may comprise determining or varying the frequency with which individual doses may be dispensed by the inhaler.

Any optional feature defined herein in relation to any one aspect may be applied to any further aspect wherever practicable. Any reference to an inhaler herein may be reference to a personal handheld inhaler, i.e. a self-contained and portable device having/receiving a product reservoir/canister and mouthpiece for inhaling individual doses of a product in the reservoir by an end user. As used herein, the term "user", "patient", "subject" or variants thereof, are used interchangeably throughout.

Detailed Description

**Figure 1** shows a perspective view of an inhaler device and a sensor module;

**Figure 2** shows a first perspective view of the inhaler device;

**Figure 3** shows a second perspective view of the inhaler device;

**Figure 4** shows a perspective view of the inhaler device with a cap in an open position;

**Figure 5** shows a perspective view of the inhaler device with a closure in an open position;

**Figure 6** shows a first perspective view of the inhaler device with an outer housing removed;

**Figure 7** shows a second perspective view of the inhaler device with the outer housing removed;

**Figure 8** shows a side sectional view of the inhaler device;

**Figure 9** shows a perspective sectional view of the inhaler device;

**Figure 10** shows a first close-up sectional view of a variable dose valve mechanism in a first usage condition;

**Figure 11** shows a second close-up sectional view of the variable dose valve mechanism in a second usage condition;

**Figure 12** shows a third close-up sectional view of the variable dose valve mechanism in a third usage condition;

**Figure 13** shows a fourth close-up sectional view of the variable dose valve mechanism in a fourth usage condition;

**Figure 14** shows a fifth close-up sectional view of the variable dose valve mechanism in a fifth usage condition;

**Figure 15** shows a sixth close-up sectional view of the variable dose valve mechanism in a sixth usage condition;

**Figure 16** shows a schematic view of the operation of a validation system

**Figure 17** shows a perspective view of a canister;

**Figure 18** shows a first close-up sectional view of the canister;

**Figure 19** shows a second close-up sectional view of the canister;

**Figure 20** shows a close-up sectional view of the air channel of the inhaler;

**Figure 21** shows a third perspective view of the inhaler device with the outer housing removed;

**Figure 22** shows a perspective view of the sensor module;

**Figures 23a-c** show a schematic view of the sensor module;

**Figure 24** shows a flow chart of the operation of the sensor module;

**Figure 25** shows a flow chart of the operation of a spirometry system;

**Figure 26** shows a schematic view of a flowrate vs time graph;

**Figure 27** shows a schematic view of a volume vs time graph;

**Figure 28** shows a flow chart of the operation of a dosage adjustment system;

**Figure 29** shows a flow chart of the operation of a training system;

**Figure 30** shows a first perspective view of a partially dissembled inhaler according to a second embodiment of the inhaler;

**Figure 31A** shows a second perspective view of the partially dissembled inhaler according to the second embodiment of the inhaler;

**Figure 31B** shows a perspective view of a piston assembly;

**Figure 31C** shows a perspective view of a carriage assembly;

**Figure 32** shows a first sectional side view of the second embodiment of the inhaler;

**Figures 33 and 34** shows a sectional side view of the piston assembly;

**Figure 35-39 and 41** shows a second sectional side view of the second embodiment of the inhaler during an operation sequence thereof;

**Figures 40A and 40B** show schematic diagrams of a piston discharge rate;

**Figure 42** shows a first perspective view of a partially dissembled inhaler according to a third embodiment of the inhaler;

**Figure 43** shows a second perspective view of the partially dissembled inhaler according to the third embodiment of the inhaler;

**Figures 44-49** shows a second sectional side view of the third embodiment of the inhaler during an operation sequence thereof;

**Figures 50-52** show a perspective view of a valve arrangement of the third embodiment of the inhaler.

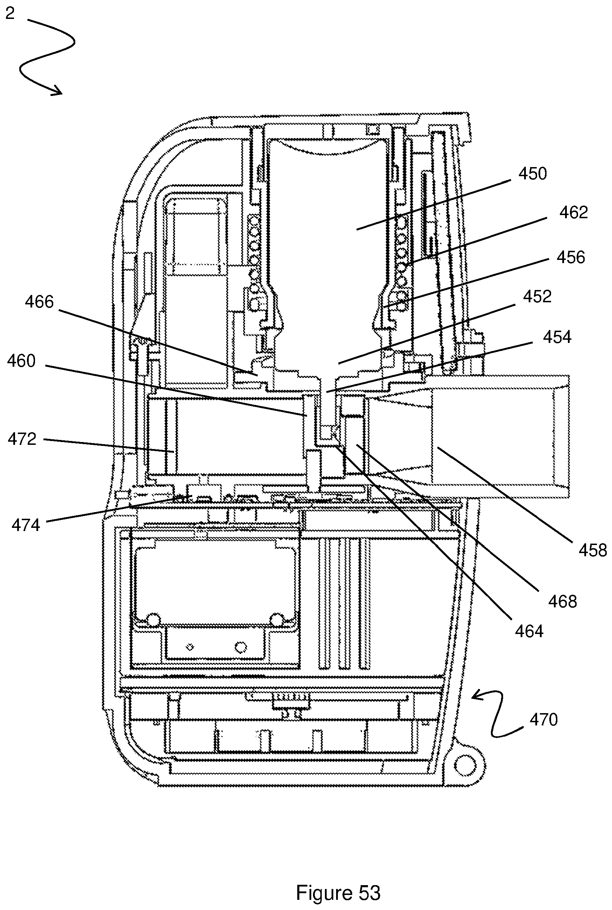

**Figure 53** shows a sectional side view of a first metered dose inhaler;

**Figure 54** shows a sectional side view of a second metered dose inhaler.

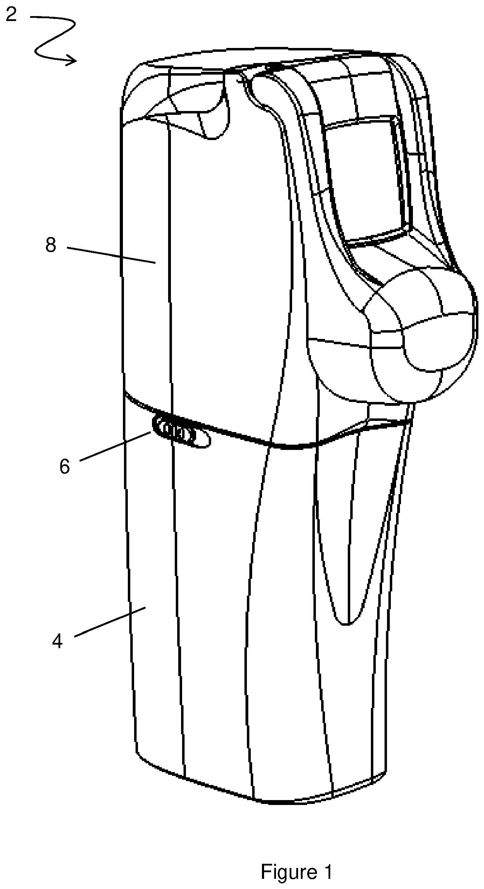

An inhaler device 2 is shown in figures 1-5. The inhaler 2 is configured to dispense a dose of medicament to a patient's airways via inhalation of the user. The inhaler device 2 is configured to dispense any suitable medicament, for example, a dry powder (e.g. DPI), suspended power, liquid (e.g. pMDI), or gas.

The inhaler 2 comprises a sensor module 4. The sensor module 4 may be selectively attachable to the inhaler 2. The sensor module 4 may therefore be detached from the inhaler 2 when not required.

The sensor module 4 in this example is attachable to the inhaler via a releasable attachment means, for example, a release latch or clamp. A release mechanism 6 is provided on an external portion of the sensor module 4 to allow the user to manually detach the sensor module 4. The release mechanism may comprise a slide latch. Operation of the sensor module 4 will be described in detail later.

The inhaler 2 comprises a housing 8. The housing encloses/encases the inhaler 2, thereby protecting the internal components of the inhaler 2 from interference by the user or contamination. The housing 8 comprises a polymeric material (e.g. a rigid polymer). Suitable polymeric materials include those that provide a high surface quality, dimensional stability, hear resistance, low electrostatic potential and high robustness, such as acrylonitrile butadiene (ABS), methacrylate acrylonitrile butadiene (MABS), polypropylene (PP), polycarbonate (PC), and such like. The housing 8 may therefore be easy to clean. The housing 8 may be moulded such as injection-moulded. The housing 8 may be formed from a single, moulded piece and may have a cap/closure attached thereto as will be described below. The housing 8 may be transparent or translucent.

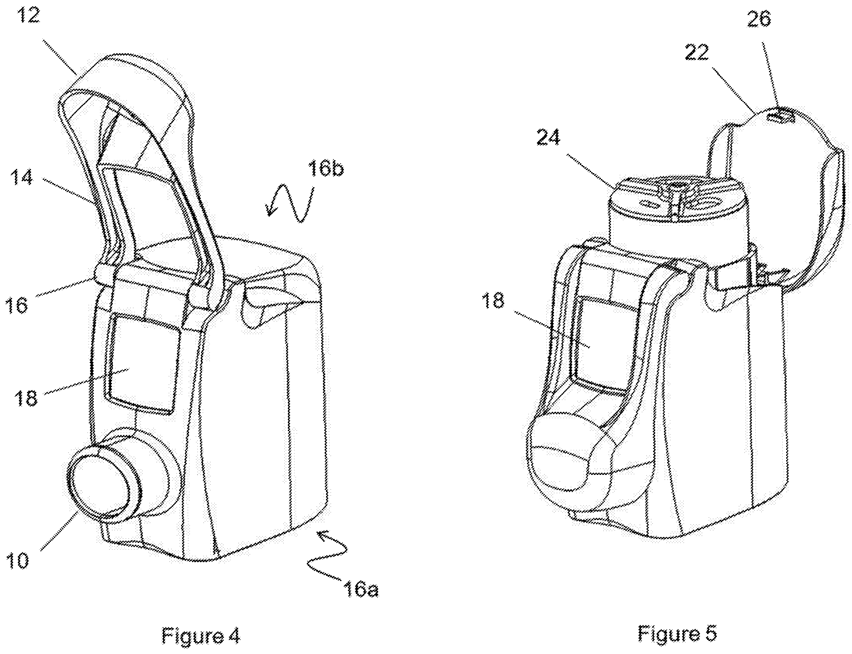

The inhaler 2 comprises a mouthpiece 10 (see figure 4), from which a dose of medicament is dispensed in use. The mouthpiece 10 may be ovular or otherwise shaped for insertion into a user's mouth. The mouthpiece is provided toward a lower end 16a of the inhaler. The mouthpiece protrudes forwardly from the housing 8. The mouthpiece 10 may be transparent or translucent.

The inhaler 2 comprises a cap 12 to cover/enclose the mouthpiece 10. The cap 12 is rotatably/pivotably mounted to the inhaler 2. This prevents accidental loss of the cap 12. The cap 12 is connected to the inhaler 12 via a plurality/pair of arms 14. The arms are attached to the housing 8 via respective hinges 16. The cap 12 may therefore swing away from the mouthpiece 10. The hinges are provided at an upper end 11b of the inhaler 2, such that the cap 12 rotates upwards and away from the mouthpiece 10. The cap can be rotated by at least 180°, e.g. away from a user's face when using the inhaler. The cap 12 may be transparent or translucent.

An electronic display 18 is provided. The display 18 may provide information for the user, as will be described later, e.g. in the form of a display screen for presenting visual, graphic and/or textual information. The electronic display 18 may comprise any conventional display technology, for example, LCD, TFT, LED, OLED technology etc. The display 18 is provided above the mouthpiece 10 (i.e. between the mouthpiece 10 and the upper end 16b). The display 18 therefore faces the user in use. The arms 14 of the cap 12 are provided either side of the display 18, and thus do not obscure the display when the cap 12 is closed.

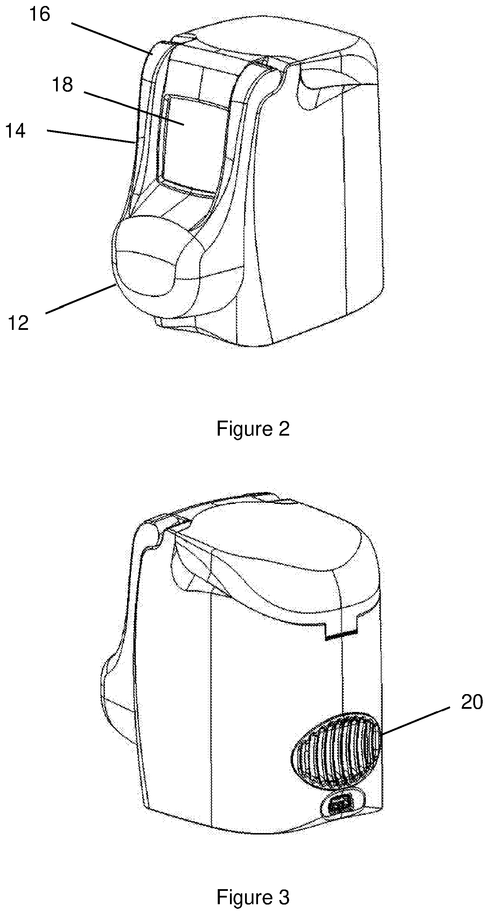

The housing 8 comprises an air outlet 20 (see figure 3). The air outlet 20 is provided at an opposing side of the housing 8 to the mouthpiece. If the mouthpiece is defined as protruding from the front of the device, the air outlet 20 is thus on the rear of the device. The air outlet may be of the same shape as the mouthpiece and/or may be aligned with the mouthpiece, i.e. arranged on a common axis with the mouthpiece. The air outlet 20 comprises a grating/grille or the like in this example. Although, described as an outlet, it can be appreciated that air may pass either direction through the outlet 20.

As shown in figure 5, the inhaler 2 comprises an openable closure 22. The closure 22 allows insertion of a medicament canister 24. The canister 24 provides a reservoir for storing a medicament on the device. The closure is provided at the upper end 16b of the inhaler 2. The closure 22 comprises a latch 26 configured to hold the closure in a closed condition with the housing 8, e.g. using a snap-fit or clip-fit engagement.

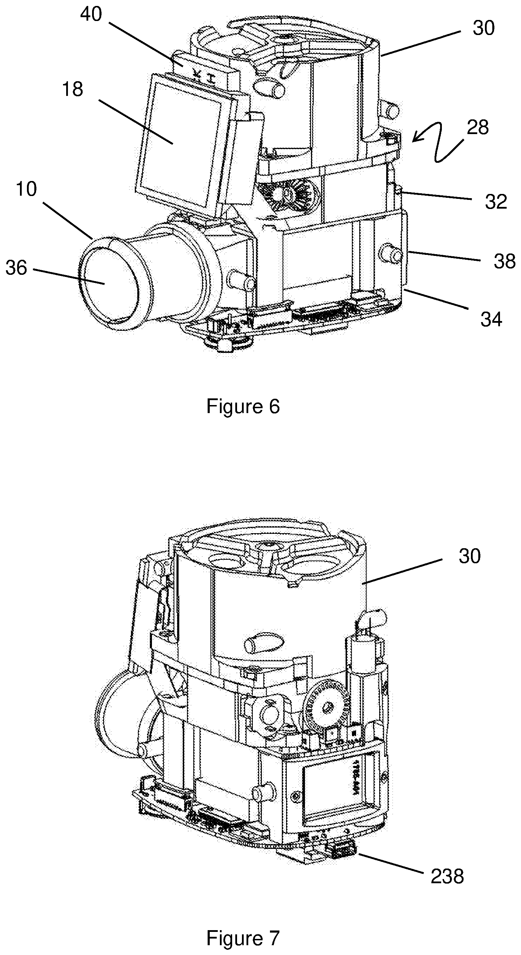

The internal portion of the inhaler 2 is shown in closer detail in figures 6 to 9. The inhaler comprises a chassis 28 to support or otherwise hold the internal components in place. The chassis 28 comprises a canister holder 30 to support the canister 24. The canister holder 30 may comprise a retaining means to retain the canister 24 in position. For example, the retaining means may comprise a releasable latch or the like. Alternatively, the canister 24 is held in place by the closure 22.

The chassis 28 supports a variable dose valve mechanism 32, operation of which will be described later.

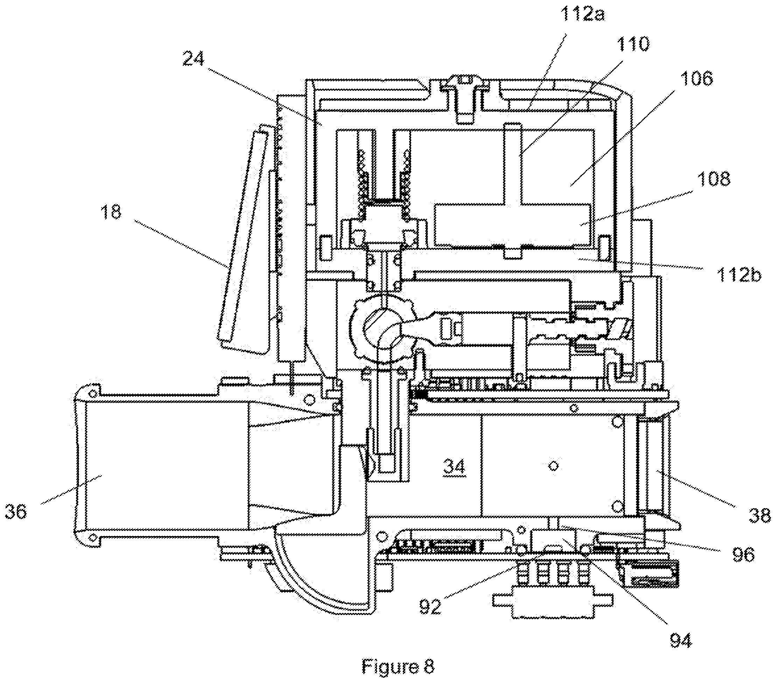

As best shown in figure 8, the chassis 28 provides a channel 34. The channel 34 extends between a first orifice 36 provided by the mouthpiece 10 and a second orifice 38 provided on an opposing side of the inhaler 2. The channel 34 may be provided proximal the lower end 16a of the inhaler 2. The channel 34 thus extends through the inhaler 2 (i.e. from one side of the inhaler 2 to an opposing side thereof). The second orifice 38 is aligned with the air outlet 20 provided in the housing 8. The channel 34 is substantially straight/linear and/or of constant cross-sectional profile along its length (i.e. there is line of sight through the inhaler along an axis between orifices 36 and 38). Thus, any air passing through the channel 34 travels in a substantially linear path.

The inhaler comprises a battery 40. The battery 40 may provide power for the display 18 and/or other electronic components. The battery 40 is located behind the display 18. The battery 40 may be rechargeable. The battery 40 may be rechargeable via a physical and/or wireless interface. The battery 40 may comprise an Li-ion battery. The battery 40 may be removable/exchangeable.

The variable dose valve mechanism 32 is described in detail with reference to figures 8-15. The valve mechanism 32 comprises a valve member 46. The valve member 46 is received within a valve casing or block 48. The valve block 48 encloses/surrounds the valve member 46. A seal 50 is provided between the valve member 46 and the valve block 48.

The valve member 46 has at least one internal channel with inlet and outlet ends. Thus, when the ends of the internal channel are aligned with adjacent flow paths/channels, the internal channel can connect the adjacent flow paths to allow flow there-between through the valve member 46. When either or both ends of the internal channel are offset from the adjacent flow paths the valve member prevents flow between said flow paths. Thus, rotation of the valve member 46 can be used to selectively open or block flow between said adjacent flow paths.

The internal channel turns through an angle within the valve member, e.g. a perpendicular angle. Thus, the valve member 46 can selectively connect different flow paths at different angular orientations depending on the rotational orientation of the valve member.

The valve member 46 is rotatable. The axis of rotation extends in/out of the plane of the page (i.e. about a horizontal axis in use). The valve member 46 is connected to a motor to effect rotation thereof. The motor may comprise a stepper motor (i.e. the valve member 46 may be rotated between predetermined angular positions). Alternatively, the motor may provide continuous movement of the valve member 46.

In some embodiments, rotation of the valve member 46 may be manual. For example, the valve member 46 may be operatively connected to a handle or crank or the like.

The canister 24 comprises an outlet 52 through which a medicament may be released. The outlet 52 is fluidly connected to the valve member 46 via a channel 54, e.g. passing through the seal 50.

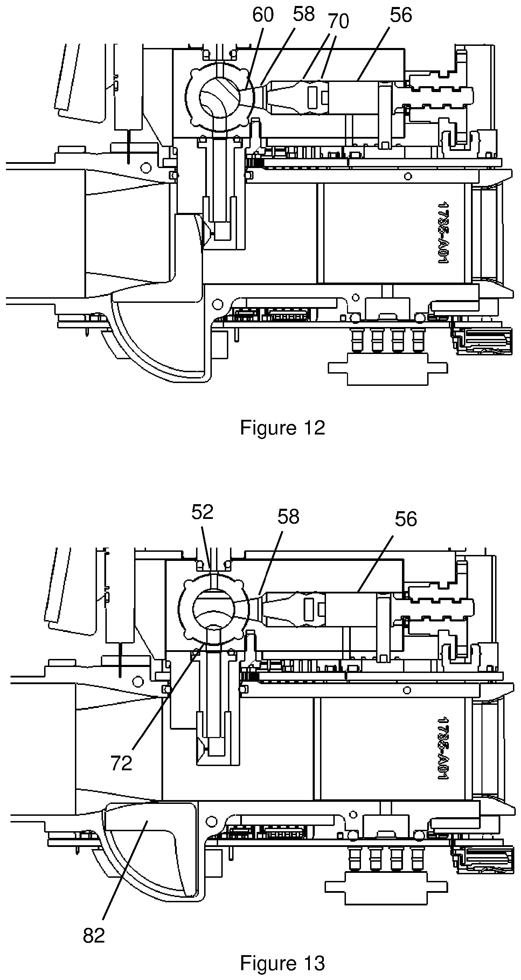

The variable dose valve mechanism 32 comprises a dosing chamber 56. The dosing chamber 56 is provided within the valve block 48. The dosing chamber 56 is configured to provide a predetermined dose of medicament. The dosing chamber is fluidly connected to the valve member 46 via an outlet 58 (see figure 12). A channel 60 passing through the seal 50 fluidly connects the outlet 58 and the valve member 42. The outlet 58 and channel 60 are tapered toward the valve member 42.

The dosing chamber 56 comprises a movable wall configured to vary the effective internal volume of the dosing chamber 56. In the present embodiment, the wall comprises a piston 64. The piston 64 is operatively connected to a motor (not shown) to drive the piston 64 forward/backward within the dosing chamber 56. The piston 64 may be driven via a screw drive 66, e.g. for accurate positioning and movement control of the piston 64, although other drive mechanisms may be provided.

The piston can move between a predetermined set position defining a desired internal volume of the chamber and the end of the chamber adjacent the valve member 46. In this manner the piston can carry out filling and emptying strokes, i.e. movements in opposing directions to/from the end of the chamber. The movement of the piston 64 may be continuous, thus allowing any dose required.

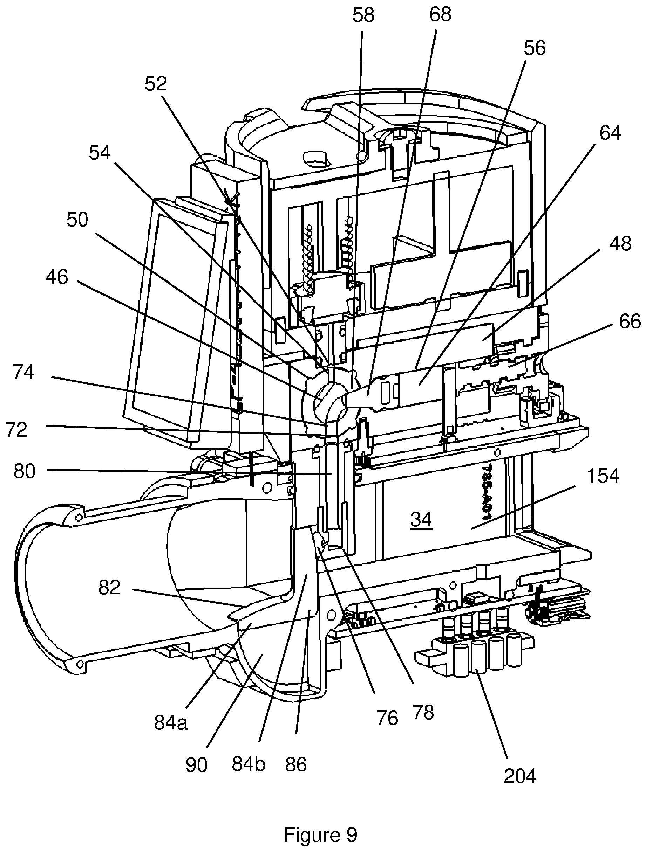

The piston 64 comprises a head portion 68. The head portion 68 is tapered. The head portion 68 therefore corresponds to the shape of the outlet 58 and channel 60. As shown in figures 8 and 9, the head portion 68 extends into the valve member 46 when the piston is in a fully extended state. The piston 64 comprises one or more seals 70 (see figure 12) configured to seal with the side wall of the dosing chamber 56. The seals 70 are provided as an integral part of the head portion 68. The head portion 68 may comprise a resiliently deformable material (e.g. rubber or silicone) to provide a seal against the dosing chamber 56 and/or outlet 58.

The variable dose valve mechanism 32 comprises an outlet 72 configured to dispense the medicament dose in use. The outlet 72 is fluidly connected to the valve member via a channel 74 extending through seal 50. The outlet 72 is fluidly connected to a nozzle 76. The nozzle 76 is provided within the channel 34. Thus, as medicament is dispensed from the nozzle 76, the medicament may be entrained by the air passing through the channel 34 during inhalation of the user. The nozzle 76 may be provided proximal a centre thereof (i.e. proximal the central axis). The nozzle 76 is mounted on a sump 78, which protrudes into the channel 34. A channel 80 extends through the sump 78 to fluidly connect the outlet 72 to the nozzle 76.

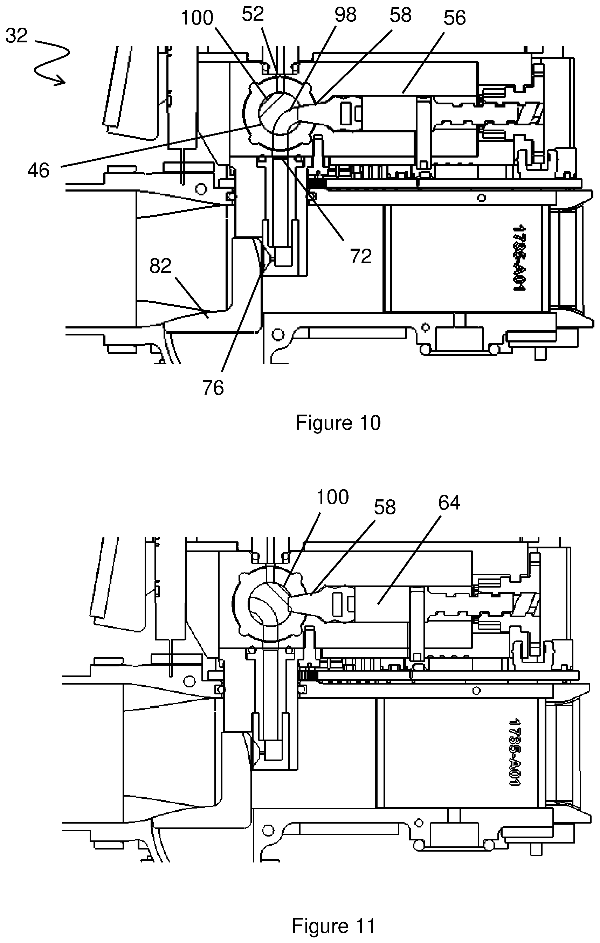

A shield 82 is configured to selectively obscure the nozzle 76. The shield thus provides a valve. The shield 82 is rotatable between a first position where a portion of the shield 82 obscures the nozzle 76 (see figures 9 and 10) and a second position where the shield 82 is displaced away from the nozzle 79 (see figure 13). The shield comprises first and second arms 84a,b. The arms 84a,b are arranged at right angles. The shield 82 is thus L-shaped. The shield 82 is pivotally mounted to the inhaler at the apex 86 thereof. The pivot is provided adjacent the apex 86. The shield 82 is therefore rotatable in and out of the channel 34. A compartment 90 is provided to accommodate the shield 82 in the second position. The compartment may be sector shaped (e.g. a quarter circle).

In the first position, the second arm 84b is projects into the channel 34. The second arm 84b engage the sump and/or the nozzle 76. The first arm 84a is substantially parallel to the side walls of the channel 34. The first arm 84a seals the compartment 90. In the second position, the second arm 84b is substantially parallel to the side walls of the channel 34. The second arm 84b therefore seals the compartment 90. The first arm 84a projects away from the channel 34 and into the compartment 90.

As best seen in figure 9, in the first position, the shield 82 only extends across a portion of the width of the channel 34. The shield 82 therefore does not obstruct the airway through the channel. The shield 82 occupies less than 50%; preferably, less than 40%; preferably less than 30%; preferably, less than 20% of the width of the channel 34. The sump 78 may comprise a same width as the shield. The shield 82 only extends a portion of the height of the channel 34. However, it can be appreciated that the sump 78 partially obscures the channel 34 regardless, and so the shield 82 may extend the full height of the channel 34, substantially affecting the airflow therethrough.

The shield 82 is operatively connected to a motor (shown) to effect rotation thereof.

The inhaler 2 comprises a pressure sensor 92 (see figure 8). The pressure sensor 92 is configured to determine the pressure within the channel 34. The pressure sensor 92 is configured to detect a pressure drop within the channel 34. The pressure sensor 92 may therefore be used to detect inhalation through the inhaler 2 by the user.

The pressure sensor 92 is provided with a chamber 94. The chamber 94 is separated from the channel 34 by the side wall thereof. The pressure sensor 92 is therefore displaced from the channel 34. A channel 96 fluidly connects the chamber 94 with the channel 34. The channel 96 is narrow. For example, the channel 96 may comprise a diameter less than 5mm; preferably less than 3mm. The pressure sensor 92 is provided beneath the channel 34 (i.e. at the lower end 16a of the inhaler 2). The pressure sensor 92 is spaced from the mouthpiece 10 (i.e. provided toward the opposing side of the inhaler 2 from the mouthpiece 10). The pressure sensor 92 is provided upstream of the nozzle 76 during inhalation by the user.

The pressure sensor 92 comprises an electronically readable sensor. The pressure sensor 92 may comprise any suitable pressure sensor or sensor technology, for example, inter alia: piezoresistive strain gauge; capacitive; electromagnetic; piezoelectric; optical; or potentiometric.

Operation of the variable dose valve mechanism 32 will be described with reference to figures 10-15. The variable dose valve mechanism 32 is shown in a "starting" position in figure 10. The valve member 46 is provided in a position (i.e. an angular orientation) such that a first channel 98 in the valve member 46 is aligned with the dosing chamber outlet 58 and the valve outlet 72. The dosing chamber 56 and the nozzle 76 are therefore fluidly connected. The first channel 98 is right-angled. The shield 82 is provided in the first (closed) position.

The canister outlet 52 is aligned with a second channel 100 within the valve member 46. The second channel 100 is obstructed/occluded by the valve seal 50. The canister outlet 52 is therefore fluidly disconnected from the dosing chamber 56 and the valve outlet 72.

In a "filling" stage shown in figure 11, the valve member 46 is rotated to a filling position. In the filling position, the canister outlet 52 and the dosing chamber outlet 58 are fluidly connected via the second channel 100. The canister 24 and the dosing chamber 56 are therefore fluidly connected. The piston 64 is in a forward position, thus preventing flow of medicament from the canister 24 to the dosing chamber 56.

As shown in figure 12, the piston 64 is driven to a predetermined retracted position along the dosing chamber 56.

The vacuum/partial vacuum created by movement of the piston 64 can draw medicament from the canister 24 into the dosing chamber 56. The extent of movement of the piston 64 determines the amount of medicament drawn into the dosing chamber, thus providing a predetermined and multiply/continuously variable dose thereof. The predetermined position is calculated by a controller to achieve a required dosage volume and thus represents a stop position of the piston 64 when retracted.

In a "ready" stage shown in figure 13, the valve member 46 is rotated such the canister outlet 52, the dosing chamber outlet 58 and the valve outlet 72 are mutually disconnected. The valve member 46 occludes the canister outlet 52 and the dosing chamber outlet 58. Both the dosing chamber 56 and the canister 24 are therefore effectively sealed. The dosing chamber 56 is thus primed with the predetermined dose. The shield 82 can be moved to the second (open) position by the motor. The inhaler 2 is now ready to dispense a dose of the medicament.

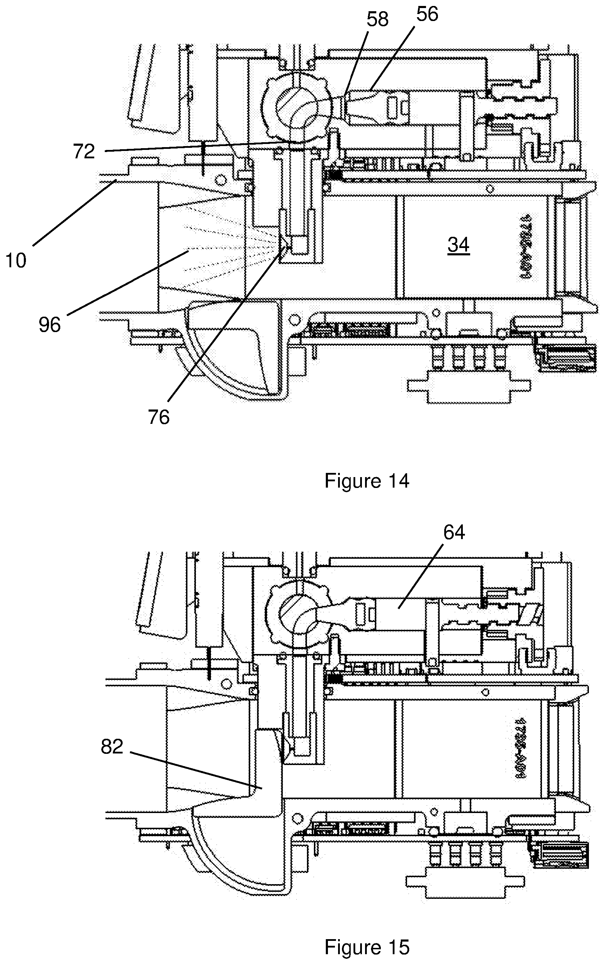

In a "dispensing" stage shown in figure 14, the valve member 46 rotates such that the dosing chamber 56 and the nozzle 76 are fluidly connected. The piston 64 is driven to a forward position, thus expelling the volume of the dosing chamber 56 through the nozzles. The medicament 96 is expelled through the nozzle and into the channel 34. The user inhales, drawing air through the channel 34 and out the mouthpiece 10, thus entraining the medicament 96.

The piston 64 moves to reduce the volume of the dosing chamber 56 to effectively zero, e.g. providing a full ejection stroke and leaving a zero resulting chamber volume. The piston 64 moves into the dosing chamber outlet 58 and seal channel 60. The piston 64 thus expels the full dose of medicament contained within the dosing chamber 56. The piston 64 is actively driven during the dispensing cycle. This ensures a full and accurate dose is dispensed with no dose remaining in the chamber 56. A positive ejection stroke of this kind can help ensure that any residue is removed and promotes the removal of any potential blockages or air pockets in the valve system, e.g. positively displacing them with each and every stroke of the piston.

As shown in figure 15, the piston 64 returns to the "starting" position in preparation for dispensing of the next dose. The shield 82 moves back to the closed position.

A dispensing cycle of the inhaler 2 will now be described. The inhaler 2 is first powered on. In the present embodiment, the inhaler 2 is configured to power on when the cap 12 is open. The inhaler 2 may perform a boot/initiation sequence.

In some embodiments, the user may perform one or more test to determine lung function, details of which will be described later. The user is prompted to select which test they wish to perform. Once the test is complete, the inhaler 2 begins the dispensing cycle.

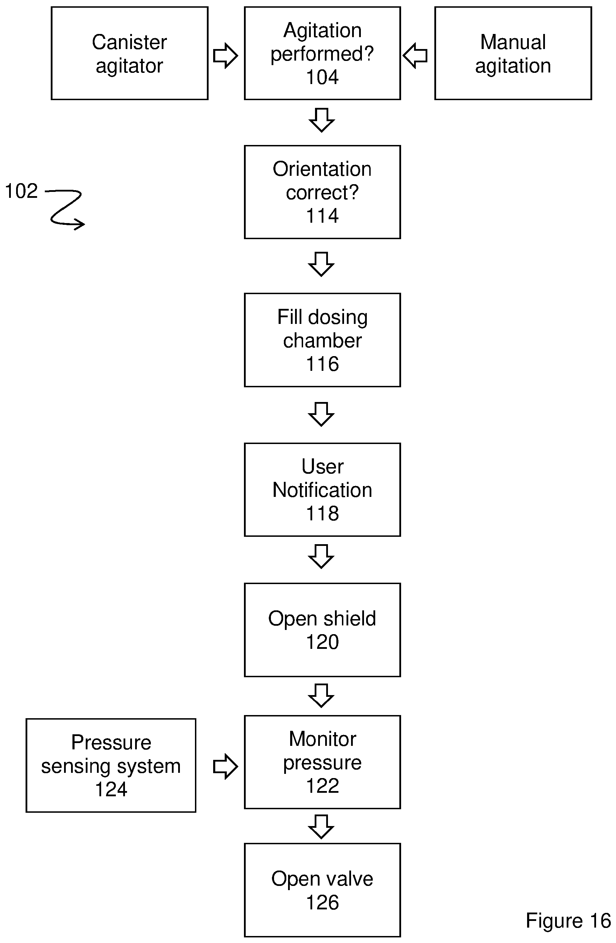

In some embodiments, the inhaler 2 comprises a validation system configured to ensure correct operation of the device during the dispensing cycle. The validation system 102 is shown in figure 16.

In first step 104, the validation system 102 ensures sufficient agitation of medicament is performed.

In a first embodiment, the canister 24 comprises an agitator 106 therein. The agitator 106 is configured to agitate or otherwise disperse and/or mix the medicament therein. This ensures the canister dispenses a homogenous medicament (e.g. where the medicament comprises a suspension). The agitator comprises a plurality of paddles or baffles 108. The paddles 108 are mounted to a spindle rotatably mounted to the canister 24. The paddles 108 are therefore rotatable within the canister 24. The paddles are circumferentially spaced about the spindle 110. Four paddles 108 re provided in the present embodiment. However, it can be appreciated any number of paddles may be provided.

The spindle 110 extends between a top surface 112a and a bottom surface 112b of the canister 24. The spindle therefore rotates about a vertical axis in use. The spindle 110 is offset from the centre of canister 24. The paddles 108 may extend along a portion of the height of the canister 24. For example, the paddles may extend less than 50%; preferably, less than 40% of the height of the canister 24. The paddles 108 are provided adjacent the bottom surface 112b of the canister 24. The paddles 110 therefore help to disperse any solid particulate settled on the bottom surface of the canister 24.

Rotation of the agitator 106 is effected by an electric motor. In the present embodiment, the agitator 106 forms a rotor portion an electric motor. The rotor interacts with a stator mounted to the inhaler to effect rotation of the rotor, and thereby the agitator 106. The electric motor may comprise an induction motor. The rotor may therefore comprise an electrically conductive material configured to generate a reactive magnetic field in response to the applied field from the stator. This provides a simple and "brushless" arrangement, allowing simple disconnection of the canister 24.

In other embodiments, the rotor comprises an electromagnet to provide a DC motor.

The above first embodiment may be used instead of, or in combination with the second embodiment described below. If the second embodiment is used, the first embodiment may or may not be needed.

In a second embodiment, the inhaler 2 comprises a movement sensor. The movement sensor is configured to detect whether the user has manually shaken the inhaler 2, thereby agitating the medicament. The movement sensor may comprise a multi-directional movement sensor, e.g. one or more sensor for detecting movement in different/orthogonal directions. The movement sensor may comprise an accelerometer, inertia/gyroscope sensor, orientation sensor or other displacement sensor. The sensor may determine force/acceleration, magnitude of movement and/or direction of movement.

The validation system 102 may determine sufficient manual agitation based on a number of factors:

- The magnitude of one or more movement/acceleration events
- The absolute number or frequency of one or more movement/acceleration events
- The acceleration profile of one or more movement/acceleration events.
- The direction/velocity of one or more movement/acceleration events.
- The duration of one or more movement/acceleration events.

In a first example, the validation system 102 determines sufficient manual agitation is performed if the magnitude of a single acceleration event (i.e. a hand shake of the user) exceeds a predetermined threshold for one or more of the sensed parameters. For example, if an acceleration of greater than 30ms$^{-2}$ in any single event, then the agitation may be deemed sufficient. Whilst this provides a simple algorithm, a single event may not enough to provide sufficient agitation of the medicament.

In a second example, the validation system 102 determines sufficient manual agitation is performed if the magnitude of a plurality of acceleration events exceed a predetermined threshold a predetermined number of times within a predetermined time period. For example, if an acceleration of greater than 30ms$^{-2}$ is detected 5 times within a 5 second period, then the agitation is deemed sufficient.

In a third example, each acceleration event is assigned a weighting according to their magnitude. If the sum of the respective weightings exceeds a predetermined threshold within a predetermined time period, then agitation is deemed sufficient. For example, if an acceleration of 1-20ms$^{-2}$ is assigned a weight of 1, 20-30ms$^{-2}$ a weight of 3 and greater than 30ms$^{-2}$ a weighting of 5, then if the sum total of the weightings exceeds 25 within 10 seconds, agitation is deemed sufficient. Such an arrangement allows flexibility in the manner in which the shake the inhaler 2. For example, a less able patient can shake the inhaler 2 more gently, but for an increased time.

The predetermined time period is greater than or equal to 5 seconds, preferably between 5 seconds and 15 seconds. The predetermined time period is less than or equal to 15 seconds.

The validation system 102 ensures agitation is performed via either one or both of the canister agitator (first embodiment) or the movement sensor (second embodiment) according to the one or more predetermined criteria. If the criteria are met, the validation system 102 then proceeds to the next step 114 of ensuring the inhaler 2 is provided at the correct orientation. If the criteria are not met, then the user may be prompted to perform agitation again and the process is repeated.

The inhaler 2 comprises a tilt sensor (not shown). The tilt sensor is configured to determine the angle at which the inhaler 2 is orientated compared to a desired angle. Typically, the desired angle is where the upper surface 16b faces directly upwards and the lower surface 16c faces directly downwards. Two tilt sensors (e.g. angular orientation sensors) may be provided to determine the tilt angle in two orthogonal axes (i.e. the axes perpendicular to the vertical axis extending between the lower and upper surface 16a,b).

A tilt event may be detected if the tilt angle of one or both of the sensors determines the difference present angle of orientation of the inhaler exceeds a predetermined threshold. For example, a tilt event may be determined if the inhaler is tilted at greater than about 5 degrees, or 10 degrees, or 15, degrees, or 20 degrees or more from the desired angled.

The validation system 102 monitors the tilt angle and if the tilt of the device is within the acceptable threshold, then the validation system proceeds to a filling 116 step. If the correct orientation is not detected within a certain time period, then the medicament in the canister 24 may re-settle. Agitation is then required again. The validation system 102 is provided with a predetermined time period in which filling must begin after agitation. If the inhaler 2 is not provided at the correct orientation within the predetermined time period, then filling is prevented. The time period may be between 2 seconds and 5 seconds. If filling is prevented, the validation system 102 may prompt the user to repeat the manual agitation. The validation system 102 may prompt or alert the user that the end of the predetermined time period is approaching. For example, the inhaler 2 may alert the user 2 or 3 seconds before end of the predetermined time period.

In the filling step 116, the dosing chamber 56 is filled with the medicament to provide the predetermined dose thereof. The variable dose valve mechanism 32 proceeds to fill the dosing chamber 56 as described previously with reference to figures 10 to13. The valve mechanism 32 remains in the "ready" state (figure 13).

The agitator 106 may continue to operate during the filling stage. The validation system 102 may continually monitor the tilt angle during the filling stage. If the tilt angle exceeds the desired angle, then the filling stage may stop. The user may then be prompted to restarted the process.

In the next step 118, the validation system 102 is configured to notify the user the inhaler is in the "ready" state. The notification therefore indicates the inhaler 2 is ready/primed for inhalation by the user. The notification may instruct the user to inhale.

The notification is provided by an indicating means on the inhaler and may take any suitable form. The notification may be displayed on the electronic display 18. Additionally or alternatively, the notification may comprise an audible notification. The notification may comprise a beeping sound, buzzer, haptic or the like.

Once the dosing chamber 54 is filled, the composition of the medicament is fixed. Notification of the user and/or actual inhalation by the user may therefore occur at any time after filling of the dosing chamber 54 as settling/segregation of the medicament does not have a significant effect. Agitation and/or determination of tilt angle is therefore not required once filling is complete.

In the next step 120 the shield 82 moves to the open position (i.e. such that the nozzle 76 is unobscured). In the present embodiment, the shield 82 moves to the open position after the notification is provided. However, it can be appreciated the shield 82 opening may occur before or during the notification step 120.

In the next step 122, the inhaler 2 is configured to detect the inhalation of user.

The pressure sensor 92 is operatively connected to a pressure sensing system 124. The pressure sensing system 124 monitors the pressure in the channel 34 to detect inhalation of the user according to one or more predetermined criterion. In the present embodiment, the pressure sensing system 124 is configured to detect a reduction in pressure and/or increase in flow rate in response to inhalation.

In some embodiments, an inhalation event is determined by the pressure sensing system 124 when the pressure drop reaches a predetermined magnitude. This prevents unintentional dispensing of the inhaler due to random fluctuations in pressure etc. For example, an inhalation event is determined when a pressure drop of greater than 1 kPa; preferably greater than 5 kPa; such as 10 kPa is detected.

In some embodiments, the pressure drop may be converted to or calibrated to determine a flow rate through the channel 34 for a given flow rate. Thus, the pressure sensing system 124 may determine an inhalation event based on the calculated flow rate through the channel 34. For example, an inhalation event is determined when a flow rate (e.g. instantaneous flow rate) of greater than 10L per minute; preferably greater than 20L per minute; such as 30 litres per minute or more is detected.

In some embodiments, an inhalation event determined by the pressure sensing system 124 when the pressure drop lies within a predetermined range. Providing an upper bound to the magnitude of the pressure drop ensures that the user is inhaling at a desired flow rate to ensure effective medicament application and/or delivery.

For example, an inhalation event is determined when a pressure drop of less than 15 kPa; preferably 10 kPa, or less; or less than 8 kPa is detected.

An inhalation event may be determined when a pressure drop of between 1 kPa and 15 kPa; preferably, 10 kPa is detected.

In some embodiments, an inhalation event is determined when a rate in change of pressure reaches a predetermined magnitude. For example, an inhalation event is determined when a pressure drop rate of greater than 5 kPa per second; preferably greater than 10 or 20 kPa per second is detected.

In some embodiments, pressure the sensing system 124 uses an algorithm to detect an inhalation event based on the pressure measured in the channel 34 over a predetermined time period. The algorithm may use one or both of the absolute pressure or the rate of change of pressure to determine an inhalation event. This may help to filter erroneous pressure drops not caused by user inhalation. The pressure the sensing system 124 may require the magnitude of the pressure drop to exceed a predetermined threshold for a predetermined period of time. There is therefore a delay between when a pressure drop is first detected and an inhalation event is determined. For example, the pressure drop must exceed 5 kPa or 8 kPa or 10 kPa or more for at least 0.5 seconds or 1 second or 2 seconds or more, to indicate an inhalation event.

Upon detection of the inhalation event, the pressure sensing system is configured to send a signal to the validation system 102 to indicate user inhalation has been detected. If inhalation is not detected, the validation system may prompt the user. The prompt may be provided after a predetermined time period.

At a final stage 126, the validation system 102 is configured to move the valve member 46 such that the dosing chamber 56 and the nozzle 76 are fluidly connected (figure 14). Medicament 96 is therefore dispensed from the dosing chamber 56. Upon successful dispensing of the dose, the inhaler 2 may notify the user. The inhaler 2 may record and/or transmit data indicative of a successful dispensing cycle. The user may then power down the device by closing the cap.

In some embodiments, the inhaler 2 may only dispense medicament after a predetermined period of time after agitation thereof. This may allow the medicament to at least partially settle. The predetermined period of time may initiate once agitation has ceased. If the user attempts to inhale before the predetermined period, the valve mechanism does not actuate. The user may receive a notification indicating the inhaler will not dispense and/or prompting the user to wait.

The predetermined period of time may be at least 1 second; at least 2 seconds; or at least 5 second.

In some embodiments, the user may be required to inhale multiple doses. The inhaler 2 may be configured to repeat some or all of the validation system 102 described above. In some embodiments, further agitation may not be required (e.g. if the dose is dispensed within a predetermined time period). The second or further dose may be dispensed from a partially ejected dosing chamber (i.e. the dosing chamber may provide multiple doses).

Dispensing of the further dose may only be permitted after a predetermined period of time from dispensing of the previous dose. The user must therefore wait between doses (e.g. to hold their breathe). The valve mechanism may therefore only actuate after a predetermined period of time after a previous actuation. The predetermined period of time may be at least 3 seconds; at least 5 seconds; at least 7 seconds; or at least 10 seconds.

It can be seen that filling of the dose chamber 54 and dispensing of medicament are perform as independent/asynchronous steps (in contrast to conventional devices where refilling of a metered dose occurs immediately after/-

synchronously dispensing of dose). This means that the agitation and/or orientation validation need only be perform during the filling state and not during the dispensing stage. The use of an electronic pressure sensor means that the filling and dispensing stages can be provided independently, and that the pressure threshold for activation can be adjusted easily.

The system may record any or all data relating the to agitation, orientation and/or pressure sensing (e.g. in memory therein). The system may record whether each of the stages was successful (i.e. within desired parameters). The data may be transmitted to a remote device (e.g. a healthcare professional's device). The healthcare professional may therefore review the data to ensure the patient is correctly using the system. The data may comprise the indication whether each stage was successful. This provides a quick reference to the healthcare professional. For example, a "traffic light" system could be provided to indicate the patient's use of the system. The system would provide a red indicator if the measured orientation/agitation/pressure is outside desired parameter; a yellow indicator if the measured parameters are close to desired parameters; and a green indicator is the measure parameters are within the desired parameters.

In some embodiments, the system may still dispense medication if the measured parameters are outside the desired parameters (i.e. the shield and/or valve open regardless of the measured parameters). The system may therefore record/transmit the data for manual review. This ensures the patient can always access the medication.

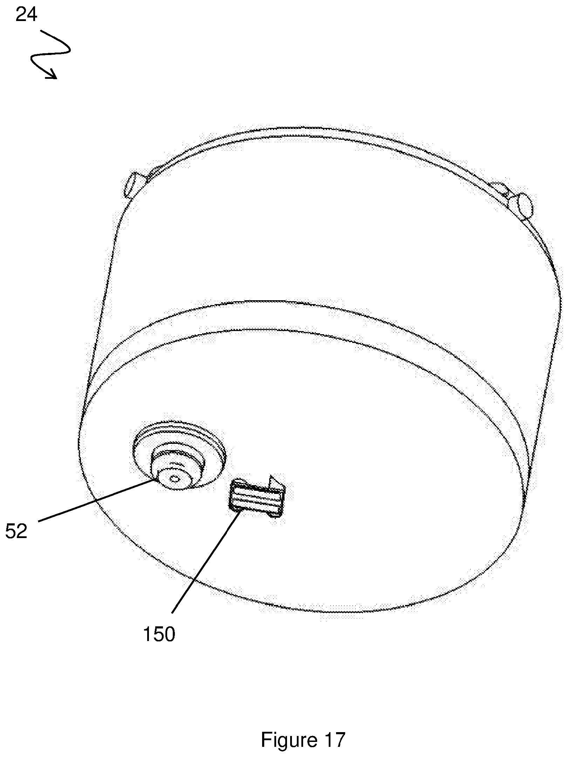

The canister 24 is shown in closer detail in figure 17. The canister 24 is substantially cylindrical. The axial length of the canister 24 is less than width thereof. The canister 24 is thus "puck" shaped. Such a shape allows for the incorporation of the agitator 106.

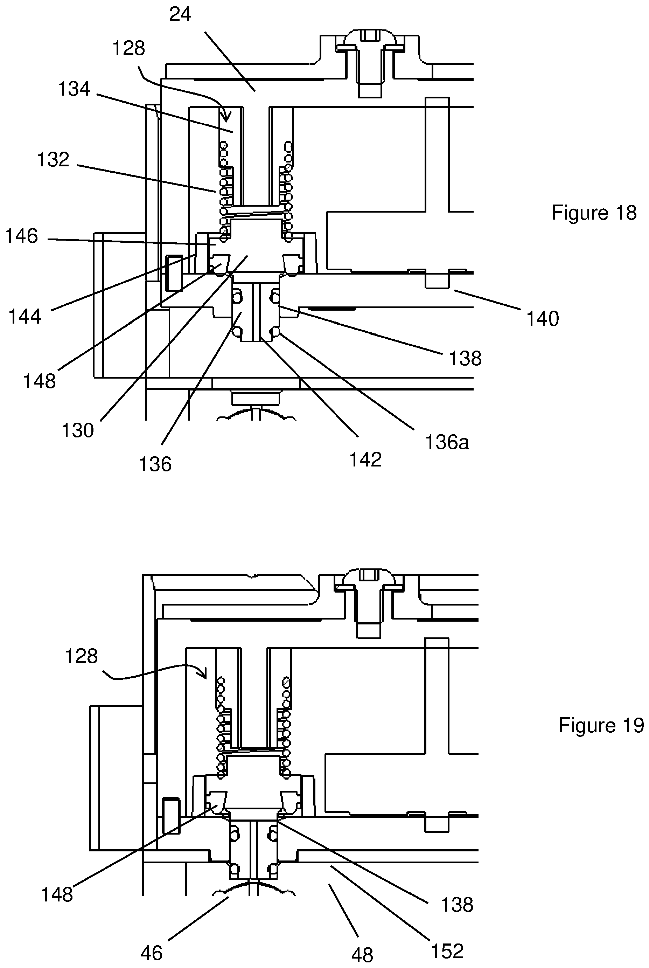

As shown in figures 18 and 19, the canister 24 comprises an outlet 52. The outlet 52 is received within the valve block 48. The outlet 52 is adjacent the valve member 46. As shown in closer detail in figures 18 and 19, the outlet 52 comprises a valve arrangement 128. The valve arrangement 128 is configured to fluidly connect the canister 24 and the variable dose valve mechanism 32 when the canister 24 is received within the inhaler 2.

The valve arrangement 128 comprises a valve member 130. The valve member 130 is movably mounted within the canister 24. The valve member 130 is mounted to the canister 24 via a resilient biasing means (i.e. a spring 132). The spring 132 is mounted on an arm 134 within the canister 24. The valve member 130 is therefore biased away from the arm 134. An outlet portion 136 of the valve member 130 extends through an outlet 138 in the canister wall 140. The valve member 130 may therefore be moved from outside the canister 24. The outlet portion 136 comprises a fluid passage 142 extending therethrough. The outlet portion 136 comprises a plurality of seals 136a configured to engage the outlet 138.

The valve member 130 is received within a sleeve 144 surround the canister outlet 138. The member valve 130 member comprises a flange 146 configured to engage the sleeve 144 to prevent lateral movement of the valve member 130. The sleeve 144 comprises a plurality of apertures to allow fluid to flow to the interior of the sleeve 144. The flange 146 comprises a seal 148 (e.g. a gasket or the like). The seal 148 is configured to engage the canister wall around the outlet 138 when the valve 130 is in a closed position (see figure 18). Additionally or alternatively, the seal 148 blocks the apertures in the sleeve 144. The seal 148 therefore seals the outlet 138. In the open position in figure 19, the seal 148 is displaced away from the canister wall/sleeve apertures, allowing fluid to flow out from the outlet 138 and into the variable dose valve mechanism 32. The valve arrangement 128 remains open when the canister 24 is retained in position, thus allowing flow of medicament into the inhaler 2. The valve 128 automatically closes upon release of the canister 24.

Referring back to figure 17, the canister 24 comprises an electronic memory device 150. The memory device 150 is provided on the underside of the canister 24 (i.e. the side comprising the valve arrangement 128). However, it can be appreciated the memory device 150 may be provided at any suitable location on the canister 24. The memory device 50 may be fully or partially embedded in the walls of the canister 24. The memory device 50 may lie flush with the wall of the canister 24 to prevent damage thereof.

The memory device 150 is configured to be read by the inhaler 2. The memory device 150 may comprise an EEPROM. The inhaler 2 comprises an electronic reading device 152 configured to read to data from the memory device 150. The electronic reading device 152 may be provided in the valve block 48. The reading device 152 may comprise a plurality of contact pins or like (i.e. a physical connection). In other embodiments, communication may be provided wirelessly (e.g. NFC or the like). The data stored on the memory device 150 may comprises one or more of:

- a name of the drug, or one or more active ingredient contained within the canister. The name may comprise one or more of: a brand name; a generic name; or a chemical/IUPAC name etc. The name of each respective drug/active ingredient may be provided
- any one or more of a: time/date of manufacture; manufacturer name; manufacturer location; batch number.
- the volume/mass of the medicament and/or the number of doses therein (i.e. at new, completely-filled state).
- a genuine product identifier. The data may comprise a key, unique key, code and/or other security data to indicate the canister comprises a genuine product. The inhaler 2 is configured to validate the identifier. If the identifier is not valid, then inhaler 2 may notify the user and/or may not permit dispensing thereof.
- a unique identifier for the canister.
- instructions on a dosing regime. The instructions may indicate the appropriate dose size. The inhaler 2 may therefore vary the size of the dosing chamber 54 accordingly. The instructions may indicate the time and/or time period for dispensing the dose (e.g. two doses twice a day etc.). The inhaler 2 may then notify the user accordingly.

The memory device 150 may further provide an indication the canister 24 is correctly inserted (i.e. the memory device 150 is only readable when the canister 24 is completely inserted).

In some embodiments, the data and/or a portion thereof may be read only (i.e. stored on ROM such as an EEPROM). This may prevent tampering of the canister. In some embodiments, the data and/or a portion thereof may be writable. For example, the instructions may be re-writable if the dosage regime requires amendment.

In some embodiments, the inhaler 2 is configured to write data to the memory device 150. The data stored on the memory device 150 may comprises one or more of:

- the mass/volume of the medicament used. Due the variable dose of the inhaler 2, non-standard doses may be dispensed and thus it may be difficult to determine the remaining volume/mass of medicament by counting of the number of doses alone. Therefore, each time a dose is dispensed by the inhaler 2, the volume/mass of the dose is recorded on the memory device 150 by re-writing the previous value with the new cumulative total. In some embodiments, the mass/volume of each respective dose is recorded on the memory device 150. The inhaler 2 may determine the cumulative mass/volume by summing the mass/volume of the respective doses.
- the mass/volume of the medicament remaining in the canister. It may be appreciated that such data is generated in the same way as described above. However, the inhaler 2 first reads the mass/volume of the new canister and subtracts the mass/volume of each dispensed dose accordingly.
- details of each dispensed dose. The details may comprise the volume/mass dispensed and/or the time/date of dispensing. The details may comprise an indicated of the medicament used. The canister 24 thus comprises a full record of the usage of the inhaler 2, allowing an audit or review by a healthcare professional.

In some embodiments, the inhaler 2 is configured to extract the data or portions thereof and store the data therein. For example, the inhaler 2 may extract the used/remaining amount of medicament. The inhaler 2 may then determine the number of doses remaining based on the volume/mass of variable dose dispensed (i.e. by dividing the remaining amount of the amount of each dose). This may be displayed to the user. The remaining/used number of doses may be stored and/or calculated by the canister 24.

In some embodiments, any of the data written to the canister 24 by the inhaler 2 may also be stored in the inhaler 2. Thus, both the canister 24 and the inhaler 2 may provide an audit trail.

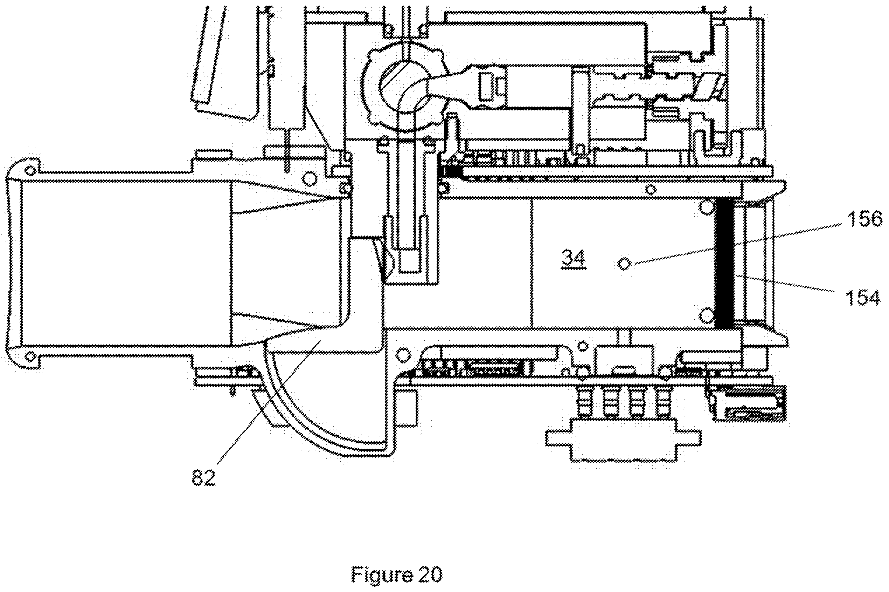

The sensor module 4 is described in detail in figures 20 to 23. The sensor module 4 is configured to sense and/or analyse one or more biomarker in exhalation of the user. In the present embodiment, the sensor module 4 is configured to detect and/or analyse Nitric Oxide in the exhalation of the user. Such an arrangement is often referred to as a "FeNo" test. Nitric Oxide may be indicative of inflammation in the lungs, and therefore indicative of the severity of respiratory inflammation.

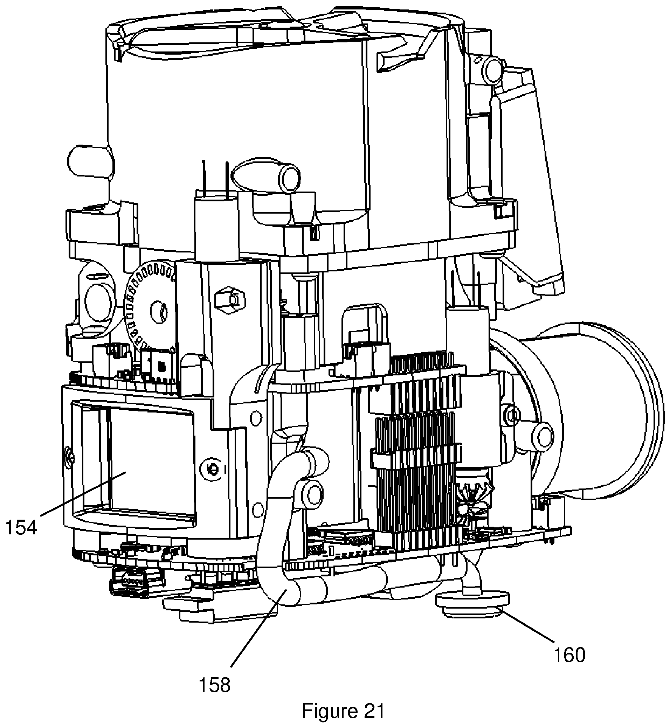

Referring to figures 20 and 21, the inhaler 2 is operable in a FeNO mode. A selectively closable door 154 is provided in the channel 34. The door 154 is provide on an opposing end of the channel 34 to the mouthpiece 10. The door 154 is mounted via a pivot at an edge thereof. The pivot extends in a vertical direction. The door 154 is therefore configured to swing into the channel 34 in the open position. In the open position, the door 154 lies flat against the interior wall of the channel 34 (see figure 9). In the closed position, the door 154 is configured to close (i.e. completely obscure) the channel 34.

An outlet 156 is provided in the channel 34. The outlet 156 is provided on side wall of the channel 34. The outlet 156 is provided proximal the pressure sensor 92. Thus, when the door 154 is closed with respect to the main flow path through channel 34, air is diverted into the outlet 156. When the door 154 is opened with respect to main flow path through channel 34, the outlet 156 is obscured by the door 154.

The outlet 156 is fluidly connected to a pipe 158. The pipe 158 extends outside the channel 34 and toward the lower end 16a of the inhaler 2. The pipes 158 is connected to a connector 160. The connector 160 is provided on the lower end face 16a of the inhaler 2. The pipe 158 thus provides a fluid pathway between the channel 34 and the connector 160.

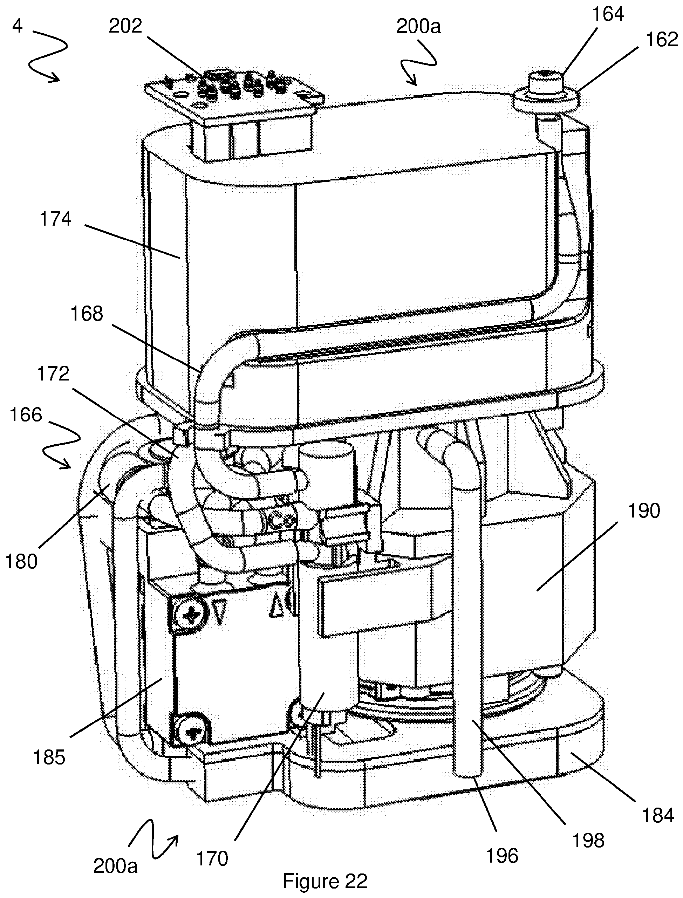

As shown in figure 22, the sensor module 4 comprises a connector 162 configured to engage the inhaler connector 160. The sensor connector 162 comprises a male portion 164 configured to be received within the inhaler connector 160. The male portion 164 may comprise a resilient material to provide an interference fit the inhaler connector 160.

The sensor connector 162 is fluidly connected to a sensor arrangement 166. The sensor connector 162 and sensor arrangement are in connection via a pipe 168. It can be seen that the channel 34 is fluidly connected to the sensor arrangement 166. Thus, during exhalation, exhaled air passes through into the sensor arrangement 166.

The sensor arrangement 166 described herein provides a biomarker sensor arrangement, i.e. suitable for sensing one or more biomarker in the expired air of the user. Whilst the following description proceeds in relation to a nitric oxide (NO) sensor arrangement, this provides just one example of a suitable biomarker and the following sensor system description, or individual features thereof, may be applied to other biomarker sensor types.

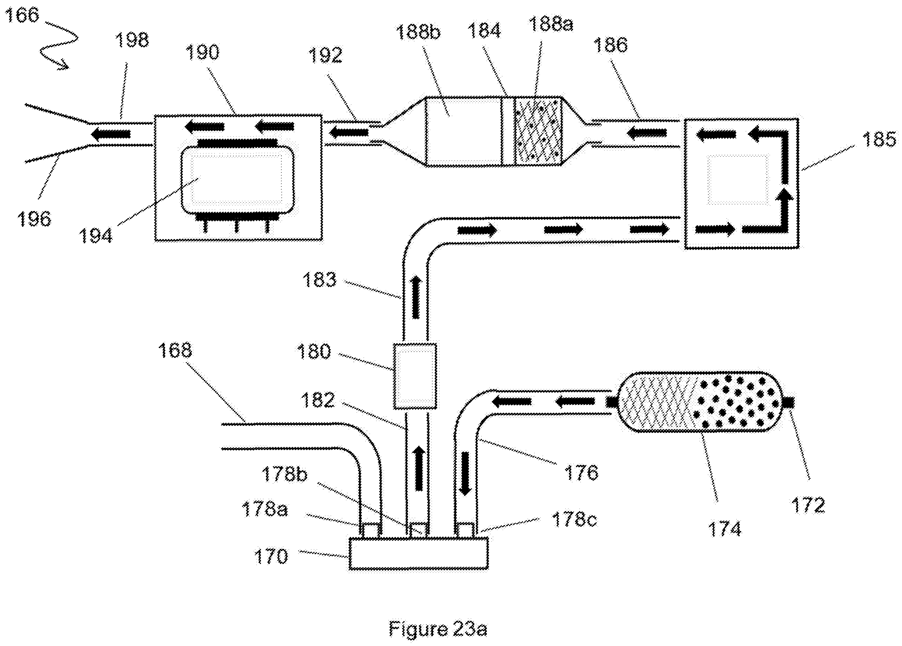

The NO sensor arrangement 166 is shown schematically in figures 23a-c. The pipe 168 is connected to a transfer valve 170, operation of which will be described later. The sensor arrangement 166 comprises an auxiliary inlet 172. The inlet 172 is connected to ambient environment. The inlet 172 is fluidly connected to a filter 174. The filter 174 is configured to filter out NO and/or particulates in the air passing therethrough. The filter 174 may therefore comprise an NO scrubber. The filter 174 may comprise a HEPA filter or the like. The filter 174 may be configured to filter out moisture (e.g. a dehumidifier). The filter 174 may comprise a condenser dehumidifier. The filter 174 may comprise a Peltier dehumidifier. In some embodiments, the moisture filter may comprise a desiccant or hygroscopic material. The filter 174 is connected to the transfer valve 170 via a pipe. The transfer valve 170 is thus fluidly connected to the ambient environment by the filter 174

The transfer valve 170 comprises a plurality of ports 178a-c. A first port 178 is connected to the intake pipe 168. A second port 178b is fluidly connected to a filter 180. The outlet 178 and filter 180 are connected via a pipe 182. The filter 180 is configured to filter out particulates in the air passing therethrough. The filter 180 may comprise a HEPA filter or the like. A third port 178c is fluidly connected to the NO scrubber 174.

The filter 180 is fluidly connected to a pump 185. The filter 180 and pump 185 are connected via a pipe 183. The pump 185 is configured to pump air from the transfer valve 170 (i.e. increase the flow rate thereof). Additionally, the pump 185 may help maintain sufficient pressure through the system due to the drops in pressure created by the filter 180.

A second filter 184 is fluidly connected to the pump 182. The second filter 184 and pump are connected via a pipe 186. The second filter 184 comprises a first filter portion 188a configured to filter particulate material. The first filter portion 188a may comprise a HEPA filter or the like. The first filter portion 188a may comprise a filter of finer grade than the first filter 180. The second filter may therefore catch particulate matter missed by the first filter 180.

A second filter portion 188b is configured to filter out moisture (e.g. a dehumidifier). The second filter portion 188b may comprise a condenser dehumidifier (i.e. a heat exchanger dehumidifier). The second filter portion 188b may comprise a Peltier dehumidifier. The air is cooled using a cold sink. Any excess moisture (i.e. above the saturation humidity of the cooled temperature) condenses. The air is then heated to a predetermined temperature. As humidity is limited by the saturation humidity in the cold state, when heated, the air comprises a known humidity. This allows consistent results between samples. One or more temperature sensors may determine the temperature of the cooling/heating stage. This ensures compliance with a test routine, or allows correction/calibration of an test results. Additionally or alternatively, the moisture filter may comprise a desiccant or hygroscopic material.

In some embodiments, the sensor module 4 comprises a thermometer. The thermometer is configured to measure the temperature in or around the sensor module 4. If the temperature is below a predetermined threshold, then sensor module 4 provides an alert to the user. The sensor module 4 may also prevent a detection cycle. This prevents the build-up of condensation within the device. The temperature is monitored continuously or at predetermined intervals. When the measured temperature reaches the desired temperature, the user is alerted and the detection cycle is permitted.

Additionally or alternatively, the detection cycle may determine if the temperature exceeds a predetermined temperature. This may provide more accurate and consistent results (e.g. in the dehumidifying stage). The sensor module 4 may therefore alert the user and/or prevent operation if the measured temperature lies outside a predetermined range.

The second filter 184 is fluidly connected to a Nitric Oxide (NO) sensor 190. The second filter 184 is connected to the NO sensor 190 via a pipe 192. The sensor 190 comprises a diffuser configured to diffuse air entering from the pipe 192. The NO sensor 190 comprises an electronic NO detector 194. The NO detector 194 is configured to determine the partial pressure/fractional concentration of NO passing through the NO sensor 190. The NO detector 194 may comprise a conventional sensor. The NO sensor may comprise a detection range between about 5ppb and 300ppb, with alerts generated if the NO level is above 50 ppb for adults or 35 ppb for children.

The NO sensor 190 is fluidly connected to an outlet 196. The NO sensor 190 and the outlet 196 are connected via a pipe 198. The outlet 196 and/or the NO sensor 190 may comprise a one-way valve to prevent ingress of air or particulates etc. into the NO sensor 190. The outlet 196 is provided at a lower end 200a of the sensor module 4 (see figure 22).

The use of the pipes 166,176,182,183,192 between the respective components 170,174,180,184,185,190 allow the components to be placed in a space efficient arrangement that would otherwise not be possible if the components were provided in a linear or combined fashion. The NO sensor can therefore be provided as a compact, detachable module. Detection of NO requires a clean and low moisture air sample. The filters 180,184 thus ensure the air sample is clean and low moisture, such that the relative humidity (RH) of air passing over NO sensor 90 is less than about 60 % RH, or preferably less than 50 %, 40 %. 30 %, 20 %, 10 % or lower.

Operation of the NO sensor module 4 will now be described. Referring back to figure 23a, the sensor module 4 is configured to operate in a "purge mode". The purge mode is configured to purge the air in the system to remove any residual NO, thereby ensuring accurate results. The first port 178a is closed via the transfer valve 170 and the third port 178c is opened. The second portion 178b remains open. The pump 185 is activated, drawing air into the NO scrubber 174, through the filters 180,185 and the NO sensor 190. Any residual NO in the system 166 is therefore dispersed within the airway.

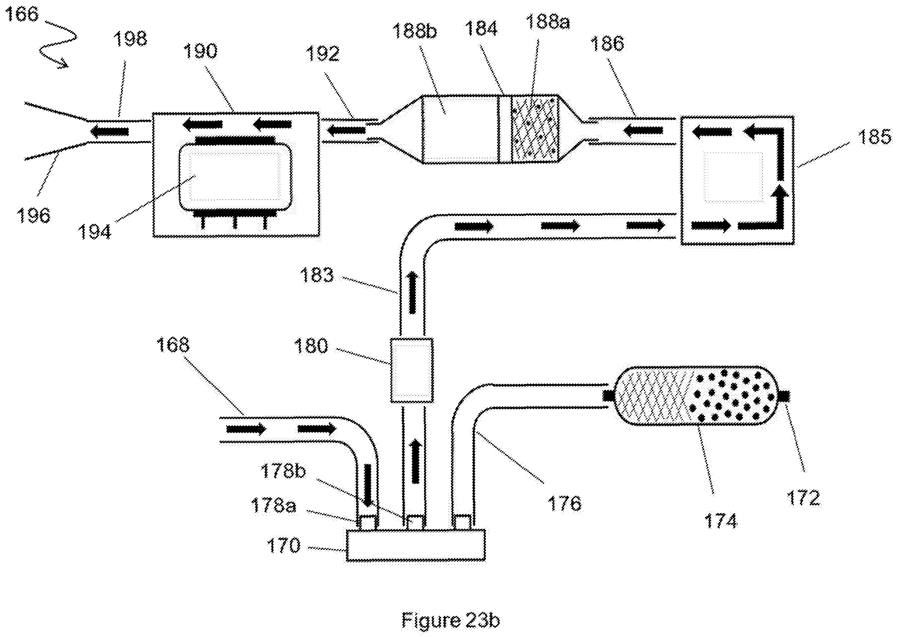

Referring now to figure 23b, an ambient air sample analysis is performed. This may establish a baseline for the amount of NO in the ambient environment. The first port 178a is opened, the third port 178c is closed and the second port 178b remains open. The pump 185 draws air through the inlet pipe 168 and through the to NO sensor 190. A reading is taken and recorded.

A notification may be provided to the user that purge or ambient testing stages are being performed. The inhaler 2 may be disconnected from the sensor module 4 during the purge and/or ambient testing stages. The inhaler 2 may therefore communicate to the sensor module wirelessly (e.g. to initiate the purge or ambient testing stages)

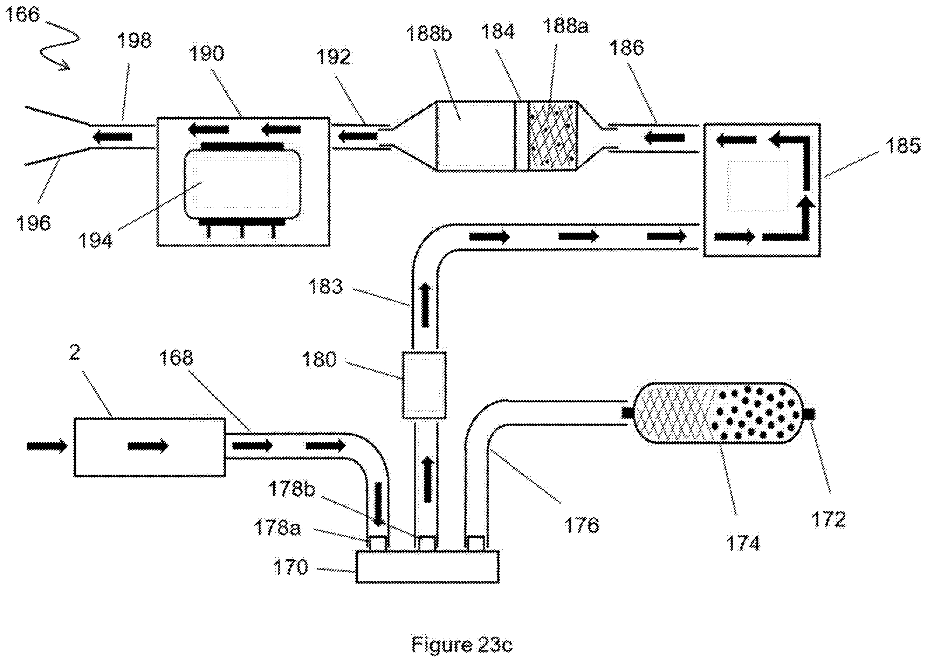

Referring now to figure 23c, the first port 178a remains opened, the third port 178c remains closed and the second port 178b remains open. The inhaler 2 is connected to the sensor module 4, thereby fluidly linking the mouthpiece 10 of the inhaler 2 with the NO sensor 190. The inhaler 2 may there provide an extended mouthpiece. The user may receive a prompt to connect the sensor module 4 to the inhaler 2. A sensor is provided to detect connection of the sensor 4 to the inhaler 2. Once connection is detected, the inhaler 2 prompts the user to exhale.

The pump, in combination with the user's exhalation, draws exhaled air into the NO sensor 190. The NO levels are detected. The NO levels are compared with the ambient NO level, and a resultant NO level is determined, indicative of the NO exhaled by the user. The exhaled NO levels are recorded and/or indicated to the user.

The pressure sensing system 124 is configured to determine whether an inhalation event has occurred, as previously described within respect to the validation system 102 and will not be repeated for the sake of brevity. If inhalation is detected, the user may receive a notification to indicate the test has been successful. The user is then prompted to disconnect the inhaler 2 and the sensor module 4. The sensor then detects disconnection of inhaler and the sensor module 4 to complete the FeNO test. If inhalation is not detected, then user is prompted to repeat the test.

The inhaler 2 may prompt otherwise guide the user during exhalation. An indication of the magnitude and/or duration of the pressure drop/flow during inhalation. For example, the display may comprise a countdown or graphical indicator to indicate progress through the exhalation cycle.

The sensor module 4 comprises an electrical connector 202. The electrical connector is configured to engage an electrical connector 204 provided on the inhaler 2 (see figure 9). The inhaler 2 may provide electrical power to the sensor module 4 (i.e. the inhaler 2 and the sensor module 4 comprise a shared power system). The inhaler 2 and the sensor module 4 may communicate electronically. The sensor module 4 may transmit data to the inhaler 2 and/or vice versa. For example, the sensor module 4 transmits FeNO data to the inhaler 2.

In alternative embodiments, the sensor module 4 may be configured to determine the presence/concentration of alternative exhaled indicators. For example, the sensor module 4 may be configured to detect/measure oxygen levels and/or carbon dioxide and/or carbon monoxide levels. The FeNO sensor module may be calibrated against a 'known datum' NO sample.

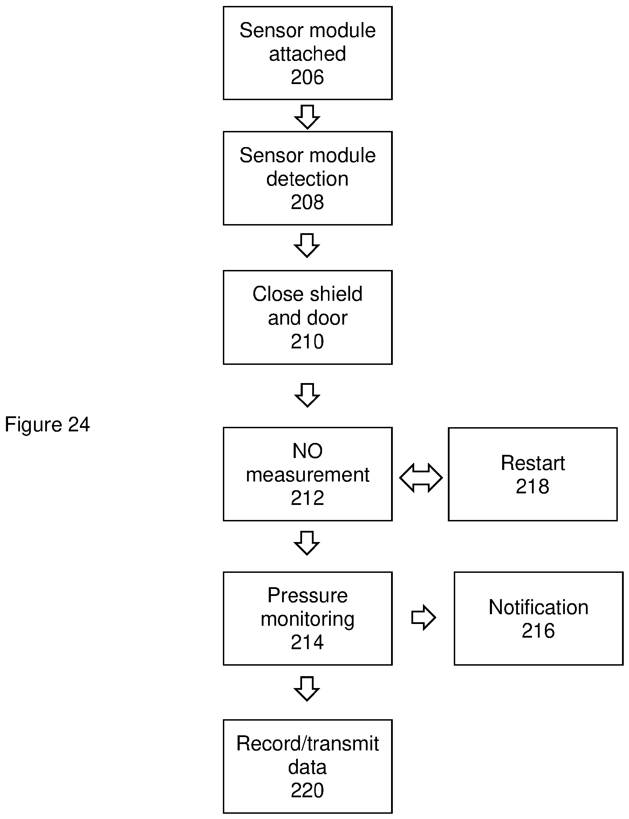

Operation of sensor module 4 will be described with reference to figure 24. In a first step 206, the sensor module 4 is attached to the inhaler 2. This is performed after the sensor module has performed the purging and ambient air sampling stage. The user may receive a prompt to connect the sensor module 4 to the inhaler 4.

In a second step 208, the sensor module 4 communicates with the inhaler 2 to indicate the sensing module 4 is attached. The inhaler 2 therefore detects the sensor module 4. The inhaler 2 may determine the type of the sensor attached.

In the next step 210, the door 154 and the shield 82 are moved to the closed position (see figure 20). The channel outlet 156 is now unobscured. The valve member 46 is provided in the "dispensing" position.

In the next step 212, the NO measurement routine begins. The inhaler 2 may provide an audible/visual indicator that the inhaler 2 and sensor module 4 are ready for measurement. The user then exhales into the mouthpiece 10. The exhaled air travels though the channel outlet 156 and into the sensor module 4. The air then passes through the NO sensor arrangement 166 to the NO sensor. The direction of airflow is indicated by the black arrows in figure 23. The pump 182 directs the air into the NO sensor 190.

During NO measurement 212, the pressure sensing system 214 is configured is configured to monitor the pressure within the channel 34. The inhaler 2 thus ensures the user is exhaling at the correct pressure rate to ensure accurate measurement of the NO concentration.

The pressure sensing system 214 is configured to monitor if exhalation lies with a predetermined flowrate range. The pressure sensing system 214 thus monitors is the pressure measured by the pressure sensor 92 lies within a predetermined pressure range.

The predetermined flow may comprise a flow rate greater than or equal to 100ml/min, preferably greater than 200ml/min. The predetermined flow may comprise a flow rate less than or equal to 500ml/min, preferably greater than 400ml/min. Typically, the predetermined flow rate is between 200ml/min and 300ml/min, preferably, between 250ml/min and 350ml/min.

If the measured pressure/flowrate falls outside the desired ranges, the inhaler 2 is configured to notify the user. The inhaler 2 may notify whether the exhalation flowrate is too low and/or too high. For example, the display 18 may display a symbol if the flow rate is too low and/or too high. Additionally or alternatively, the notification may comprise an audible and/or haptic notification. The notification may comprise a beeping sound, buzzer, vibration or the like. This provides a feedback loop to allow the user to correct their exhalation rate.

The inhaler 2 is configured to determine if the flow rate is sufficient to provide an accurate test. For example, if the user provides an incorrect flowrate for a significant portion of the test, the measured data may be invalid. In such a case, the test may need to be repeated. In some embodiments, if the flowrate/pressure lies outside the predetermined range for a pre-determined period of time, then the inhaler 2 indicates the test is deemed not sufficient. For example, if the flowrate/pressure lies outside 250ml/min-350ml/min for longer than 3 seconds consecutively, for example, the test is deemed not sufficient.

In some embodiments, if the flowrate/pressure lies outside the predetermined range for a cumulative pre-determined portion of the total time of the test, then the inhaler 2 indicates the test is deemed not sufficient. For example, if the flowrate/pressure lies outside 250ml/min-350ml/min for cumulatively longer than 3 seconds in a 10 second period, for example, the test is deemed not sufficient.

In some embodiments, the inhaler 2 is configured to determine whether the test is sufficient using the duration of the user exhalation. If the length of the duration is below a predetermined threshold, the then the inhaler 2 indicates the test is deemed not sufficient. For example, if the duration of exhalation is less than 7 seconds, the test is deemed not sufficient. The duration of the exhalation may be determined by measuring the duration the flowrate/pressure is above a predetermined threshold. For example, if the flowrate is below 250ml/min-, then exhalation may be deemed to not be occurring.

The inhaler 2 may prompt otherwise guide the user during the exhalation cycle. An indication of the magnitude and/or duration of the pressure drop/flow during inhalation. For example, the display may comprise a countdown or graphical indicator to indicate progress through the exhalation cycle. This is provided in real time. Additionally or alternatively, an audible indicator may indicate when to start and/or stop exhalation. The inhaler 2 may display the value of the flow rate and/or pressure drip in real time, and/over provide an average value after the inhalation is complete.

If the test is not deemed sufficient, then the inhaler 2 initiates a restart step 218. The inhaler 2 may notify the patient a restart of the test is required. The NO measurement step 212 is then repeated. The inhaler notifications above may comprise a symbol on the screen, or an audible sound, haptic feedback or any combination of such notifications.

During NO measurement, the NO concentration is recorded. The NO data may be stored in volatile/non-volatile memory on the sensor module 4. Additionally or alternatively, the NO data is transmitted to the inhaler 2 (e.g. via the electronic connector 202). The NO data is therefore logged by the inhaler 2 and/or sensor module 4. Pressure/flow rate and/or test restart data may be record. This may help to identify if a user is having difficulty using the device.

Although, in the present embodiment, the sensor module 4 is shown in combination with a pressured metered dose inhaler (pMDI), it can be appreciated that such a module can be used with any suitable inhaler device. For example, the sensor module 4 may be used with a dry power inhaler (DPI).

In other embodiments, the sensor module 4 is configured to detect additional or alternative biomarkers present in exhaled gases. The biomarkers may comprise one or more of:

- A ketone. This may by a by-product of diabetic ketoacidosis (DKA). The ketone may comprise acetone.
- A biomarker indicative of lung or colorectal cancer. This may include a Volatile Organic Compound (VOC). The biomarker may comprise one or more of: a saturated hydrocarbon (ethane, pentane, aldehyde, decane), unsaturated hydrocarbon (isoprene), sulphur containing VOC (ethyl mercaptane, dimethylsulfide) and nitrogen containing VOC (dimethylamine, ammonia). In a specific embodiment, the sensor module 4 is configured to detect decane and analogs thereof.
- A biomarker indicative of liver disease. The biomarker may comprise one or more of: dimethyl sulphide; ethanol; acetaldehyde; or short chain alkanes (e.g. ethane and/or pentane). In some embodiments, the biomarker comprises a cyclic monoterpene, for example, limonene.
- A biomarker indicative of one or more of: COVID-19 (SARS-CoV-2) or other coronavirus infections; urinary tract infection (UTI) or other urinary infections; brain cancer; Parkinson's disease; Alzheimer's disease; or other neurodegenerative diseases._

In a further embodiment, the sensor module 4 is configured to detect one or more VOCs in the exhaled gases of a subject or user, including Acetone, Acetic acid, Isoprene, Propanal, Butanal, Pentanal, Hexanal, Heptanal, Octanal, Nonanal, Decanal, Methanol, Propanol, Butanol, Pentanol, Pentanoic acid, Hexanoic acid Hydrogren sulphide, Hydrogen cyanide, Acetaldehyde, Formaldehyde, Phenol, Methyl phenol, Ethyl phenol or Ammonia, wherein the presence and/or concentration of said VOC may be indicative of lung, colorectal or another cancer being present, optionally whereupon the inhaler may dispense a specific and/or personalised dose of a suitable therapeutic agent.

The sensor module 4 may comprise means to analyse the presence and/or concentration of one or more biomarkers via ionization, for example, where the gas sample is ionized and then separated into constituent parts that can be detected individually, employing techniques including the likes of Ion Mobility Spectrometry (IMS) and Field Asymmetric Ion Mobility Spectrometry (FAIMS), also known as Differential Mobility Spectrometry (DMS).

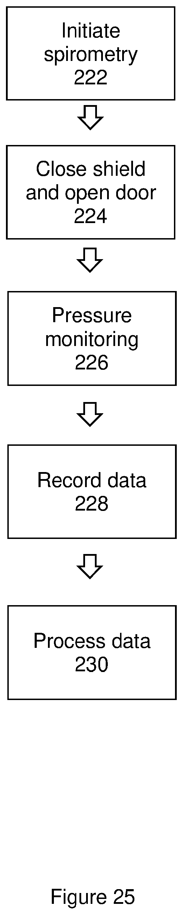

In a second mode, the inhaler 2 is configured to provide a spirometer. The inhaler 2 therefore measures the flowrate, speed, volume etc. of air during the user's exhalation and/or inhalation. The inhaler 2 therefore provides a medicament dispensing tool in combination with a diagnostic tool. The flowrate measurement is described with reference to figure 25.

In a first step 222, the spirometry mode is initiated. The user spirometry mode may be user initiated. For example, the user may select to perform a spirometry test and the inhaler 2 is configured to perform the stages of the test automatically. Alternatively, the inhaler 2 may prompt the user to begin a test (e.g. at set intervals).

In the next step 224, the shield 82 is moved to the closed position (if open). The door 154 is moved to the open position (if closed) and channel outlet 156 is obscured. The valve member 46 is provided in the "dispensing" position. The user is then prompted to exhale.

In the next step 226, the pressure sensing system 124 monitors the pressure in the channel 34. The pressure sensing system 124 may therefore determine the flowrate through the channel 34. The pressure sensing system 124 is configured to determine whether an inhalation event has occurred, as previously described within respect to the validation system 102 and will not be repeated for the sake of brevity. If inhalation is detected, the user may receive a notification to indicate the test has been successful. If inhalation is not detected, then user is prompted to repeat the test.

During this process, the data is recorded 228. The data may be stored on volatile/non-volatile memory in the inhaler 2. The data may comprise the flowrate at a given time.

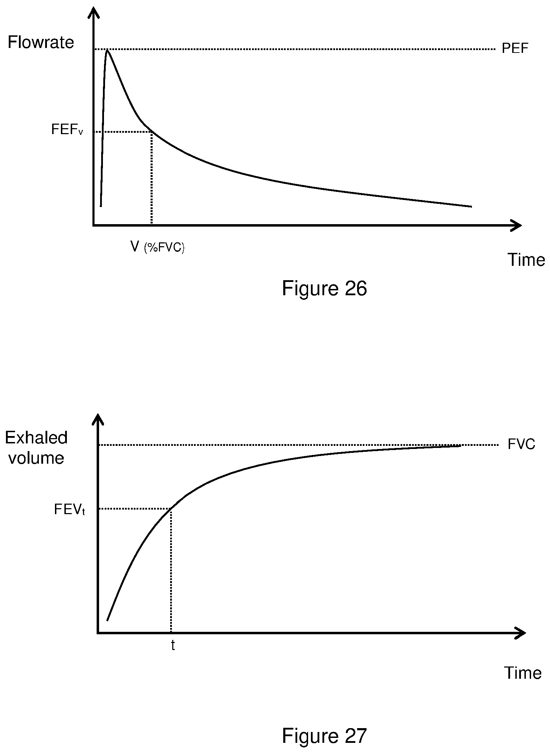

The data may then be processed 230. As shown in figure 26, a flowrate vs time graph may be constructed from the data. It can be appreciated that the flowrate curve is merely exemplary. As shown in figure 27, the flowrate may be integrated over time to provide an exhaled air volume over time graph. The processing may involve extracting one or more indicator(s) from the data. The indicators may comprise one or more of:

- Force vital capacity (FVC). FVC is the total volume of air that can forcibly be blown out after full inspiration (shown in figure 27).
- Forced expiratory volume (FEVt). $FEV_t$ is the volume of air expelled over a given time period (shown in figure 27). Typically, $FEV_1$ is measured, which is the volume of air expelled in the first 1 second of exhalation.
- FEV1/ FVC ratio-The percentage of the FVC expired in one second.
- $FEV_6$ -Forced expiratory volume in six seconds.
- Peak expiratory flow (PEF). PEF is the maximum flowrate during exhalation (shown in figure 26).
- Forced expiratory flow (FEF). FEF is the flow rate at a given time of the exhalation (shown in figure 26). The given time may be expressed as a fraction of the total exhalation volume FVC. For example, the given time may represent 25%, 50% or 75% of the FVC (FEF25, FEF50 or FEF75 respectively).
- Another FEF-based parameter includes $FEF_{25-75\%}$, as used in predicting the development of COPD. It is defined according to forced expiratory flow over the middle one half of the FVC; the average flow from the point at which 25 percent of the FVC has been exhaled to the point at which 75 percent of the FVC has been exhaled.
- MVV-Maximal voluntary ventilation.
- ERV-Expiratory reserve volume; the maximal volume of air exhaled from end-expiration.
- IRV-Inspiratory reserve volume; the maximal volume of air inhaled from end-inspiration.
- RV-Residual volume; the volume of air remaining in the lungs after a maximal exhalation.
- VT -Tidal volume; the volume of air inhaled or exhaled during each respiratory cycle.
- FRC-Functional residual capacity; the volume of air in the lungs at resting end-expiration.
- IC-Inspiratory capacity; the maximal volume of air that can be inhaled from the resting expiratory level.
- TLC-Total lung capacity; the volume of air in the lungs at maximal inflation.
- VC-Vital capacity; the largest volume measured on complete exhalation after full inspiration.

Any other indicators may be extracted from the data, as is conventional in the art. The indicators may be determined from the flowrate data using conventional processing techniques or algorithms. The indicators are stored in memory in the inhaler 2.

Although the spirometry has been described in relation to exhalation by the user, it can be appreciated, that the inhaler 2 may be used to additionally/alternatively measure the flow profile during inhalation of the user.

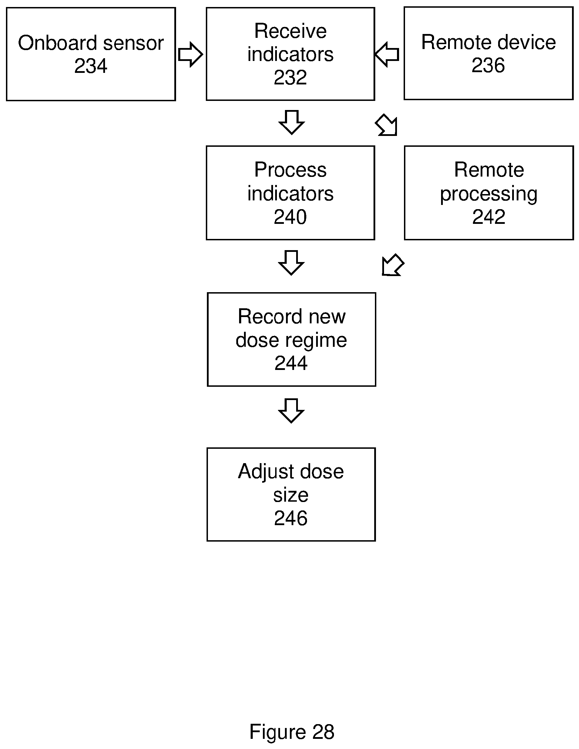

The inhaler 2 is configured to vary the dose of medicament delivered to the patient in accordance one or more factors indicative of the status or severity of the user's condition. The inhaler 2 thus adjusts the dose dispensed in accordance with the user's need. This may help to minimise the dose of medicament given to the patient whilst maintaining optimal efficacy. The dosage adjustment system is described with reference to figure 28. In a first step 232, the inhaler 2 receives one or more indicators of the severity of the user's condition.

In some embodiments, the indicative factors comprise measurements taken by the inhaler 2. The measurements are determined by any or any combination of the onboard sensors described herein. The factors may comprise one or more of:

- Spirometry data. The spirometry data may comprise one or more: $FEV_1$, or PEF, or a parameter derived therefrom
- Sensor module 4 data. The sensor module data may comprise a concentration (partial pressure) of Nitric Oxide or a parameter derived therefrom. An NO concentration greater than 50ppb for adults or greater than 53ppb may indicate the presence of inflammation.
- Environmental conditions local to the inhaler. The environmental condition may comprise one or more of, inter alia: temperature; humidity; pollution levels; pollen levels; or UV index. The inhaler 2 may comprise one or more sensor to determine the respective environmental conditions (e.g. thermometer, humidity, light sensor etc.).
- Previous or historical data relating to medicament(s) dispensed by the inhaler 2 and recorded thereon. The data may comprise an indication of the one or more medicament or active ingredients thereof. The data may comprise the dosage and/or usage frequency of the respective medicament(s).

With reference to FENO values obtained, various inferences can be made regarding the subject's status and/or responsiveness to therapies, particularly corticosteroids. For adult, FENO must be greater than 10 ppb to diagnose asthma; FENO greater than 45 ppb suggests poorly controlled asthma; treatment should be initiated if FENO is greater than 35 ppb; FENO greater than 47 ppb predicts steroid responsiveness; FENO of 35 ppb is the threshold value for the prediction of inhaled corticosteroid responsiveness; the inhaled corticosteroid treatment dose may be increased if FENO is greater than 26 ppb; the inhaled corticosteroid treatment dose may be decreased if FENO is less than 16 ppb or less than 26 ppb on two consecutive occasions; FENO less than 30 ppb suggests that asthma exacerbation is unlikely to occur; if FENO increases by more than 4 ppb, the increase is most likely due to inflammation rather than normal variability.

For children, FENO should be less than 15 to 25 ppb, depending on age and atopy; FENO of 49 ppb predicts relapse of symptoms; a FENO of 22.9 ppb predicts an asthma exacerbation; FENO greater than 22 ppb during the reduction of an inhaled corticosteroid dose suggests that the reduction has not been successful; for children with asthma, using FENO for ICS dose titration every 3 months for 1 year may be superior to the conventional treatment guided by symptoms alone.

Alternatively, the recommendations of the American Thoracic Society/European Respiratory Society (ATS/ERS) may be adopted when interpreting the FENO values obtained. For example, FeNO less than 25 ppb (< 20 ppb in children) may be used to indicate that eosinophilic inflammation and responsiveness to corticosteroids are less likely; FeNO greater than 50 ppb (> 35 ppb in children) may be used to indicate that eosinophilic inflammation and, in symptomatic patients, responsiveness to corticosteroids are likely; FeNO values between 25 ppb and 50 ppb (20-35 ppb in children) should be interpreted cautiously and with reference to the clinical context; recommend accounting for persistent and/or high allergen exposure as a factor associated with higher levels of FeNO; recommend the use of FeNO in monitoring airway inflammation in patients with asthma; using the following values to determine a significant increase in FeNO: greater than 20% for values over 50 ppb or more than 10 ppb for values lower than 50 ppb from one visit to the next; using a reduction of at least 20% in FeNO for values over 50 ppb or more than 10 ppb for values lower than 50 ppb as the cut point to indicate a significant response to antiinflammatory therapy.

In some embodiments, the inhaler 2 is configured to receive data from external source (i.e. a remote device 236). The inhaler 2 comprises a communication module configured to transmit and/or receive data from the remote device. The communication module may comprise any suitable form. The communication module may be configured to be physically connected to the external data source (e.g. via a wire). A physical interface 238 (see figure 7) is provided. The physical interface 238 comprises a USB port. The physical interface 238 may provide a charging port for the battery 40. Additionally or alternatively, the communication module may be configured for wireless communication (e.g. via Bluetooth (RTM) or Wi-fi (RTM), NFC or GSM or other suitable wireless/radio communication). The communication module may be configured to communicate with remote devices via the internet.

The remote device may comprise any suitable computer or storage medium, including inter alia: a personal computer; laptop; mobile phone; tablet; remote server; internet connected device; or remote sensor. The inhaler device may download or upload data, e.g. for receipt from or transmission to the remote device.

The external data may comprise the one or more factors indicative of the severity of the user's condition. This may comprise one or more of:

- Previous or historical data relating to one or more diagnostic test. For example, this may comprise historical spirometry or FeNO tests.
- Previous or historical data relating to one or more qualitative input. For example, this may include whether the user has experienced "mild", "moderate" or "severe" symptoms on a particular day.
- Previous or historical data relating to usage of medicaments and the dosage regime thereof. This may comprise the name of the medicament(s); the period of time they have been using the drug; and dosage thereof during the period of time. For example, this may comprise data indicating X drug has been used for Y months at a dosage of Z per day.
- Local environmental conditions. The environmental condition may comprise one or more of, inter alia: temperature; humidity; pollution levels; pollen levels; or UV index. This may be used where it is not possible to determine environmental conditions using sensors on the inhaler 2.

The historic test data may be transferred electronically in a direct manner (i.e. electronically from the remote device). For example, the healthcare professional may upload data from their computer system. The inhaler 2 may retrieve data from the internet or other remote server. The inhaler 2 may be configured to periodically communicate with the remote device to download any relevant data. For example, if new test data is uploaded by the healthcare professional, the inhaler 2 may automatically retrieve the data. The inhaler 2 may communicate with the remote device to download software/firmware updates.

Additionally or alternatively, the user may manually input data. For example, the inhaler 2 may link with an app on the user's mobile phone or tablet, etc. The user may input historical data manually or download the data from a remote device and transfer the data into the app. The user may be provided with a health questionnaire. The data in the questionnaire is processed by the app and transmitted to the inhaler 2. The questionnaire may be provided before first use of the inhaler 2 and/or periodically throughout use thereof.

In some embodiments, the data (or a backup copy thereof) may be transferred from the previously used inhaler device. This may be useful if the user acquires a new device.

In some embodiments, the inhaler 2 is configured to communicate with a "reliever" inhaler. "Reliever" inhalers comprise a fast-acting medicament configured to reduce inflammation (e.g. a short-acting $\beta_2$ agonist). However, the "reliever" medicaments typically only effective for a short period of time. Usage of the "reliever" inhaler is indicative that a dose of "preventer" medicament (e.g. a corticosteroid) is not sufficient. The inhaler 2 is operatively connected to the reliever inhaler (i.e. via the communication module) such that the reliever inhaler may transmit usage data thereto.

In the next step 240, the inhaler 2 is configured to process the indicators to determine the appropriate dosage regime. The exact determination of how the indicators may influence the dose of a particular medicament will be known by the person skilled in the art and will not be described in detail here. The processing step 240 may use an artificial neural network (ANN). This allows determination of the dosage regime using many independent or interdependent indicators. The indicators may be given a weighting according to their magnitude of influence on the severity of the condition. For example, FeNO measurements may provide a stronger indication of condition severity than the local temperature - FeNO may therefore be assigned an increase weighting.

The dosage regime may determine the size of a dose (i.e. the volume/mass thereof). The dosage regime may determine the frequency of the dose (e.g. how many times a day, or how many individual doses dispensed at a time). The dosage regime may provide a respective dosage regime for a plurality of different medicaments. The inhaler 2 may record which medicaments are used with the inhaler 2 (e.g. via the validation chip on the canister 24), and provide a dosage regime accordingly.

The inhaler 2 may use any or all of the indicators described above to determine the dosage regime. In some embodiments, only the onboard sensors may be used (i.e. so the inhaler 2 can operate "offline"). In some embodiments, only data from the remote device may be used.

In some embodiments, the data is processed by the inhaler 2 (e.g. via an onboard processor/controller). The data is thus retained within the inhaler and may be used "offline". The inhaler 2 may comprise a database and/or ANN and/or machine learning in order to process the data. The raw and/or processed data (i.e. the dosage regimen) may sent to and/or recorded by the remote device.

In some embodiments, indicators/variables are sent to the remote device (i.e. before processing). The remote device may be operated by a healthcare professional and/or other authorised person. The healthcare professional may then analyse the indicators to determine the dosage regime. The process may be automated and the healthcare professional may merely approve the dosage regime. In some embodiments, the process may be semi-automated. For example, the healthcare professional may select from a plurality of pre-generated dosing regimen. Alternatively, the healthcare professional may manually review the indicators. The healthcare professional may then determine the dosage regimen accordingly (e.g. using their expertise and/or according to predetermined regime). Once the appropriate dosage regimen is determined, the dosage regimen is subsequently sent to the inhaler.

The inhaler 2 may be configured to receive messages or notifications from the remote device. The messages/notifications may be predetermined and/or custom. For example, the healthcare professional may send a message to the patient to indicate over-usage of a reliever inhaler.

In some embodiments, the data may be processed remotely 242. This allows a more complex dataset to be processed. The inhaler 2 may collate the indicators. The inhaler 2 sends the indicators to the remote processor. The remote processor may be provided by the user's device (e.g. the phone app). The remote processor may comprise an internet connected server. This may allow collections of a plurality of user's data, for example, to train the ANN. In some embodiments, a mixture of onboard and remote processing may be performed.

In some embodiments, remote processor comprises a local device (e.g. the remote device is located in the same room/building as the inhaler 2). The inhaler 2 may connect to the remote processor via a direct link, for example, Wifi or a physical connection. This prevents the transmissions of data through the internet or other unsecured connections, thereby helping to increase data security.

In the next step 244, the determined dosage regime is recorded by the inhaler 2. Where the data is processed onboard, the inhaler 2 may upload the dosage regime to a remote service.

In the next step 246, the dispensed dose is adjusted according to the determined dosage regime. During the dispensing cycle, the volume of the dosing chamber 56 is adjusted according to the dosage regime. The movement of the piston 64 is controlled accordingly. The determine dosage is then dispensed by the inhaler 2. Once dispensed, the inhaler 2 may communicate with the canister 24, to update the dose remaining/used.

The inhaler 2 directly measures lung functionality of the user and adjusts the dosage of the medicament accordingly. The size of the dosing chamber 56 and/or movement of the piston 64 is controlled according to one or more sensors on the inhaler 2. The inhaler 2 thus provides a dosage regime feedback mechanism.

In the present embodiment, current and/or historic data relating to the measured indicators and the determined dosage are stored on the inhaler device memory. Thus, data is securely retained on the device. The data may uploaded to remote device. This may occur at a predetermined time interval, and/or when the device memory is full or nearly full. The device memory may then be erased and the data is logged during use accordingly.

In some embodiments, the inhaler 2 comprises a training mode. The training mode is configured to assess the user's breathing technique to determine whether they are inhaling in a manner which provides effective administration of the medicament.

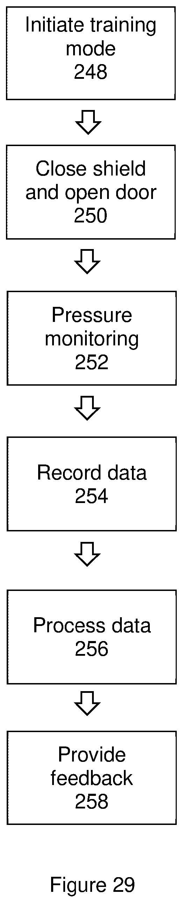

The training mode is described with reference to figure 29. In a first step 248, the training mode is initialised. The training mode may be manually initialised by the user or a healthcare professional. Additionally or alternatively, the training mode is automatically initialised upon first use of the inhaler 2, or the first use of an inhaler 2 after a predetermined period or non-use. Additionally or alternatively, the training mode may be initialised at a predetermined time period. For example, the training mode may be automatically initialised once a week.

In the next step 250, the shield 82 is moved to the closed position (if open). The door 154 is moved to the open position (if closed) and channel outlet 156 is obscured. The valve member 46 is provided in the "dispensing" position.

In the next step 252, the pressure sensing system 124 monitors the pressure in the channel 34. The pressure sensing system 124 may therefore determine the flowrate through the channel 34. A notification may be given to the user to begin inhalation such as an audible noise.

During this process, the data is recorded 254. The data may be stored on volatile/non-volatile memory in the inhaler 2. The data may comprise the flowrate at a given time. No medicament is dispensed during the training test.

The data is then processed 256. The relevant indicators are extracted from the data. The indicators may comprise one or more of: the maximum flow rate; the total duration of exhalation; the average flow rate over the exhalation; or the variance of the flow rate over the exhalation.

The indicators are compared with predetermined ideal values. If one or more of the indicators lies outside the ideal values, then the inhaler 2 indicates the inhalation technique is not effective.

In some embodiments, if the maximum flow rate exceeds a predetermined flowrate, then inhalation may be deemed not effective. This may indicate the user is breathing too sharply, which results in the medicament impacting on the throat and not efficiently entering the lungs. The predetermined maximum flowrate may be 100L/min; or 60L/min, or 40L/min or less.

In some embodiments, if the maximum flow rate drops below a predetermined flowrate, then inhalation may be deemed not effective. This may indicate the user is breathing too slowly, which results in the medicament not entering deep enough into the lungs. The predetermined flowrate may be 20L per minute; preferably 15L per minute or 10L per minute.

The ideal maximum flow may therefore lies with a predetermined range. The ideal range may be between 10L and 35L per minute, preferably, between 15L and 30L per minute.

In some embodiments, if the duration of the exhalation lies outside a predetermined time range, then inhalation may be deemed not effective. A short duration or long duration may be indicative the user is exhaling too quickly or too slowly respectively. The predetermined range may be 2 second and 10 seconds, preferably between 3 second and 7 seconds.

In some embodiments, if the average flowrate lies outside a predetermined flowrate range, then inhalation may be deemed not effective. Again, this may be indicative the user is exhaling too quickly or too slowly respectively. The predetermined range may be between 15L and 30L per minute.

In some embodiments, if the flowrate over the exhalation exceeds a predetermined variance, then inhalation may be deemed not effective. A high flowrate variance may indicate the user is breathing in an uncontrolled way or may be inhaling and exhaling. The predetermined variance range may be 5L and 15L per minute.

The inhaler 2 may use any one or combination of the above indicators to determine effective breathing technique. The indictors may be weighted according to their real-world effect on the administration of the medicament. The ideal values may also vary according to a user a profile. For example, the ideal values may be vary depending on one or more of: the age; the sex; the health; or the weight of the user. Additionally or alternatively, the ideal values may be determined using the sensor data (i.e. spirometry, NO, environmental sensors, reliever inhaler usage) and/or the determined medicament dose.

In the next step 258, the inhaler the inhaler 2 provides feedback to the patient. If the indicators lie within their respective ideal values, the inhaler 2 may indicate the training test was successful. If one of or more of the indicators lie outside their respective ideal values, then the inhaler may notify the user accordingly. The notification may indicate to the user to breathe more quickly, more slowly or more evenly. For example, the maximum flow rate exceeds the ideal value, the inhaler 2 notifies the user they should breathe more slowly. If the training test was not successful, the inhaler 2 may notify the patient that a further training mode test is required. The process may repeat until a successful test is completed.

The inhaler 2 may record any test data and/or the outcome of each test. The healthcare professional may be able to review the data and provide any feedback to the user.

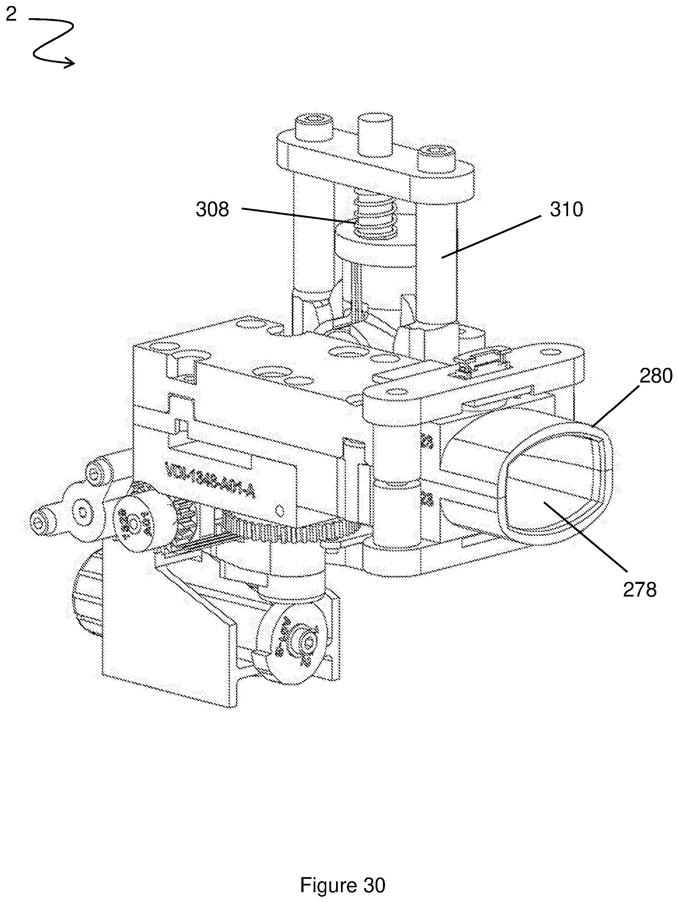

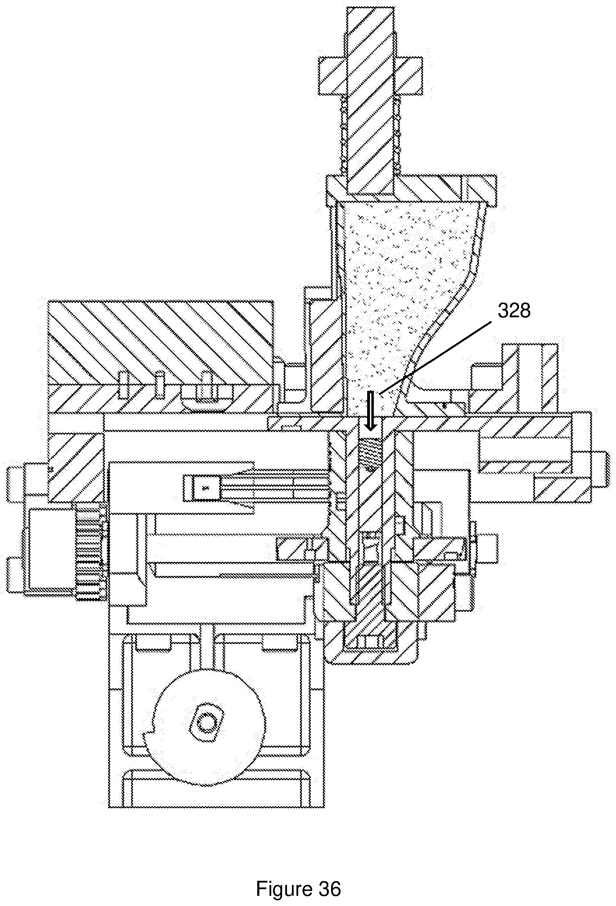

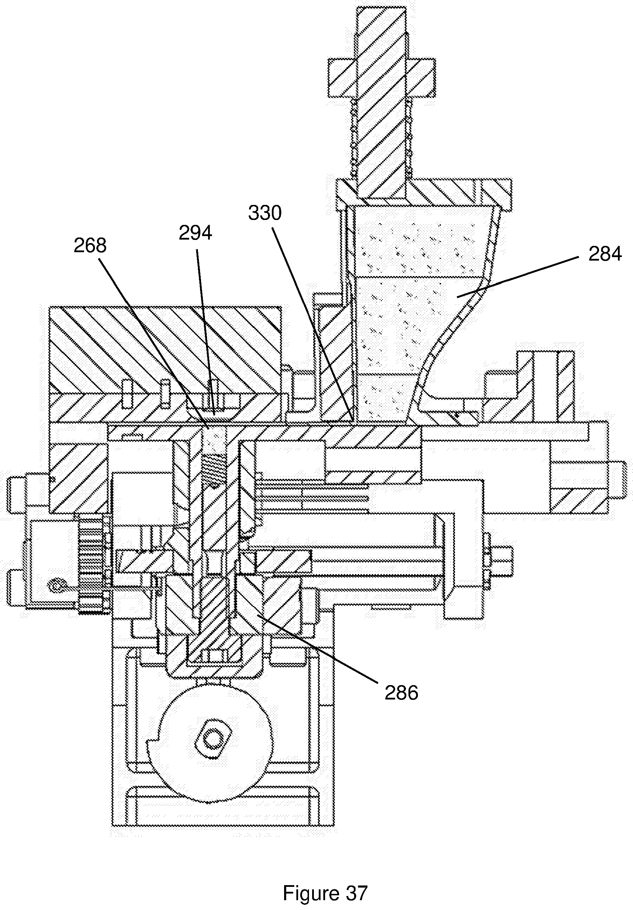

A second embodiment of the inhaler device 2 is shown in figures 30-41. In this embodiment, the inhaler 2 is configured to dispense a "dry" medicament. The inhaler 2 may therefore provide a Dry Powder Inhaler (DPI). The medicament is solid (i.e. non liquid/gaseous). The medicament is provided in a powder or granular form. The medicament is therefore fluent.

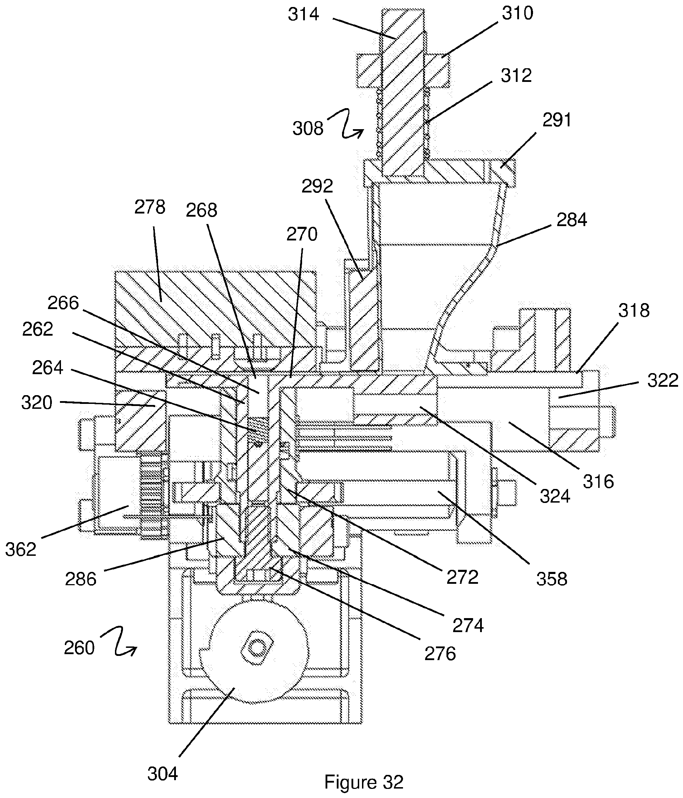

The inhaler 2 comprises a variable dose mechanism 260 configured to dispense a predetermined variable dose of the medicament. As best seen in figure 32, variable dose mechanism 260 comprises a dosing chamber 262. A piston 264 is received within the chamber 262. The piston 264 is movable to define a cavity 266. The volume of the cavity 266 is therefore variable to define a variable dose of the medicament to be delivered to the user. The piston 264 is continuously movable. The volume of the cavity 266 is therefore continuously variable (i.e. may take any value between a minimum and maximum value).

The dosing chamber 262 comprises an open end 268 to allow ingress/egress of the medicament. A flange 270 surrounds the open end 268. The flange 270 may provide a plate or the like. The flange 270 extends perpendicular to the cavity 266/movement of the piston 264. A collet or sleeve 272 surrounds and grips the dosing chamber 262. A carrier 274 is mounted to an opposing end of the dosing chamber 262 from the open end 268. The carrier 274 comprises a plug 276 to seal the opposing end of the dosing chamber 262. The plug 276 may comprise a screw or the like. The screw 276 thus helps to retain the piston 264 within the dosing chamber 262. The collet 272 is interposed the flange 270 and the carrier 274.

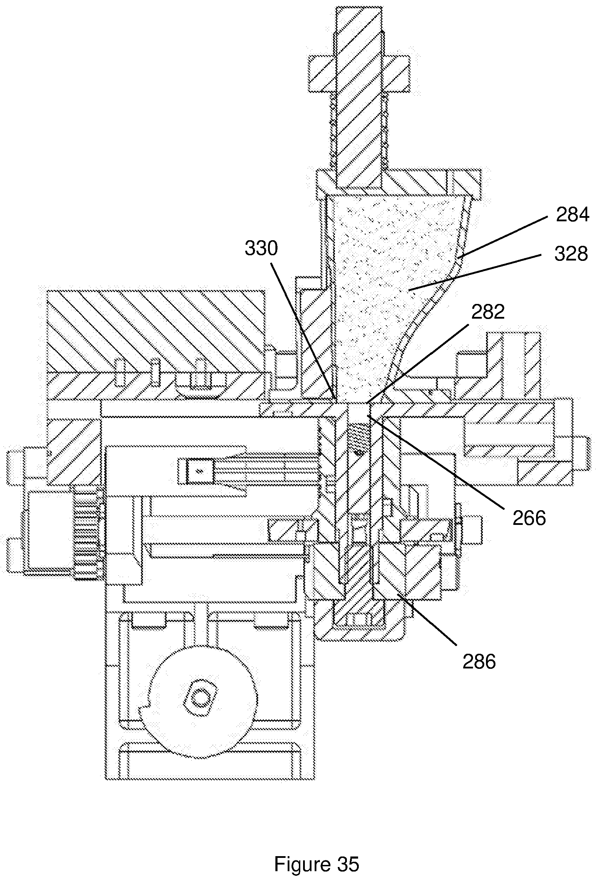

The variable dose mechanism 260 is movably mounted to the inhaler 2. The variable dose mechanism 260 is movable between a first position where the open end 268 is aligned with an airway 278 operatively connected to a mouthpiece 280 (best seen in figure 32), and a second position where the open end 268 is aligned with an outlet 282 of a medicament reservoir 284 (best seen in figure 35). The first position provides a dispensing position, and the second position provides a filling position.

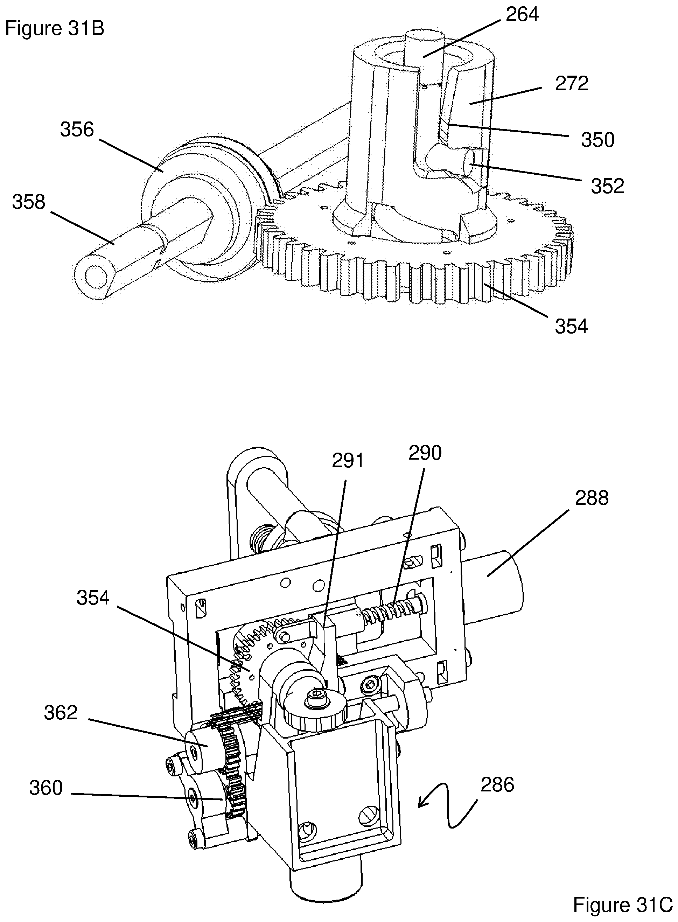

As best seen in figure 31B, the sleeve 272 comprises a track 350 therein. The track 350 comprises an elongate slot. A pin 352 extends laterally from the piston 264. The pin 352 is received within the track 350. The track 350 extends at least partially in the axial direction of the piston 264 (i.e. in a helical direction). Thus, movement of the pin 352 along the track 350 provides axial movement of the piston 264.

The sleeve 272 is mounted to a gear 354. The gear 354 is provided at the base of the sleeve 272. The gear 354 is configured to operatively engage (i.e. mesh) with a gear 356 mounted to a shaft 358. The shaft is effected by a motor 360 (see figure 31C). Thus rotation of the motor 360/shaft 358 effects axial movement of the of the piston 264. The motor 360 is operatively connected to the shaft via a gearbox 362.

The dosing chamber 262, collet 272 and the carrier 274 are movable together as a unit, thus defining a carriage 286. As best seen in figure 31C, movement of the carriage 286 is effected by an actuator 288. The actuator comprises a motor or the like. A screw drive 290 operatively connects the motor 288 and the carriage 286. The screw drive 290 provides linear translation of the carriage 286 upon rotation of the motor 288. The carriage 286 is therefore translatable in a first direction. The carriage 286 comprise an arm 291 configured to receive the screw drive 290. The arm 291 comprises a screw thread or the like.

The reservoir 284 is provided above the dose mechanism 260 in use. Thus, the medicament may flow under gravity into the dosing chamber 262. A tilt sensor (not shown) may be provided to determine the orientation inhaler 2. Filling of the dose mechanism 260 may be prevented if the orientation is not within a predetermined range. This ensures correct filling of the dosing chamber 262.

The reservoir 284 comprises a funnel or tapered shape. This funnels the medicament toward the outlet 282. The reservoir 284 comprises a closure 290. The closure 290 may be removable to allow refilling of the reservoir 284. In some embodiments, the complete reservoir 284 may be removable to allow a replacement reservoir. In some embodiments, a container (e.g. a capsule or flexible bag) may be received within the reservoir 284.

A vibration mechanism 292 is provided adjacent the reservoir 284. The vibration mechanism 292 is configured to vibrate medicament in or adjacent the outlet 282. This help to provide flow of the medicament from the outlet 282 and into the dose mechanism 260. The vibration mechanism 292 may comprise any suitable means, for example, a motor comprising an unbalanced mass and/or piezoelectric vibrator.

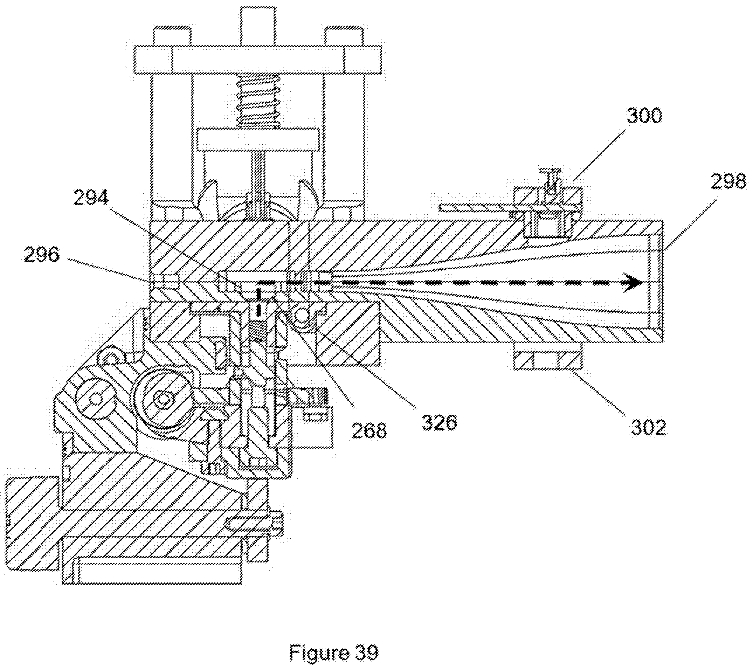

As best seen in figure 39, the airway 278, comprise a first inlet 294 configured to overlie the open end 268 of the dosing chamber 262. The first inlet 294 may be approximately the same size/shape as the open end 268. The airway 278 comprises a second inlet 296. The second inlet 278 opens to the ambient environment. An outlet 298 is provided in an opposing end of the airway 278 (i.e. axially spaced therefrom). The outlet 298 is provided within the mouthpiece 280. During inhalation by the user, air is drawn in through the second inlet 296 and out through the outlet 298.

The airway 278 increases in width/diameter toward the outlet 298. The airway 278 therefore tapers toward the first/second inlets. This creates a Venturi nozzle like arrangement. This may create a low pressure region adjacent the first inlet 294, thereby helping to draw the medicament out from the dosing chamber 262.

The inhaler 2 comprises a pressure sensing system 300. The pressure sensing system 300 comprises a pressure sensor configured to determine the air pressure within the airway 278. The pressure sensing system 300 is provided adjacent the outlet 298 (i.e. adjacent the mouthpiece 280). This provides accurate measurement of the air pressure change caused by inhalation/exhalation of the user. The pressure sensing system 300 may be provided on an upper side of the airway 278 in use (i.e. an opposing side to the dose mechanism 260). The pressure sensing system 300 is mounted to a bracket 302 surrounding the airway 278. The pressure sensing system 300 is otherwise substantially the same as pressure sensing system 124 of the first embodiment and will not be described further.

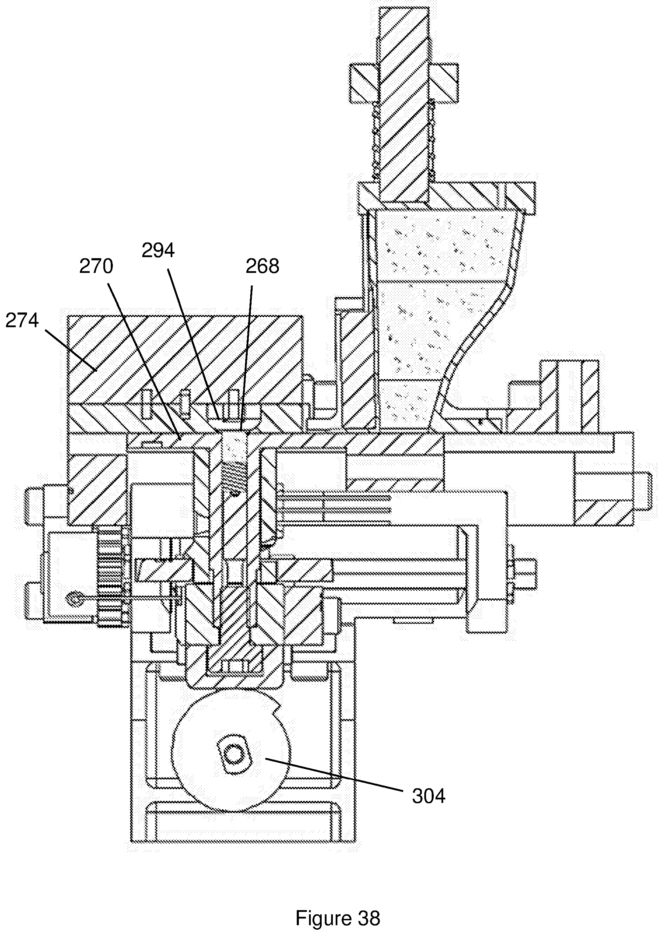

The carriage 286 is configured to be moved toward/away from the airway 278 and/or the reservoir 284. The carriage 286 is therefore configured to be moveable in a second translation direction. The second direction is substantially perpendicular to the first direction. The second direction is a vertical direction in use. Movement in the second direction is effected by an eccentric cam 304. The eccentric portion of the cam 304 is configured to engage the carrier 274, moving the carriage 286 toward the airway 278 (see figure 38). The cam 304 is effected by an actuator 306 (e.g. motor). The respective actuators 288,306 for the first and second directions are independently actuatable. Thus, movement in the first and second directions may be performed independently.

A biasing mechanism 308 biases the carriage 286 toward a position displaced away from the airway 278. The biasing mechanism 308 is operatively mounted to the reservoir 284. Thus, the biasing force of the biasing mechanism 308 is transferred through the reservoir 284. This biases the reservoir 284 against the flange 270 of the dosing chamber 262, thus ensuring an effective seal therebetween. This prevents leakage of medicament.

The biasing mechanism 308 is mounted to a bracket 310. The bracket 310 extends over the upper end of the reservoir (i.e. the closure 290). The biasing force is thus provided by compression of the biasing mechanism 308. In the present embodiment, the biasing mechanism 308 comprises a helical spring 312. The spring 312 is supported on a shaft 314. However, it can be appreciated, the biasing mechanism 308 may comprise any suitable resilient bias means, for example: a gas spring; leaf spring; torsion spring; compressible elastic element etc. In some embodiments, the biasing force may be provided a tension force (i.e. by a stretching of a spring or elastic element).

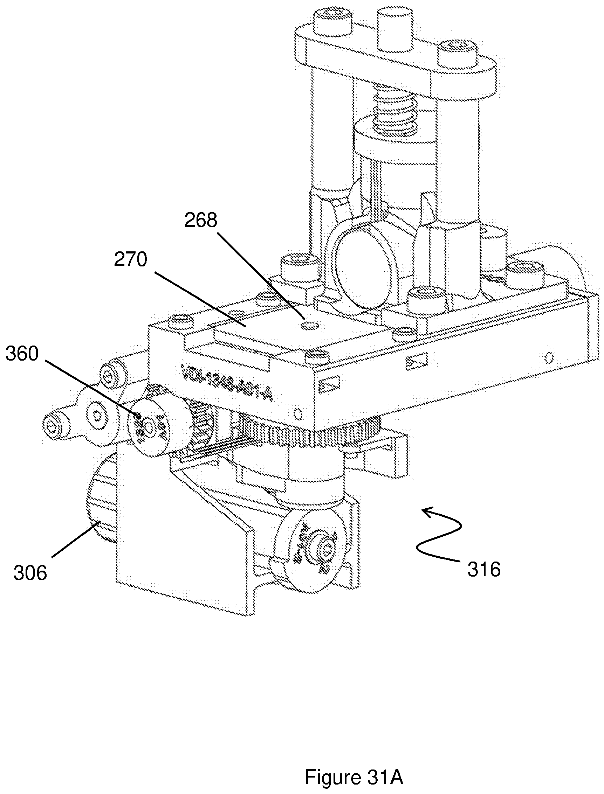

As best seen in figures 31 and 32, the dosing chamber 262 is received within a cavity 316 in the chassis 318 of the device 2. The flange 270 abuts a first end 320 of the cavity 316 in the first position and a second end 322 in the second position. The extent of movement of the carriage 286 in first direction is limited by the cavity 316. The cavity 316 and the airway 278 constrain movement in the second direction. A guide member 324 is attached to the flange 270. The guide member 324 comprise a sleeve or hollow cylinder configured to receive a shaft mounted to the chassis 318. The guide member 324 is received in a second cavity 326 (see figure 39).

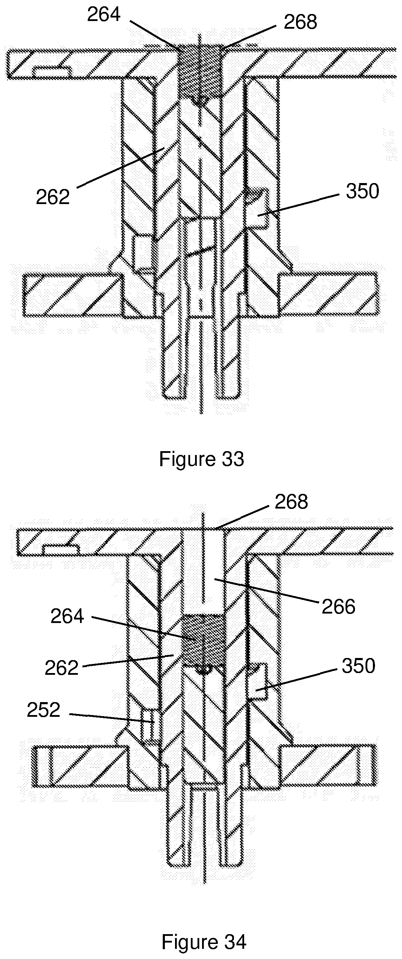

Operation of the device will now be described with reference to figures 33 to 41. Initially the piston 264 is provided in a "home" position. The piston 264 is provided adjacent the open end 268 of the dosing chamber 262. The cavity 266 comprises an effective volume of zero. In this position, the piston 264 may partially protrude from the dosing chamber 262. The pin 252 is provided at an upper end of the track 350.

In the next step shown in figure 34, the piston 264 is moved down the dosing chamber 262 to define a cavity 266 of predetermined volume. The predetermined volume may be determined by any of the means as previously described, for example, using the dosage adjustment system described with reference to figure 28. The pin 252 is provided at a lower end of the track 350.

Referring to figures 35 and 26, the carriage 286 is moved to a position beneath the reservoir 284. The reservoir outlet 282 and the open end 268 are aligned, allowing transfer of the medicament. Medicament 328 flows into the dosing chamber 262, filling the cavity 266 to provide the predetermined dose. The vibration 292 is activated to aid with flow of the powder. The tilt sensor may monitor the orientation of the device. If the orientation lies outside desired parameters, then a notification may be provided to the user to correct the orientation.

It can be appreciated that movement of the piston 264 may be performed before, during or after translation of the carriage 286, provided that sufficient time is provided to allow filling of the cavity 266.

The carriage 286 is translated such that the open end 268 and the first airway inlet 294 are aligned. As the carriage 286 is moved away from the reservoir 284, the lower edge 330 levels off medicament in the cavity ensuring a correct dose amount. A resilient seal may be provided at the lower edge 330 to ensure effective sealing against the flange 270.

Referring to figures 38 and 39, the cam 304 is rotated to bring the flange 270 into engagement with the airway 278. A seal is provided between the open end 268 and the first airway inlet 294, thus ensuring no medicament is lost. The inhaler 2 is now in "ready" position. A notification may be provided to the user to indicate the ready state. The pressure sensing system 300 is configured to monitor the air pressure in the airway 278, as previously described.

Once an inhalation event is detected, the piston 264 is driven to reduce the effective volume of the cavity 266 to zero. This pushes the medicament into the airway 278. The medicament is then entrained within the air flowing between the second inlet 296 and the outlet 298. The piston 264 may at least partially protrude into the airway 278.

In a first embodiment, the ejection rate of the medicament (i.e. the volume of medicament ejected in a given time period) remains constant, independently of the dose to be dispensed. The movement of the piston 264 may therefore be fixed, providing a simple and reliable arrangement.

In a second embodiment, the ejection rate of the medicament is determined according to the dose to be given. For example, the ejection rate may be proportional to the amount of dose. The ejection of the medicament is therefore completed within a given time, independent of the total volume of the dose.

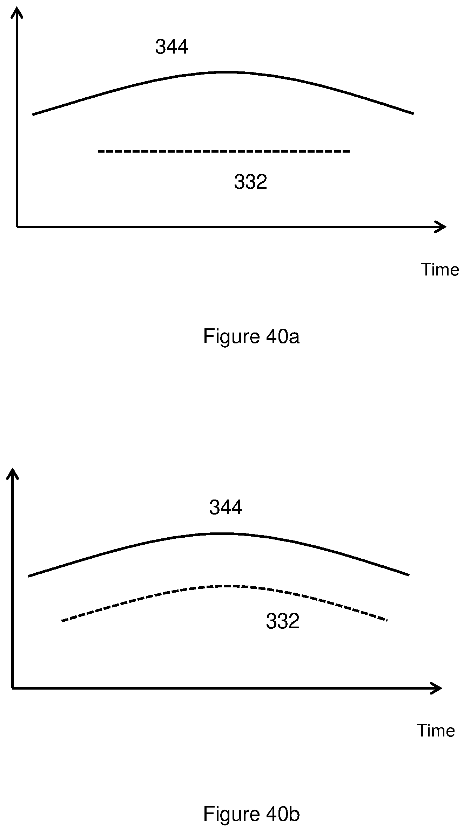

As shown in figure 40a, the ejection rate 332 is constant and independent of the flow profile 334 (i.e. the flow rate of inhalation) of the user.

In a third embodiment, the ejection rate of the medicament is determined according to the flow profile of the user. In the example shown in 40b, the ejection rate 332 is proportional to the flow rate 344 of inhalation of the user. A high flow rate results in a high ejection rate and vice versa. The ejection rate thus "tracks" the flow profile. A simple regime may be described by:

$$ejection\ rate = K[inhaltion\ flow\ rate]$$

Where the **K** is an arbitrary constant. The flow rate of the user is measured by the pressure sensing system 300. The flow may be measured by determining the pressure drop in the airway, and thus the pressure drop can be seen as a proxy of the flow rate. This can be performed in real time, thus allowing real time adjustment of the ejection rate/speed of the piston 264. Such a regime allows the correct dose to be dispensed according to the user's flow profile, thus ensuring the dose is not dispensed too quickly or too slowly.

In other embodiments, the correlation between the ejection rate and the flow rate may be determined by more complex regimes (e.g. using higher order polynomials, exponentials, series etc.). Determination of a suitable regime will be understood by a person skilled in the art.

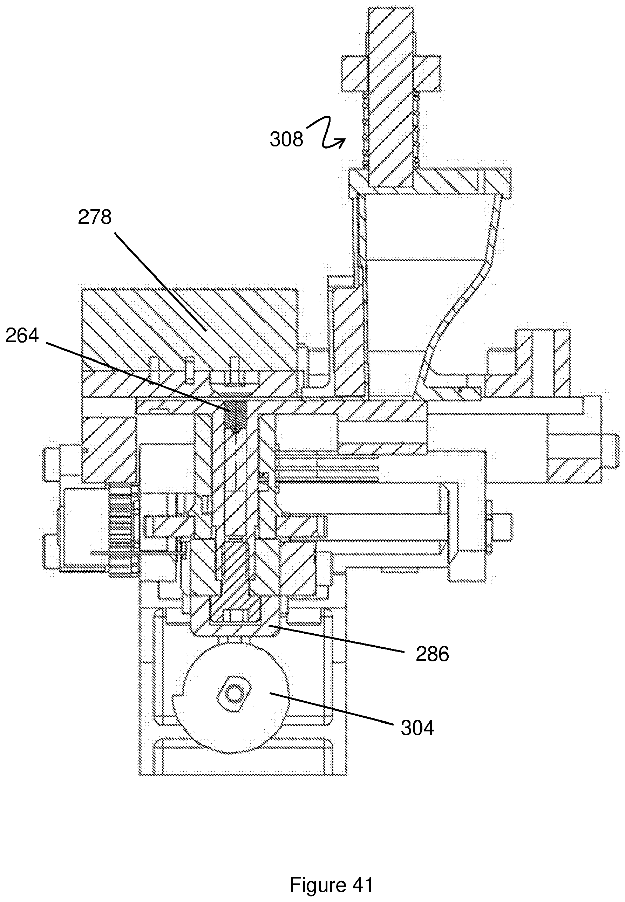

Once the dose has been fully dispensed and/or the flow rate has fallen below a given threshold, the dispensing sequence is considered complete. Referring to figure 41, the cam 304 is rotated out of engagement with the carrier 274. The biasing mechanism 308 biases the carriage 286 away from the airway 278. The piston 264 remains at the "home" position, awaiting the next dispensing cycle.

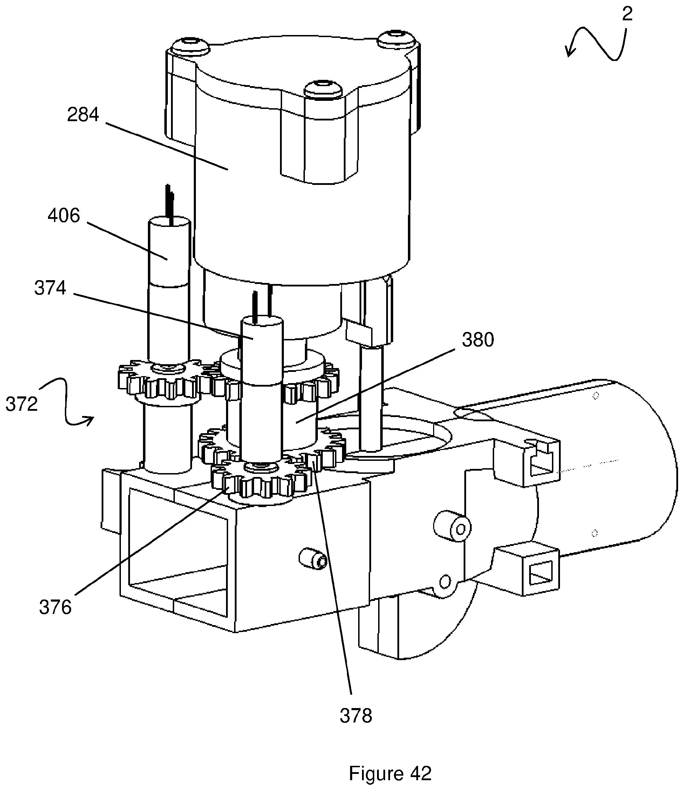

A third embodiment of the inhale 2 is shown in figures 42-52. The variable dose mechanism 260 is mounted directly beneath/adjacent the medicament reservoir 284. The mechanism is thus provided above the airway 278 in use (i.e. the mechanism 260 is interposed the airway 278 and the reservoir 284). A piston 264 is received within a dosing chamber 262. The piston 264 comprises a head portion 370. The head portion 370 comprises a resiliently deformable material. The material may comprise an elastomer/polymer (e.g. silicone, HNBR, Santoprene (RTM) etc.). The dosing chamber 262 may comprise a low friction material. The material may comprise the body of the chamber 262 and/or be provided as a coating thereon. The material may comprise a ceramic (e.g. polished ceramic). The material may comprise a low-friction polymer.

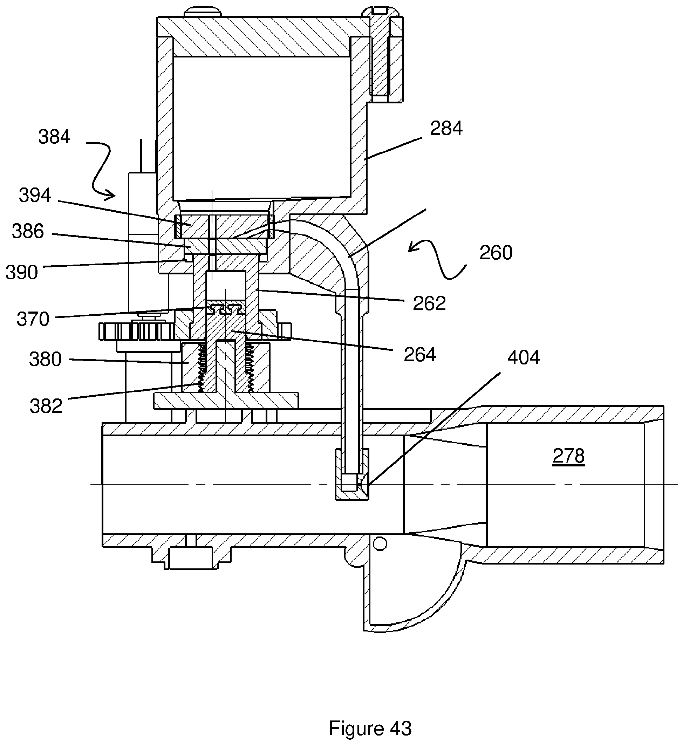

The position of the piston 264 is variable with the dosing chamber 262 via an actuation mechanism 372 (see figure 42). The mechanism 372 comprises an actuator 374 configured to drive a gear 376. The gear meshes with a second gear 378. The second gear 378 is mounted on a sleeve 380 surrounding the piston 264. The sleeve 380 is rotatable (i.e. relative to the airway 278/reservoir 284). Referring back to figure 43, the sleeve 380 and the piston 264 are operatively connected by co-operating helical threads 382. Thus, upon rotation of the sleeve 380, the piston 264 is axially driven along the dosing camber 262. The gears 376/378 provided a gearbox.

The device 2 comprises a valve mechanism 384 configured to control the flow of medicament from the reservoir 284 and in/out of the dosing chamber 262. The valve mechanism 384 comprises a rotatable portion 386. The rotatable portion 386 comprises a rotatable disc or the like. The disc 386 is mounted or otherwise fixed to the dosing chamber 262. The disc 386 and the dosing chamber 262 are connected by a plurality of plugs 388 provided on the dosing chamber 262. However, it can be appreciated any suitable means may be used to provide a connection (e.g. fasteners, welding, adhesive etc.).

The disc 386 is supported within the chassis of the device 2. The chassis comprises a flange/rim 388 configured to retain the disc 386. A bearing 390 is provided between the flange 388 and the disc 386. A channel 392 extends through the disc 386. The channel 392 extends perpendicular to the plane of the disc 386. The channel 392 is aligned with an outlet in the dosing chamber, thus providing a single continuous channel 392.

The valve mechanism comprises a second disc 394. The second disc 394 is fixed to the chassis. The second disc 394 is therefore immovable relative to the reservoir 284. The second disc 394 is sealed to the chassis via a sealing element 396. The sealing element 396 may comprise a gasket or the like. A first channel 398 extends through the second disc 394. The first channel 398 extends substantially perpendicularly to the plane of the second disc 394. A second channel 400 extends through the disc 394. The second channel extends across/obliquely the second disc 394. In the use, the channel 392 in the first disc 386 extends between the dosing chamber 262 and the second disc 394. The first channel 398 in the second disc extends between the reservoir 284 and the first disc 386. The second channel 400 extends between the first disc 386 and an outlet 402. The outlet 402 is fluidly connected to a nozzle 404 (see figure 43).

One or both of discs 386/394 comprises a low-friction material. The material may comprise the body of the disc 386/394 and/or be provided as a coating thereon. The material may comprise a ceramic (e.g. polished ceramic). The material may comprise a low-friction polymer. In some embodiments, one or both of the discs 386/394 may form part of and/or be integral to the dosing chamber 262 and/or the reservoir 384 respectively. For example, the discs 386/394 may be provided as a coating thereon, or integrally moulded therewith. The use of a low friction disc-type arrangement allows movement of the valve, whilst maintaining a close seal therewith. It can be seen the discs form a rotary valve. The rotary valve rotates about a substantially vertical axis in use (i.e, perpendicular to the airway 278).

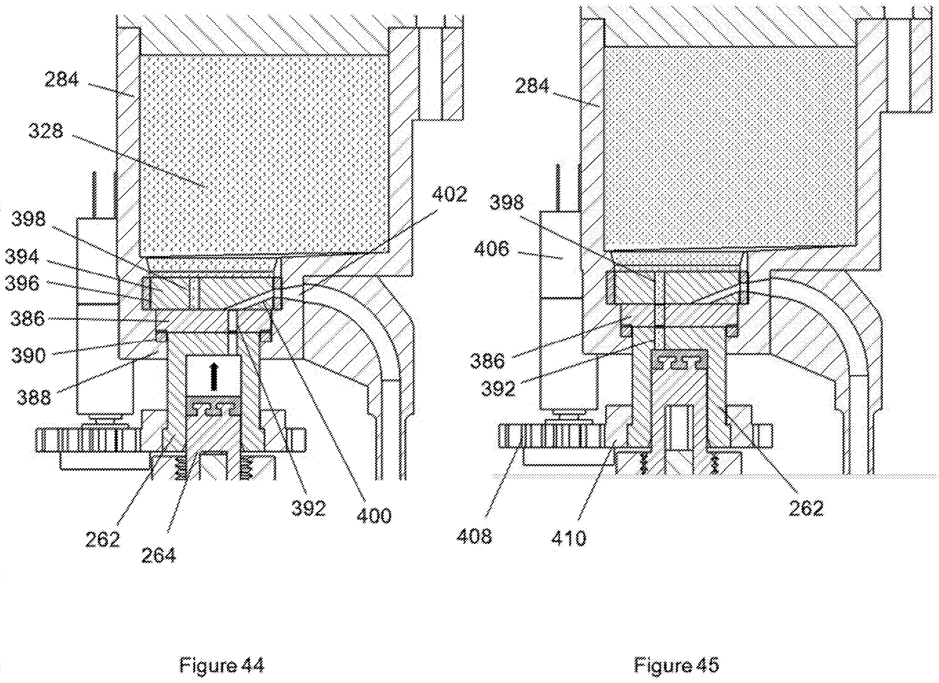

Operation of the variable dose mechanism 260 will now be described with reference to figures 44-52.

In a first stage shown in figure 44, the piston 264 is axially moved along the dosing chamber 262 toward the reservoir 284. This expels any excess air in the system and/or clear the nozzle 404.

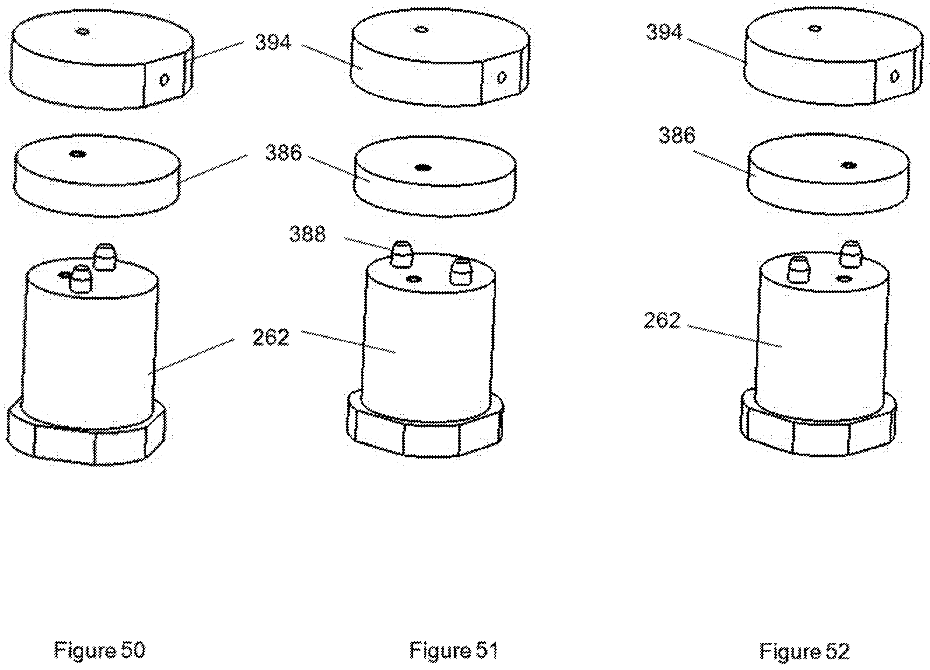

As shown in figure 45, the piston 264 is moved into abutment with the end of the dosing chamber 262. The dosing chamber 262 and the first disc 386 are rotated in unison such that the channel 392 is aligned with the first channel 398 in the second disc 394. Thus, the reservoir 284 and the dosing chamber 262 are fluidly connected, and medicament is free to flow into the dosing chamber 262. This arrangement is shown in figure 50. Rotation of the first disc 386 is effected by an actuator 406. The actuator 406 is operatively connected to a first gear 408. The first gear meshes with a second gear 410 on the dosing chamber 262. The second gear 410 surrounds the dosing chamber 262 (e.g. provides a flange/collar).

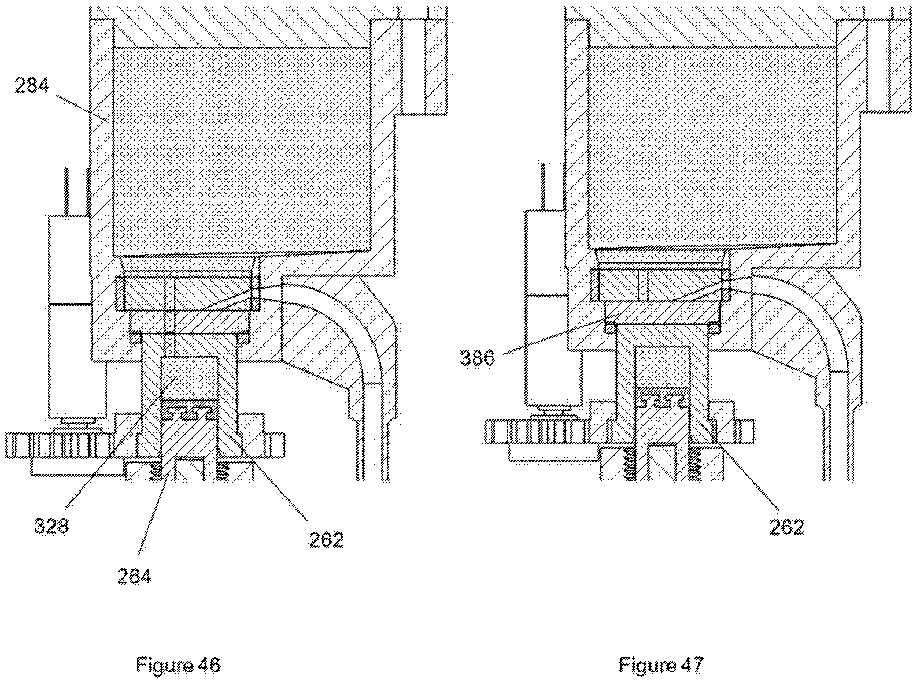

As shown in figure 46, the piston 264 is moved away from the reservoir 284, thereby drawing medicament 328 into the dosing chamber 262. The piston 264 moves until a predetermined dose of medicament is provided in the dosing chamber 262.

In the next stage shown in figure 47, the dosing chamber 262 and first disc 386 are rotated such that the channel 392 in the first disc 386 is no longer aligned with the first channel 398 in the second disc 394. The dosing chamber 262 and the reservoir 284 are thus fluidly disconnected. The dose in the dosing chamber 262 is thus fixed. This arrangement is shown in figure 51.

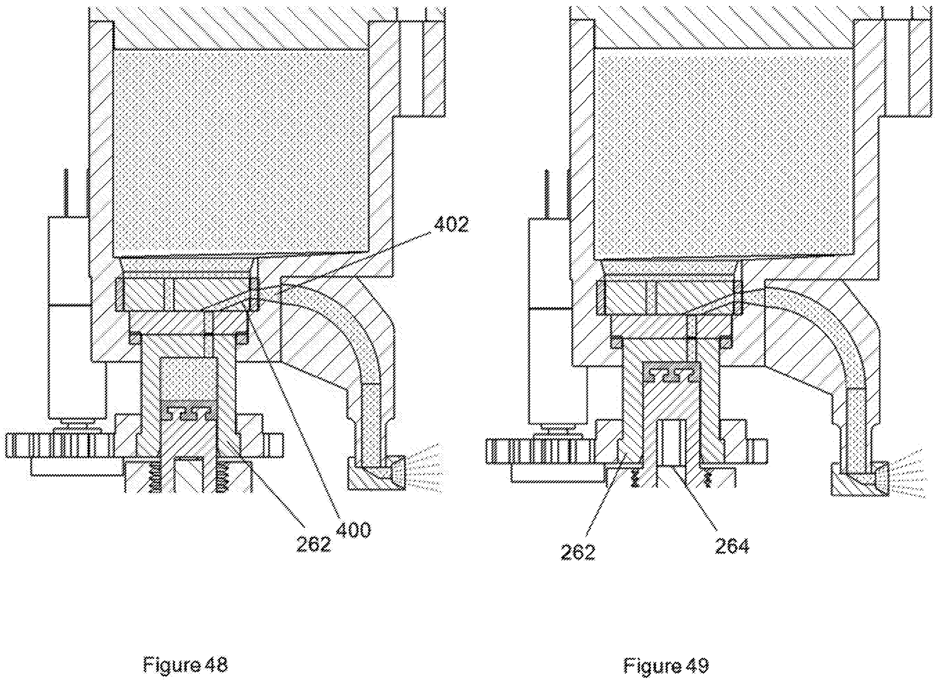

In the next stage shown in figure 48, the dosing chamber 262 and first disc 386 are rotated such that the channel 392 in the first disc 386 is aligned with the second channel 400 in the second disc 394. The dosing chamber 262 is thus fluidly connected with the outlet 402 and the nozzle 404. The medicament is thus dispensed to the user. This arrangement is shown in figure 52.

As shown in figure 49, the piston 264 is driven to abut the end of the dosing chamber 262. The medicament is thus completely expelled from the dosing chamber 262. A measured and complete dose is therefore provided.

Any aspects of the first embodiment of the inhaler 2 may be used with the second and/or third embodiment of the inhaler 2. For the avoidance of doubt, the second/third embodiment may comprise any or all of the aforementioned:

- validation system 102;
- canister 24;
- senor module 4;
- spirometry test;
- dosage adjustment system; or
- training mode.

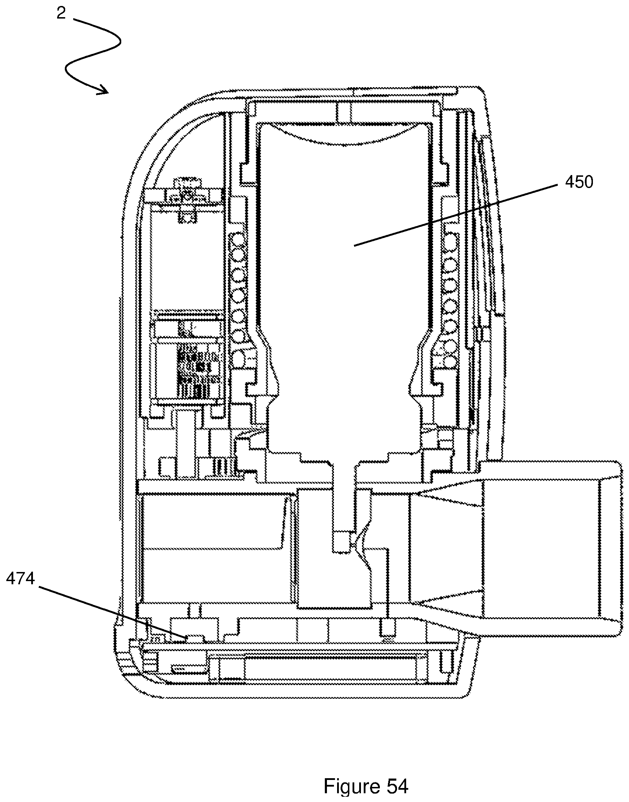

A further embodiment of the inhaler 2 is shown in figure 54. Like features of the inhaler 2 with previous embodiments disclosed herein will not be described in detail for the sake of brevity. The inhaler 2 comprises a canister 450. The canister 450 provides a reservoir comprising one or more inhalable therapeutic agents and/or one or more medicament in use. The canister 450 may be metallic. The canister 450 may be generally cylindrical. The canister 450 may be removable/detachable from the inhaler 2. This allows refilling/replacement thereof.

The canister 450 may be a standard MDI canister which meets the requirements of the US, EP and/or JP pharmacopoeias, or similar such compendial standards. The canister 450 may be a standard 10 ml, 14 ml, 17 ml, 19 ml or 22 ml canister. The canister may be manufactured from aluminium, aluminium alloy, stainless steel, glass, etc. Where the canister 450 is metallic, it may be manufactured by deep drawing or impact extrusion. The canister 450 may be thin-walled (suitable for use with CFC propellants) or thick-walled (for use with the higher pressures associated with HFA propellants). The interior surface of the canister 450 may be coated or uncoated or the surface energy performance is improved, thus helping to prevent active content of a drug formulation sticking to the canister wall. Suitable canisters may be suppled by PressTeck (RTM) sPa (Italy) or H&T Presspart (RTM)(UK/Germany), or such like.

The canister 450 may be in the form as a component of a finished pharmaceutical product, e.g. marketed as Airomir, AirSalb, Salamol, Ventloin (salbutamol), Atimos Modulite Neovent, Serevent (containing LABAs); Atrovent MDI (ipratropium); Clenil Modulite MDI, Qvar Easi-Breath; Flixotide Evohaler, Qvar Autohaler, Qvar MDI (each containing an inhaled corticosteroid (ICS)), Fostair MDI, Flutiform MDI, Seretide EVohaler, Sirdupla MDI or Symbicort MDI (ICS/LABA combination). The above brand names are registered trademarks of their respective owners.

The canister 450 comprises a valve mechanism 452. The valve mechanism 452 is configured to dispense a dose of the one or more inhalable therapeutic agents from within the canister 450. In the present embodiment, the valve mechanism 452 comprise a metered dose mechanism. The valve mechanism 452 therefore dispenses a fixed dose of the one or more inhalable therapeutic agents or medicaments in use. The valve mechanism 452 comprises a moveable stem 454. The stem 454 is resiliently biased toward a closed position (i.e. away from the canister 450). Upon depression of the stem 454, medicament is configured to be dispense therethrough. Such metered dose mechanisms are well known and will not be described further.

The canister 450 is mounted within a carriage 456. The carriage 456 provides a sleeve/collar or the like. The carriage 456 is resiliently biased toward the inhaler air passage 458. This biases the stem 454 into engagement with a sump 460. The bias is provided via a spring 462. The carriage is interposed the spring 462 and the canister 450.

The sump 460 is provided within the air passage 458. The sump 460 comprises a nozzle 464 or the like. A sealing collar 466 is provided between the canister 450 and the air passageway. This prevents air entering the space containing the canister 450. A nozzle cover 468 may selectively close the nozzle 464 as previously described.

A sensor module 470 is attached to the inhaler 2. The module 470 may be integrally attached or detachable from the inhaler 2. The module 470 may be as previously described (e.g. an NO sensor). A closure 472 may selectively close the air passageway 458 to direct air into a sensor module 470. The closure 472 may be pivotally mounted to the airway 458 (i.e. at an edge thereof). The closure 472 may be configured to close the airway 458 upon exhalation by the user and open the airway 458 upon inhalation by the user. For example, the closure 472 may be resiliently biased toward the closed position. The closure 472 may be biased into abutment with a stop member or the like in the closed position. During exhalation, air passes through the airway 458 and into engagement with the closure 472. The air pressure further biases the closure 472 into engagement with the stop member, thus retaining the closure 472 in the closed position. During inhalation, air pressure moves biases the closure 472 away from the stop member and against the biasing force. The closure 472 moves toward the open position, thus letting air flow therethrough.

A pressure sensor 474 is operatively connected to the air passageway 458 to measure the pressure in the air passageway 458. This may be used to detect inhalation and/or spirometry as previously described. The pressure sensor and/or pressure sensing system is configured to measure the pressure at one or more predetermined time interval (for example, every 10ms).

An actuation system is configured to facilitate the dispensing of a medicament dose comprising one or more inhalable therapeutic agents, in response to the detection of an inhalation event. The actuation comprises an actuator (e.g. a motor or solenoid) configured to move the collar 466 toward the air passageway 458. This allows movement of the canister 450 under bias of the spring 462. In turn, this depresses the stem 454, thus dispensing a dose. When the actuator comprises a rotary motor, the motor may comprise an eccentric cam or such like, configured to engage the collar 466. The spring 462 helps to overcome the biasing force of the stem 454, thus reducing the force needed to be applied via the actuation system.

In other embodiments, the actuator may directly engage the canister 450 and/or carriage 456 to provide dispending thereof (e.g. move the canister 450 toward the air passageway 458). Additionally or alternatively, the actuator may be configured to move the sump 460.

The dispensing of the medicament is electronically controlled (i.e. the actuation system is electronically controlled). This ensures accurate timing and/or coordination of the dispensing with the user inhalation, ensuring accurate delivery of the medicament suitably comprising one or more inhalable therapeutic agents.

A further embodiment of the inhaler 2 is shown in figure 54. The inhaler 2 is substantially the same as that described in figure 1, however, the sensor module 470 is not provided. The inhaler 2 comprises a pressure sensor 474. The device may therefore be used for breath actuated inhalation, of for example a fixed dose of one or more inhalable therapeutic agents, and/or spirometry.

In some embodiments, the canister 450 may comprise an identifier and/or electronic memory. This may allow identification of the medicament used and/or record dose usage etc. as previously described.

As the canister 450 in the above embodiments is configured to dispense a fixed dose of medication or one or more inhalable therapeutic agents, the inhaler 2 may be configured to dispense a plurality of discrete, fixed doses according to the dosage regimen. The actuation system may be configured to actuate a plurality of times accordingly. Each actuation may be provided upon detection of an inhalation event. The inhaler 2 may record the number of doses dispensed for each given dosage cycle. Once the required number of doses has been dispensed, the inhaler 2 may prevent activation of the actuation system. This ensures the correct dosing.

In some embodiments, the canister 450 may comprise a non-metered valve. Medicament may therefore be dispensed according with the time period the valve is opened. The inhaler may therefore be configured to adjacent the time period of activation of the actuation system in accordance with the required dose. The dose may be provided as a plurality of doses to ensure an appropriate dose is delivered with each inhalation.

The inhaler 2 of either embodiment may comprise any of the aforementioned system and/or features, where not mutually exclusive. For the avoidance of doubt, the inhaler 2 may comprise, inter alia, any or all of the aforementioned:

- • validation system
- • spirometry test;
- • dosage adjustment system; or
- • training mode.

"Active ingredient", "therapeutically active ingredient", "inhalable therapeutic agents", "active agent", "medicament", "drug" or "drug substance" as used herein interchangeably, means the active ingredient of a pharmaceutical, also known as an active pharmaceutical ingredient (API).

"Fixed dose combination" as used herein refers to a pharmaceutical product that contains two, three or more active ingredients that are formulated together in a single dosage form or receptacle and approved for human administration in certain fixed doses.

"Personalised dosage" as used herein, refers to a dosage of one or more inhalable therapeutic agents, dispensed from the inhaler device described herein, where said dosage is determined by reference to a number of variables and parameters.

In an embodiment of the invention, there is provided an inhaler configured to dispense a personalised dosage of one or more inhalable therapeutic agents to a subject, the inhaler comprising a sensor to detect and/or analyse one or more biomarkers present in the exhalation of the subject selected from ketone; volatile organic compounds (VOC) including saturated and unsaturated hydrocarbons, sulphur-containing VOC and nitrogen-containing VOC; dimethyl sulphide; ethanol; acetaldehyde; short chain alkanes (e.g. ethane and/or pentane); cyclic monoterpenes, e.g. limonene; carbon dioxide; carbon monoxide, preferably wherein said biomarker is nitric oxide. For example, said sensor is configured to determine a concentration of said one or more biomarkers, including the fractional concentration of exhaled nitric oxide (FeNO).

In a further preferred embodiment, said sensor is configured to determine a concentration of exhaled carbon dioxide and/or carbon monoxide, optionally whereupon the inhaler may dispense a specific and/or personalised dose of a suitable therapeutic agent.

In an embodiment of the invention, is provided an inhaler wherein said personalised dosage of one or more inhalable therapeutic agents is dispensed into a fluid pathway, where the sensor is configured to detect the presence of said one or more biomarkers in the fluid pathway, or where said sensor is fluidly connected to the fluid pathway via a fluid channel.

In another embodiment of the invention, is provided an inhaler which comprises a mouthpiece and an outlet such that during exhalation of the subject, exhaled air enters the inhaler via the mouthpiece and exits the inhaler via an outlet, such as where said outlet comprises a valve configured to at least partially obscure the outlet, such that at least a portion of exhaled air is diverted to the fluid pathway.

In another embodiment of the invention, is provided an inhaler where a sensor is fluidly connected to the fluid pathway via a fluid channel and where the fluid pathway comprises a filter, such as a moisture filter including a Peltier moisture filter, or wherein said fluid pathway comprises a pump, optionally where the sensor is removably attached to the inhaler.

In another embodiment of the invention, is provided a method of determining a personalised dosage for a variable dose inhaler comprising: detecting and/or analysing one or more variables using a sensor provided on the inhaler; analysing input parameters, and, adjusting the personalised dosage delivered to the subject according to the one or more variables and/or input parameters.

In a further embodiment of the invention, is provided a method of determining a personalised dosage for a variable dose inhaler comprising: detecting and/or analysing one or more variables using a sensor provided on the inhaler; analysing input parameters, and, adjusting the personalised dosage delivered to the subject according to the one or more variables and/or input parameter, wherein said one or more variables and input parameters comprises:

- dose and frequency of inhaled therapies over the preceding 7 days or more, such as reliever therapy
- environmental conditions such as relative humidity, pollen count, pollution levels such as nitrogen dioxide or PM 2.5 levels
- training mode data as described herein
- measured flowrate using integral flow meter (spirometry), such as FEV1, or PEF, or a parameter derived therefrom
- data from biomarker analysis, suitably wherein said biomarker is FeNO

In an embodiment of the invention, is provided an inhaler configured to dispense a personalised dosage of one or more inhalable therapeutic agents to a subject, wherein said personalised dosage is proportional to the severity of the subject's symptoms.

In yet another embodiment of the invention, is provided a method for treating a condition or disease in a subject, the method comprising: providing an inhaler device of the invention described herein, and delivering a personalised dosage of one or more inhalable therapeutic agents to the subject.

In yet a further embodiment of the invention, is provided a method for treating a condition or disease in a subject, the method comprising: providing a device of the invention described herein, and delivering a personalised dosage of one or more inhalable therapeutic agents to the subject. further comprising activating the inhaler device to detect and/or analyse one or more biomarkers present in the exhalation of the subject, wherein said biomarkers determine the presence and/or severity of said condition or disease. For example, said biomarker may be indicative of diabetic ketoacidosis, such as acetone; or lung cancer, such as decane or analogs thereof; or liver cancer, such as acetaldehyde or limonene; or lung inflammation associated with elevated FeNO, such as severe asthma or SARS-Cov-2 infection.

In a further embodiment of the invention, is provided a method for treating a condition or disease in a subject, the method comprising: providing a device of the invention described herein, and delivering a personalised dosage of one or more inhalable therapeutic agents to the subject, wherein said disease is a pulmonary disease selected from: Acute Respiratory Distress Syndrome (ARDS), Adult Respiratory Distress Syndrome (Adult RDS), hyperoxic lung injury, or Bronchopulmonary dysplasia (BPD), chronic obstructive pulmonary disease (COPD), exacerbated COPD, Asthma, severe Asthma, exacerbated Asthma, allergic Asthma, Allergic Bronchopulmonary Aspergillosis (ABPA), exercise-induced bronchospasm, exercise-induced asthma, aspirin-exacerbated respiratory disease, NSAID-exacerbated respiratory disease, Acute lung injury, Airway remodeling, Bronchiolitis obliterans syndrome, pneumonia, cystic fibrosis (CF), idiopathic pulmonary fibrosis (IPF), pulmonary arterial hypertension (PAH), pulmonary alveolar proteinosis (PAP), acute bronchitis, brorichitis, bronchiectasis, primary ciliary dyskinesia, respiratory syncytial virus infection (RSV), chronic cough, SARS-Cov-2 infection or lung cancer.

In a specific embodiment of the invention, is provided a method for treating a condition or disease in a subject, the method comprising: providing a device of the invention described herein, and delivering a personalised dosage of one or more inhalable therapeutic agents to the subject, wherein said one or more inhalable therapeutic agents is selected from: beta-agonists; corticosteroids; muscarinic antagonists; RhoA inhibitors; GGTase-I or -II inhibitors; ROCK1 and/or ROCK2 inhibitors; soluble epoxide hydrolase inhibitors; fatty acid amide hydrolase inhibitors; leukotriene receptor antagonists; phosphodiesterase-4 inhibitors such as roflumilast; 5-lipoxygenase inhibitors such as zileuton; mast cell stabilizers such as nedocromil; theophylline; anti-IL5 antibodies; ariti-IgE antibodies; anti-IL5 receptor antibodies; anti-IL13/4 receptor antibodies; statins such as simvastatin, pitavastatin, rosuvastatin, and atorvastatin, and isomers, enantiomers, and diastereomers thereof; CF agents including one or more of VX809, VX661, or VX770; biologics such as mepolizumab, reslizumab, benralizumab, omalizumab, and dupilumab; beta-agonist and muscarinic antagonist combinations, including both long- and short-acting formulations; beta-agonist and corticosteroid combinations, including both long- and short-acting formulations; corticosteroids and muscarinic antagonist combinations, including both long- and short-acting formulations; and beta-agonist, corticosteroid, and muscarinic antagonist combinations, including both long- and short-acting formulations, including fixed dose dual or triple combinations thereof.

In a specific embodiment of the invention, the inhalable therapeutic agent is a beta-agonist selected from: albuterol, aformoterol, formoterol, salmeterol, indacaterol, levalbuterol, salbutamol, terbutaline, olodaterol, vilanterol, isoxsuprine, mabuterol, zilpaterol, bambuterol, clenbuterol, formoterol, salmeterol, abediterol, and carmoterol, buphenine, bopexamine, epinephrine, fenoterol, isoetarine, isoproterenol, orciprenaline, levoalbutamol, pirbuterol, procaterol, ritodrine, arbutamine, befunolol, bromoacetylalprenololmenthane, broxaterol, cimaterol, cirazoline, etilefrine, hexoprenaline, higenamine, methoxyphenamine, oxyfedrine, ractopamine, reproterol, rimiterol, tretoquinol, tulobuterol, zilpaterol, and zinterol, including stereoisomers, a salt thereof, a solvate thereof, a solvate of a salt thereof, and mixtures thereof.

In a further embodiment of the invention the inhalable therapeutic agent is a corticosteroid selected from: beclomethasone, fluticasone, budesonide, mometasone, flunisolide, alclometasone, beclometasone, betamethasone, clobetasol, clobetasone, clocortolone, desoximetasone, dexamethasone, diflorasone, difluocortolone, flurclorolone, flumetasone, fluocortin, fluocortolone, fluprednidene, fluticasone, fluticasone furoate, halometasone, meprednisone, mometasone, mometasone furoate, paramethasone, prednylidene, rimexolone, ulobetasol, amcinonide, ciclesonide, deflazacort, desonide, formocortal, fluclorolone acetonide, fludroxycortide, fluocinolone acetonide, fluocinonide, halcinonide, triamcinolone acetonide, or a salt thereof, a solvate thereof, a solvate of a salt thereof, and mixtures thereof.

In a specific embodiment of the invention, the inhalable therapeutic agent is a muscarinic antagonist selected from: ipratropium bromide, tiotropium, glycopyrrolate, glycopyrronium bromide, revefenacin, umeclidinium bromide, aclidinium, trospium chloride, oxitropium bromide, oxybutynin, tolterodine, solifenacin, fesoterodine, darifenacin, or a salt thereof, a solvate thereof, a solvate of a salt thereof, and mixtures thereof, or a ROCK inhibitor selected from: fasudil, ripasudil, netarsudil, RKI-1447, Y-27632, Y-30141, and GSK429286A, or a salt thereof, a solvate thereof, a solvate of a salt thereof, and mixtures thereof.

In a preferred embodiment of the invention, is provided a method for treating a condition or disease in a subject, the method comprising: providing a device of the invention described herein, and delivering a personalised dosage of one or more inhalable therapeutic agents to the subject, wherein said personalised dosage, comprises administration in amount of from about 1% to about 100% of the FDA approved dose of each inhalable therapeutic agent, such as between 25 % to 75 %, or between 50 % to 100 % thereof, or from about 1 % to about 100 % of the FDA approved fixed dose dual or triple combinations listed in Table 1, such as between 25 % to 75 %, or between 50 % to 100 % thereof.

**Table 1**

| Drug class | Generic name | Brand name | Manuf'r | Device | Dose |
|---|---|---|---|---|---|
| LABA/LAMA | Umeclidinium/vilanterol | Anoro® Ellipta® 55 micrograms/22 micrograms | GSK, Middlesex, UK | DPI | One dose once daily |
| | Formoterol/aclidinium | Duaklir® Genuair® 340 micrograms/12 micrograms | AstraZeneca, Bedfordshire, UK | DPI | One dose once daily |
| | Olodaterol/tiotropium | Spiolto® Respimat® 2.5 micrograms/2.5 micrograms | Boehringer Ingelheim, Berkshire, UK | Soft mist inhaler | Two puffs once daily |
| | Budesonide/formoterol | DuoResp® Spiromax® 160/4.5 micrograms; 320/9 micrograms | Teva UK Limited, West Yorkshire, UK | DPI | 320/9 micrograms twice daily |
| | | Fobumix Easyhaler® 160/4.5 micrograms; 320/9 micrograms | Orion Pharma UK Ltd, Berkshire, UK | DPI | 320/9 micrograms twice daily |
| | | Symbicort® pMDI 200/6 micrograms | AstraZeneca, Bedfordshire, UK | Aerosol | Two puffs twice daily |
| | | Symbicort Turbohaler® 200/6 micrograms; 400/12 micrograms | AstraZeneca, Bedfordshire, UK | DPI | 400/12 micrograms twice daily |
| | Fluticasone furoate/vilanterol | Relvar® Ellipta 92/22 micrograms | GSK, Middlesex, UK | DPI | One dose once daily |
| | Fluticasone propionate/salmeterol | Aerivo® Spiromax 500/50 micrograms | Teva UK Limited, West Yorkshire, UK | DPI | One dose twice daily |
| | | AirFluSal® Forspiro® 500/50 micrograms | Novartis, Surrey, UK | DPI | One dose twice daily |
| | | Fusacomb Easyhaler 500/50 micrograms | Orion Pharma UK Ltd, Berkshire, UK | DPI | One dose twice daily |
| | | Seretide® Accuhaler® 500/50 micrograms | GSK, Middlesex, UK | DPI | One dose twice daily |
| ICS/LABA/LAMA | Fluticasone furoate/ umeclidinium/vilanterol | Trelegy® Ellipta 92 micrograms/55 micrograms/22 micrograms | GSK, Middlesex, UK | DPI | One dose once daily |
| | indacaterol, glycopyrronium, and mometasone furoate | Enerzair/Zimbus Breezhaler, 114µg/46µg/136µg | Novartis Europharm Limited. | DPI | One dose once daily |
| | Beclometasone dipropionate/formoterol/ glycopyrronium | Trimbow® 87 micrograms/5 micrograms/9 micrograms | Chiesi Limited, Manchester, UK | Aerosol | Two puffs twice daily |

The brand names and/or manufacturer names in the above table may include registered trademarks (RTM) belonging to their respective owners.

In another embodiment of the invention, the one or more inhalable therapeutic agents is presented as a suspension, solution or dry powder.

In another embodiment of the invention, the one or more inhalable therapeutic agents is presented as a suspension, wherein said solution or suspension further comprises HFA propellant selected from HFA 134a, HFA 227a, HFA 152a and mixtures thereof, optionally a stabilizer selected from polysorbates, organic acids, sorbitans, fatty acid esters, polyvinylpyrrolidone and mixtures thereof, optionally a co-solvent selected from ethanol, glycerol, polyoxyethylene alcohols, polyethylene glycol, propylene glycol and mixtures thereof.

In another embodiment of the invention, the one or more inhalable therapeutic agents is presented as a dry powder, wherein said dry powder further comprises a pharmaceutically acceptable carrier or additive selected from: magnesium stearate monosaccharides, disaccharides, oligo- and polysaccharides, polyalcohols, cyclodextrins, amino acids, salts, and mixtures thereof. Also, the monosaccharide may be selected from the group consisting of glucose, fructose, and arabinose, or the polyalcohol is selected from the group consisting of sorbitol, mannitol, and xylitol, or said cyclodextrin is selected from the group consisting of a-cyclodextrin, b-cyclodextrin, c-cyclodextrin, methyl beta-cyclodextrin, and hydroxypropyl-beta-cyclodextrin, captisol, and sulfobutyl-beta-cyclodextrin or DexSol, or wherein said amino acid is selected from the group consisting of leucine, arginine or arginine hydrochloride, or wherein said salt is selected from the group consisting of sodium chloride, potassium chloride, sodium bromide, and calcium carbonate.

## Claims

1. An inhaler (2) configured to dispense a dosage of one or more inhalable therapeutic agent to a user, the inhaler comprising a sensor (166) to detect one or more biomarker present in gases expired by the user, said one or more biomarker suitably selected from: ketones; volatile organic compounds (VOC) including saturated and unsaturated hydrocarbons, sulphur-containing VOC and nitrogen-containing VOC; nitric oxide; dimethyl sulphide; ethanol; acetaldehyde; short chain alkanes (e.g. ethane and/or pentane); cyclic monoterpenes, e.g. limonene; and wherein said dosage of one or more inhalable therapeutic agent is dispensed into a flow passage (34) defined within the inhaler, and the sensor is configured to detect the presence of said one or more biomarkers in a fluid flow within the flow passage;
   further including a filter (174) for the expiratory air of the user.

2. An inhaler (2) according to claim 1, where said sensor (166) is configured to determine a concentration of said one or more biomarker, including the fractional concentration of exhaled nitric oxide (FeNO).

3. An inhaler (2) according to claim 1 or 2, where the therapeutic agent is dispensed via an outlet or nozzle (72, 76), and a closure (82) is provided to selectively obscure the outlet or nozzle.

4. An inhaler (2) according to any preceding claim, where said sensor (166) is fluidly connected to the flow passage (34) via a fluid channel (168).

5. An inhaler (2) according to any preceding claim, where the flow passage (34) comprises a mouthpiece (10) and an outlet (20) such that, expiratory gases from the user enter the inhaler via the mouthpiece and exit the inhaler via the outlet.

6. An inhaler (2) according to claim 5, where said flow passage (34) comprises a flow diverter (154) configured to at least partially obscure the flow passage, such that at least a portion of exhaled air is diverted to the sensor.

7. An inhaler (2) according to claim 6, where the flow diverter (154) at least partially obscures the flow passage (34) in a deployed condition, wherein the flow diverter is selectively actuatable between a retracted condition and said deployed condition.

8. An inhaler (2) according to any preceding claim, comprising a plurality of filters (170, 180, 184), optionally in series.

9. An inhaler (2) according to any preceding claim, where the filter (174) comprises a moisture filter, such as a Peltier moisture filter.

10. An inhaler (2) according to any preceding claim, comprising a pump (185) for pumping expiratory gases to the sensor (166).

11. An inhaler (2) according to any of claims 1 to 10, comprising a further sensor for the expiratory flow and/or an operational condition of the inhaler; optionally

where the further sensor comprises an ambient condition sensor, such as a humidity or temperature sensor; and / or
where the further sensor comprises a carbon dioxide sensor, a carbon monoxide sensor or other expiratory or inspiratory flow content sensor.

12. An inhaler (2) according to claim 11, where the further sensor comprises a flow rate or pressure sensor, e.g. a spirometry sensor; and / or
where the further sensor comprises a dosage counter, dosage frequency sensor or dosage interval sensor.

13. An inhaler (2) according to any preceding claim, where the sensor (166) is removably attached to the inhaler; optionally
where the sensor is provided within a sensor module (4) having one or more further components for cooperation with the operation of the sensor, the sensor module being removably attachable to the inhaler without preventing the dispensing a dosage of one or more inhalable therapeutic agents by the inhaler.

14. An inhaler (2) according to any preceding claim where the sensor (166) is provided as part of a sensor system, the inhaler further comprising a controller for: monitoring operation of the sensor system; controlling operation of a dose delivery mechanism of the inhaler; and/or analysing an output of the sensor.

15. An inhaler (2) according to any preceding claim, comprising a data store for logging one or more reading of the sensor (166).

**Patentansprüche**

1. Inhalator (2), der dazu konfiguriert ist, einem Benutzer eine Dosis eines oder mehrerer inhalierbarer therapeutischer Mittel abzugeben, wobei der Inhalator einen Sensor (166) zum Erkennen eines oder mehrerer in den vom Benutzer ausgeatmeten Gasen vorhandener Biomarker umfasst, wobei der eine oder die mehreren Biomarker passend ausgewählt sind aus: Ketonen; flüchtigen organischen Verbindungen (VOC) einschließlich gesättigter und ungesättigter Kohlenwasserstoffe, schwefelhaltiger VOC und stickstoffhaltiger VOC; Stickstoffmonoxid; Dimethylsulfid; Ethanol; Acetaldehyd; kurzkettige Alkane (z. B. Ethan und/oder Pentan; zyklische Monoterpene, z. B. Limonen; und wobei die Dosis eines oder mehrerer inhalierbarer therapeutischer Mittel in einen Strömungskanal (34) abgegeben wird, der innerhalb des Inhalators definiert ist, und der Sensor so konfiguriert ist, dass er das Vorhandensein des einen oder der mehreren Biomarker in einem Fluidstrom innerhalb des Strömungskanals erkennt;
ferner einen Filter (174) für die Ausatemluft des Benutzers umfassend.

2. Inhalator (2) nach Anspruch 1, wobei der Sensor (166) dazu konfiguriert ist, eine Konzentration des einen oder der mehreren Biomarker zu bestimmen, einschließlich der fraktionalen Konzentration von ausgeatmetem Stickstoffmonoxid (FeNO).

3. Inhalator (2) nach Anspruch 1 oder 2, wobei das therapeutische Mittel über einen Auslass oder eine Düse (72, 76) abgegeben wird und ein Verschluss (82) vorgesehen ist, um die Auslassdüse selektiv zu verdecken.

4. Inhalator (2) nach einem vorhergehenden Anspruch, wobei der Sensor (166) über einen Fluidkanal (168) fluidisch mit dem Strömungskanal (34) verbunden ist.

5. Inhalator (2) nach einem vorhergehenden Anspruch, wobei der Strömungskanal (34) ein Mundstück (10) und einen Auslass (20) umfasst, sodass Ausatemgase des Benutzers über das Mundstück in den Inhalator gelangen und über den Auslass aus dem Inhalator austreten.

6. Inhalator (2) nach Anspruch 5, wobei der Strömungskanal (34) einen Strömungsumleiter (154) umfasst, der so konfiguriert ist, dass er den Strömungskanal zumindest teilweise verdeckt, sodass zumindest ein Teil der ausgeatmeten Luft zum Sensor umgeleitet wird.

7. Inhalator (2) nach Anspruch 6, wobei der Strömungsumleiter (154) den Strömungskanal (34) in einem ausgefahrenen Zustand zumindest teilweise verdeckt, wobei der Strömungsumleiter selektiv zwischen einem eingefahrenen Zustand und dem ausgefahrenen Zustand betätigbar ist.

8. Inhalator (2) nach einem der vorhergehenden Ansprüche, der eine Vielzahl von Filtern (170, 180, 184), optional in Reihe, umfasst.

9. Inhalator (2) nach einem vorhergehenden Anspruch, wobei der Filter (174) einen Feuchtigkeitsfilter, beispielsweise einen Peltier-Feuchtigkeitsfilter, umfasst.

10. Inhalator (2) nach einem vorhergehenden Anspruch, der eine Pumpe (185) zum Pumpen von Ausatemgasen zum Sensor (166) umfasst.

11. Inhalator (2) nach einem der Ansprüche 1 bis 10, der einen weiteren Sensor für die exspiratorische Strömung und/oder einen Betriebszustand des Inhalators umfasst; optional

> wobei der weitere Sensor einen Umgebungsbedingungssensor, wie etwa einen Feuchtigkeits- oder Temperatursensor, umfasst; und / oder
> wobei der weitere Sensor einen Kohlendioxidsensor, einen Kohlenmonoxidsensor oder einen anderen Sensor für exspiratorischen oder inspiratorischen Durchflussinhalt umfasst.

12. Inhalator (2) nach Anspruch 11, wobei der weitere Sensor einen Durchfluss- oder Drucksensor, beispielsweise einen Spirometriesensor, umfasst; und / oder
wobei der weitere Sensor einen Dosierungszähler, einen Dosierungsfrequenzsensor oder einen Dosierungsintervallsensor umfasst.

13. Inhalator (2) nach einem vorhergehenden Anspruch, wobei der Sensor (166) abnehmbar am Inhalator angebracht ist; optional
wobei der Sensor in einem Sensormodul (4) vorgesehen ist, das eine oder mehrere weitere Komponenten zum Zusammenwirken mit dem Betrieb des Sensors aufweist, und wobei das Sensormodul abnehmbar am Inhalator angebracht werden kann, ohne die Abgabe einer Dosis eines oder mehrerer inhalierbarer therapeutischer Mittel durch den Inhalator zu verhindern.

14. Inhalator (2) nach einem vorhergehenden Anspruch, wobei der Sensor (166) als Teil eines Sensorsystems bereitgestellt wird und der Inhalator ferner eine Steuerung umfasst zum: Überwachen des Betriebs des Sensorsystems; und/oder Analysieren einer Ausgabe des Sensors.

15. Inhalator (2) nach einem vorhergehenden Anspruch, der einen Datenspeicher zum Protokollieren einer oder mehrerer Messungen des Sensors (166) umfasst.

**Revendications**

1. Inhalateur (2) configuré pour distribuer une dose d'un ou plusieurs agents thérapeutiques inhalables à un utilisateur, l'inhalateur comprenant un capteur (166) conçu pour détecter un ou plusieurs biomarqueurs présents dans des gaz expirés par l'utilisateur, lesdits un ou plusieurs biomarqueurs étant sélectionnés de manière appropriée parmi : des cétones ; des composés organiques volatils (COV), y compris des hydrocarbures saturés et insaturés, des COV contenant du soufre et des COV contenant de l'azote ; de l'oxyde nitrique ; du sulfure de diméthyle ; de l'éthanol ; de l'acétaldéhyde ; des alcanes à chaîne courte (par exemple l'éthane et/ou le pentane) ; des monoterpènes cycliques, par exemple le limonène ; et ladite dose d'un ou plusieurs agents thérapeutiques inhalables étant distribuée dans un passage d'écoulement (34) délimité à l'intérieur de l'inhalateur, et le capteur étant configuré pour détecter la présence desdits un ou plusieurs biomarqueurs dans un écoulement de fluide à l'intérieur du passage d'écoulement ; comprenant en outre un filtre (174) pour l'air expiratoire de l'utilisateur.

2. Inhalateur (2) selon la revendication 1, dans lequel ledit capteur (166) est configuré pour déterminer une concentration desdits un ou plusieurs biomarqueurs, y compris la concentration fractionnée d'oxyde nitrique (FeNO) expiré.

3. Inhalateur (2) selon la revendication 1 ou 2, dans lequel l'agent thérapeutique est distribué par l'intermédiaire d'une sortie ou d'une buse (72, 76), et une fermeture (82) est présente pour obstruer sélectivement la sortie ou la buse.

4. Inhalateur (2) selon l'une quelconque des revendications précédentes, dans lequel ledit capteur (166) est connecté de manière fluidique au passage d'écoulement (34) par un canal de fluide (168).

5. Inhalateur (2) selon l'une quelconque des revendications précédentes, dans lequel le passage d'écoulement (34) comprend un embout buccal (10) et une sortie (20) de telle sorte que les gaz expiratoires provenant de l'utilisateur pénètrent dans l'inhalateur par l'embout buccal et sortent de l'inhalateur par la sortie.

6. Inhalateur (2) selon la revendication 5, dans lequel ledit passage d'écoulement (34) comprend un déflecteur d'écoulement (154) configuré pour obstruer au moins en partie le passage d'écoulement, de telle sorte qu'au moins une partie de l'air expiré soit déviée vers le capteur.

7. Inhalateur (2) selon la revendication 6, dans lequel le déflecteur d'écoulement (154) obstrue au moins en partie le passage d'écoulement (34) dans un état déployé, le déflecteur d'écoulement pouvant être actionné de manière sélective entre un état rétracté et ledit état déployé.

8. Inhalateur (2) selon l'une quelconque des revendications précédentes, comprenant une pluralité de filtres (170, 180, 184), optionnellement en série.

9. Inhalateur (2) selon l'une quelconque des revendications précédentes, dans lequel le filtre (174) comprend un filtre à humidité, tel qu'un filtre à humidité à effet Peltier.

10. Inhalateur (2) selon l'une quelconque des revendications précédentes, comprenant une pompe (185) pour amener par pompage des gaz expiratoires au capteur (166).

11. Inhalateur (2) selon l'une quelconque des revendications 1 à 10, comprenant un capteur supplémentaire pour le flux expiratoire et/ou une condition de fonctionnement de l'inhalateur ; optionnellement

dans lequel le capteur supplémentaire comprend un capteur de condition ambiante, tel qu'un capteur d'humidité ou de température ; et/ou
dans lequel le capteur supplémentaire comprend un capteur de dioxyde de carbone, un capteur de monoxyde de carbone ou un autre capteur pour la composition du flux expiratoire ou inspiratoire.

12. Inhalateur (2) selon la revendication 11, dans lequel le capteur supplémentaire comprend un capteur de débit ou de pression, par exemple un capteur de spirométrie ; et/ou
dans lequel le capteur supplémentaire comprend un compteur de doses, un capteur de fréquence posologique ou un capteur d'intervalle posologique.

13. Inhalateur (2) selon l'une quelconque des revendications précédentes, dans lequel le capteur (166) est fixé de manière amovible à l'inhalateur ; optionnellement
dans lequel le capteur est présent à l'intérieur d'un module de capteur (4) comportant un ou plusieurs composants supplémentaires destinés à coopérer avec le fonctionnement du capteur, le module de capteur pouvant être fixé de manière amovible à l'inhalateur sans empêcher la distribution d'une dose d'un ou plusieurs agents thérapeutiques inhalables par l'inhalateur.

14. Inhalateur (2) selon l'une quelconque des revendications précédentes, dans lequel le capteur (166) présent fait partie d'un système de détection, l'inhalateur comprenant en outre un dispositif de commande pour : surveiller le fonctionnement du système de détection ; commander le fonctionnement d'un mécanisme de distribution de dose de l'inhalateur ; et/ou analyser une sortie du capteur.

15. Inhalateur (2) selon l'une quelconque des revendications précédentes, comprenant un magasin de données pour l'enregistrement d'une ou plusieurs lectures du capteur (166).

Figure 1

Figure 2

Figure 3

Figure 5

Figure 4

Figure 6

Figure 7

Figure 8

Figure 9

Figure 10

Figure 11

Figure 12

Figure 13

Figure 14

Figure 15

Figure 16

24

52

150

Figure 17

Figure 18

Figure 19

Figure 20

154

158

160

Figure 21

Figure 22

Figure 23a

Figure 23b

Figure 23c

Figure 24

```
┌─────────────────┐
│  Sensor module  │
│     attached    │
│       206       │
└─────────────────┘
         ⇩
┌─────────────────┐
│  Sensor module  │
│    detection    │
│       208       │
└─────────────────┘
         ⇩
┌─────────────────┐
│   Close shield  │
│    and door     │
│       210       │
└─────────────────┘
         ⇩
┌─────────────────┐          ┌─────────────────┐
│       NO        │          │                 │
│   measurement   │  ⇔       │     Restart     │
│       212       │          │       218       │
└─────────────────┘          └─────────────────┘
         ⇩
┌─────────────────┐          ┌─────────────────┐
│    Pressure     │          │                 │
│   monitoring    │  ⇨       │   Notification  │
│       214       │          │       216       │
└─────────────────┘          └─────────────────┘
         ⇩
┌─────────────────┐
│ Record/transmit │
│      data       │
│       220       │
└─────────────────┘
```

Initiate
spirometry
222

⇩

Close shield
and open door
224

⇩

Pressure
monitoring
226

⇩

Record data
228

⇩

Process data
230

Figure 25

Figure 26

Figure 27

| Onboard sensor 234 | ⇨ | Receive indicators 232 | ⇦ | Remote device 236 |
|---|---|---|---|---|

⇩ ⇗

| Process indicators 240 | Remote processing 242 |
|---|---|

⇩ ⇗

Record new
dose regime
244

⇩

Adjust dose
size
246

Figure 28

Initiate training
mode
248

⇩

Close shield
and open door
250

⇩

Pressure
monitoring
252

⇩

Record data
254

⇩

Process data
256

⇩

Provide
feedback
258

Figure 29

Figure 30

Figure 31A

Figure 31B

264

272

350

352

356

358

354

291　290

288

354

362

360

286

Figure 31C

Figure 32

Figure 33

Figure 34

Figure 35

328

Figure 36

Figure 37

Figure 38

Figure 39

Figure 40a

Figure 40b

Figure 41

2

284

406

374

372

380

376

378

Figure 42

384

394

386

390

370

380

382

284

260

262

264

404

278

Figure 43

Figure 44

Figure 45

Figure 47

Figure 46

Figure 49

264

262

Figure 48

402

262  400

394

386

262

Figure 52

394

386

388

262

Figure 51

Figure 50

2

450
462
456
452
454

466
460
472
474

458
468
464
470

Figure 53

Figure 54

**EP 4 329 849 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2016060863 A
- WO 2015066562 A
- WO 2004011068 A
- EP 3185940 A